# EUROPÄISCHE PATENTSCHRIFT

(11) **EP 2 941 472 B1**
(45) Veröffentlichungstag und Bekanntmachung des Hinweises auf die Patenterteilung: **25.07.2018**
(21) Anmeldenummer: 13802251.2
(22) Anmeldetag: 09.12.2013
(51) Int. Cl.: C09K 11/06, H01L 51/00, C07C 211/54, C07C 211/61, C07D 307/91, H01L 51/50, C07D 333/50, C07D 409/12, C07D 307/77, C07D 209/80

(54) **MATERIALIEN FÜR ELEKTRONISCHE VORRICHTUNGEN**
MATERIALS FOR ELECTRONIC DEVICES
MATÉRIAUX POUR DISPOSITIFS ÉLECTRONIQUES

(30) Priorität: 03.01.2013 EP 13000012
(43) Veröffentlichungstag der Anmeldung: 11.11.2015
(73) Patentinhaber: Merck Patent GmbH, 64293 Darmstadt (DE)
(72) Erfinder: HEIL, Holger, 60389 Frankfurt am Main (DE); RODRIGUEZ, Lara-Isabel, 64283 Darmstadt (DE); ECKES, Fabrice, FR 68300 Sant Louis (FR); GERHARD, Anja, 63329 Egelsbach (DE)
(86) Internationale Anmeldenummer: PCT/EP2013/003716
(87) Internationale Veröffentlichungsnummer: WO 2014/106522

(56) Entgegenhaltungen:
- EP-A2- 2 145 936
- EP-A2- 2 388 843
- WO-A1-2008/006449
- WO-A1-2010/049050

## Beschreibung

Die vorliegende Erfindung betrifft eine Verbindung einer Formel (I). Die Verbindung eignet sich zur Verwendung als Funktionsmaterial in einer elektronischen Vorrichtung, insbesondere einer organischen Elektrolumineszenzvorrichtung (OLED). Weiterhin betrifft die Erfindung bestimmte Ausführungsformen von elektronischen Vorrichtungen enthaltend die Verbindung der Formel (I) sowie Verfahren zur Herstellung der Verbindung der Formel (I).

Unter dem Begriff elektronische Vorrichtung werden gemäß der vorliegenden Erfindung allgemein elektronische Vorrichtungen verstanden, welche organische Materialien enthalten. Bevorzugt werden darunter OLEDs verstanden sowie einige weitere Ausführungsformen von elektronischen Vorrichtungen enthaltend organische Materialien, die in der Anmeldung an späterer Stelle offenbart werden.

Der allgemeine Aufbau sowie das Funktionsprinzip von OLEDs ist dem Fachmann bekannt und unter anderem in US 4539507, US 5151629, EP 0676461 und WO 1998/27136 beschrieben.

Betreffend die Leistungsdaten der elektronischen Vorrichtungen sind weitere Verbesserungen erforderlich, insbesondere in Hinblick auf eine breite kommerzielle Verwendung, beispielsweise in Displays oder als Lichtquellen. Von besonderer Bedeutung sind in diesem Zusammenhang die Lebensdauer, die Effizienz und die Betriebsspannung der elektronischen Vorrichtungen sowie die realisierten Farbwerte.

Insbesondere bei blau emittierenden OLEDs besteht Verbesserungspotential bezüglich der Lebensdauer der Vorrichtungen und den erreichten Farbwerten des emittierten Lichts.

Ein wichtiger Ansatzpunkt, um die genannten Verbesserungen zu erreichen, ist die Auswahl der Emitterverbindung, die in der elektronischen Vorrichtung eingesetzt wird.

Im Stand der Technik sind als blau fluoreszierende Emitter eine Vielzahl an Verbindungen bekannt, insbesondere Arylamine mit einer oder mehreren kondensierten Arylgruppen und/oder Indenofluoren-Gruppen. Beispiele dafür sind die in der US 5,153,073 offenbarten Pyren-Arylamine und die in der WO 2012/048780 offenbarten Pyren-Arylamine. Weitere Beispiele für Arylamin-Emitter sind Benzoindenofluorenamine, z. B. gemäß WO 2008/006449, und Dibenzoindenofluorenamine, z. B. gemäß WO 2007/140847. Außerdem werden Benzoindenofluoren-Verbindungen als fluoreszierende Emitter in WO 2008/006449 offenbart.

Weiterhin ist im Stand der Technik die Verwendung von Fluorenaminen, welche an das Fluorensystem ankondensierte aromatische Gruppen aufweisen, bekannt. Die Verbindungen, die zwei oder mehr Arylaminogruppen aufweisen, werden als fluoreszierende Emitter eingesetzt (US 2012/0161615). Die Verbindungen zeigen jedoch grüne bis grün-blaue Emission und keine blaue Emission.

Weiterhin sind aus KR 2009/131536 und WO 2004/061048 Benzofluoren-Derivate bekannt, die eine Diphenylaminogruppe tragen. Solche Verbindungen weisen jedoch zu kurzwellige Emission auf, um als blaue fluoreszierende Emitter Verwendung zu finden, bzw. bei Verwendung in OLEDs ist ihre Effizienz und Lebensdauer unbefriedigend.

Zusammenfassend liegt also die technische Aufgabe vor, tiefblau fluoreszierende Emitter bereitzustellen, die bevorzugt eine schmale Emissionsbande aufweisen. Weiterhin bevorzugt liegt die Aufgabe vor, Verbindungen bereitzustellen, mit denen eine hohe Leistungseffizienz und eine lange Lebensdauer und tiefblaue Emission der elektronischen Vorrichtung erreicht werden kann.

Überraschend wurde nun gefunden, dass Arylamin-Verbindungen, bei denen zwei oder mehr Benzofluoren-Einheiten an den Stickstoff gebunden sind, tiefblaue Farbkoordinaten und ein sehr schmales Emissionsspektrum aufweisen und dadurch die oben vorgestellte technische Aufgabe lösen. Tiefblaue Farbkoordinaten bei Emitterverbindungen sind hoch erwünscht für die Verwendung in Displays und Beleuchtungsanwendungen. Insbesondere bei blau emittierenden Emitterverbindungen ist ein schmales

Emissionsspektrum, d.h. eine Emissionsbande mit einer geringen Breite, hoch erwünscht für die Abstimmung der Farbeindrücke der verschiedenen Farben in einem Display oder bei einer Beleuchtungsanwendung.

Gegenstand der Erfindung ist somit eine Verbindung einer Formel (I) 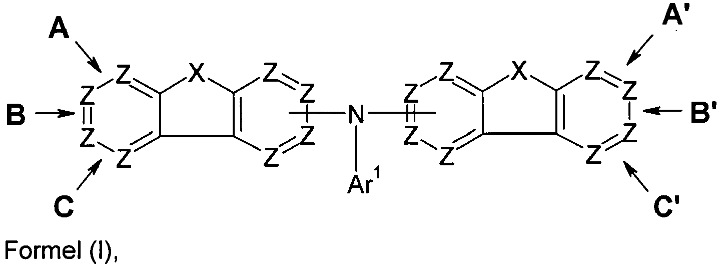 wobei gilt:
an mindestens eine der drei Bindungen gewählt aus den Bindungen A, B und C und an mindestens eine der drei Bindungen gewählt aus den Bindungen A', B' und C' ist ein aromatischer oder heteroaromatischer Sechsring ankondensiert, der mit einem oder mehreren Resten R¹ substituiert sein kann,
- Z: ist bei jedem Auftreten gleich oder verschieden CR¹ oder N;
- X: ist bei jedem Auftreten gleich oder verschieden BR², C(R²)₂, C(R²)₂-C(R²)₂, -R²C=CR²-, -R²C=N-, Si(R²)₂, Si(R²)₂-Si(R²)₂, C=O, O, S, S=O, SO₂, NR², PR² oder P(=O)R²;
- Ar¹: ist ein aromatisches oder heteroaromatisches Ringsystem mit 6 bis 40 aromatischen Ringatomen, das mit einem oder mehreren Resten R¹ substituiert sein kann;
- R¹: ist bei jedem Auftreten gleich oder verschieden H, D, F, Cl, Br, I, C(=O)R³, CN, Si(R³)₃, P(=O)(R³)₂, S(=O)R³, S(=O)₂R³, eine gerad-kettige Alkyl- oder Alkoxygruppe mit 1 bis 20 C-Atomen oder eine verzweigte oder cyclische Alkyl- oder Alkoxygruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R³ substituiert sein können und wobei eine oder mehrere CH₂-Gruppen in den oben genannten Gruppen durch -R³C=CR³-, -C≡C-, Si(R³)₂, C=O, C=NR³, -C(=O)O-, -C(=O)NR³-, NR³, P(=O)(R³) -O-, -S-, SO oder SO₂ ersetzt sein können und wobei ein oder mehrere H-Atome in den oben genannten Gruppen durch D, F, Cl, Br, I oder CN ersetzt sein können, oder ein aromatisches oder heteroaromatisches Ringsystem mit 5 bis 30 aromatischen Ringatomen, das jeweils durch einen oder mehrere Reste R³ substituiert sein kann, oder eine Aryloxy- oder Heteroaryloxygruppe mit 5 bis 30 aromatischen Ringatomen, die durch einen oder mehrere Reste R³ substituiert sein kann;
- R²: ist bei jedem Auftreten gleich oder verschieden H, D, F, Cl, Br, I, C(=O)R³, CN, Si(R³)₃, N(R³)₂, P(=O)(R³)₂, S(=O)R³, S(=O)₂R³, eine geradkettige Alkyl- oder Alkoxygruppe mit 1 bis 20 C-Atomen oder eine verzweigte oder cyclische Alkyl- oder Alkoxygruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R³ substituiert sein können und wobei eine oder mehrere CH₂-Gruppen in den oben genannten Gruppen durch -R³C=CR³-, -C≡C-, Si(R³)₂, C=O, C=NR³, -C(=O)O-, -C(=O)NR³-, NR³, P(=O)(R³), -O-, -S-, SO oder SO₂ ersetzt sein können und wobei ein oder mehrere H-Atome in den oben genannten Gruppen durch D, F, Cl, Br, I oder CN ersetzt sein können, oder ein aromatisches oder heteroaromatisches Ringsystem mit 5 bis 30 aromatischen Ring-atomen, das jeweils durch einen oder mehrere Reste R³ substituiert sein kann, oder eine Aryloxy- oder Heteroaryloxygruppe mit 5 bis 30 aromatischen Ringatomen, die durch einen oder mehrere Reste R³ substituiert sein kann, wobei zwei oder mehr Reste R² miteinander verknüpft sein können und einen Ring bilden können;
- R³: ist bei jedem Auftreten gleich oder verschieden H, D, F, Cl, Br, I, C(=O)R⁴, CN, Si(R⁴)₃, N(R⁴)₂, P(=O)(R⁴)₂, S(=O)R⁴, S(=O)₂R⁴, eine geradkettige Alkyl- oder Alkoxygruppe mit 1 bis 20 C-Atomen oder eine verzweigte oder cyclische Alkyl- oder Alkoxygruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R⁴ substituiert sein können und wobei eine oder mehrere CH₂-Gruppen in den oben genannten Gruppen durch -R⁴C=CR⁴-, -C≡C-, Si(R⁴)₂, C=O, C=NR⁴, -C(=O)O-, -C(=O)NR⁴-, NR⁴, P(=O)(R⁴), -O-, -S-, SO oder SO₂ ersetzt sein können und wobei ein oder mehrere H-Atome in den oben genannten Gruppen durch D, F, Cl, Br, I oder CN ersetzt sein können, oder ein aromatisches oder heteroaromatisches Ringsystem mit 5 bis 30 aromatischen Ring-atomen, das jeweils durch einen oder mehrere Reste R⁴ substituiert sein kann, oder eine Aryloxy- oder Heteroaryloxygruppe mit 5 bis 30 aromatischen Ringatomen, die durch einen oder mehrere Reste R⁴ substituiert sein kann;
- R⁴: ist bei jedem Auftreten gleich oder verschieden H, D, F oder ein aliphatischer, aromatischer oder heteroaromatischer organischer Rest mit 1 bis 20 C-Atomen, in dem auch ein oder mehrere H-Atome durch D oder F ersetzt sein können;
wobei der Fall ausgeschlossen ist, bei dem an beide Bindungen A und A' jeweils ein aromatischer oder heteroaromatischer Sechsring ankondensiert ist und kein weiterer aromatischer oder heteroaromatischer Sechsring an eine der anderen vier Bindungen gewählt aus den Bindungen B, C, B' und C' ankondensiert ist.

In der Formel (I) sind die Bindungen A, B, C, A', B' und C' zur besseren Deutlichkeit mit Pfeilen bezeichnet.

Unter der Formulierung, dass an eine Bindung wie beispielsweise die Bindung A ein aromatischer oder heteroaromatischer Sechsring ankondensiert ist, wird im Sinne der vorliegenden Anmeldung verstanden, dass die folgende Struktur entsteht: 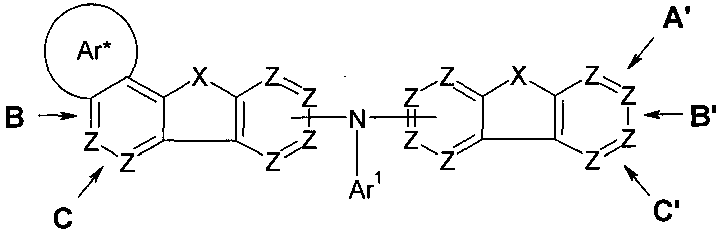 wobei Ar* ein aromatischer oder heteroaromatischer Sechsring ist, der wie oben gezeigt die zwei Ringatome der Bindung A enthält.

Im Fall, dass Ar* eine unsubstituierte Phenylgruppe darstellt, liegt dann beispielsweise die folgende Struktur vor:

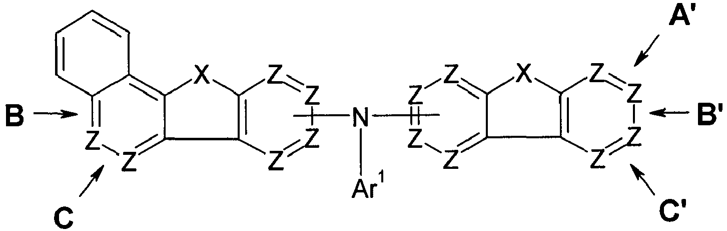

Es folgen allgemeine Definitionen für chemische Gruppen im Rahmen der vorliegenden Anmeldung:
Eine Arylgruppe im Sinne dieser Erfindung enthält 6 bis 60 aromatische Ringatome; eine Heteroarylgruppe im Sinne dieser Erfindung enthält 5 bis 60 aromatische Ringatome, von denen mindestens eines ein Heteroatom darstellt. Die Heteroatome sind bevorzugt ausgewählt aus N, O und S. Dies stellt die grundlegende Definition dar. Werden in der Beschreibung der vorliegenden Erfindung andere Bevorzugungen angegeben, beispielsweise bezüglich der Zahl der aromatischen Ringatome oder der enthaltenen Heteroatome, so gelten diese.

Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin oder Thiophen, oder ein kondensierter (annellierter) aromatischer bzw. heteroaromatischer Polycyclus, beispielsweise Naphthalin, Phenanthren, Chinolin oder Carbazol verstanden. Ein kondensierter (annellierter) aromatischer bzw. heteroaromatischer Polycyclus besteht im Sinne der vorliegenden Anmeldung aus zwei oder mehr miteinander kondensierten einfachen aromatischen bzw. heteroaromatischen Cyclen.

Unter einer Aryl- oder Heteroarylgruppe, die jeweils mit den oben genannten Resten substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Unter einer Aryloxygruppe gemäß der Definition der vorliegenden Erfindung wird eine Arylgruppe, wie oben definiert, verstanden, welche über ein Sauerstoffatom gebunden ist. Eine analoge Definition gilt für Heteroaryloxygruppen.

Ein aromatisches Ringsystem im Sinne dieser Erfindung enthält 6 bis 60 C-Atome im Ringsystem. Ein heteroaromatisches Ringsystem im Sinne dieser Erfindung enthält 5 bis 60 aromatische Ringatome, von denen mindestens eines ein Heteroatom darstellt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein sp³-hybridisiertes C-, Si-, N- oder O-Atom, ein sp²-hybridisiertes C- oder N-Atom oder ein sp-hybridisiertes C-Atom, verbunden sein können. So sollen beispielsweise auch Systeme wie 9,9'-Spirobifluoren, 9,9'-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine lineare oder cyclische Alkyl-, Alkenyl- oder Alkinylgruppe oder durch eine Silylgruppe verbunden sind. Weiterhin werden auch Systeme, in denen zwei oder mehr Aryl- oder Heteroarylgruppen über Einfachbindungen miteinander verknüpft sind, als aromatische oder heteroaromatische Ringsysteme im Sinne dieser Erfindung verstanden, wie beispielsweise Systeme wie Biphenyl, Terphenyl oder Diphenyltriazin.

Unter einem aromatischen oder heteroaromatischen Ringsystem mit 5 - 60 aromatischen Ringatomen, welches noch jeweils mit Resten wie oben definiert substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Benzphenanthren, Pyren, Chrysen, Perylen, Fluoranthen, Naphthacen, Pentacen, Benzpyren, Biphenyl, Biphenylen, Terphenyl, Terphenylen, Quaterphenyl, Fluoren, Spirobifluoren, Dihydrophenanthren, Dihydropyren, Tetrahydropyren, cis- oder trans-Indenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Kombinationen dieser Gruppen.

Im Rahmen der vorliegenden Erfindung werden unter einer geradkettigen Alkylgruppe mit 1 bis 40 C-Atomen bzw. einer verzweigten oder cyclischen Alkylgruppe mit 3 bis 40 C-Atomen bzw. einer Alkenyl- oder Alkinylgruppe mit 2 bis 40 C-Atomen, in der auch einzelne H-Atome oder CH₂-Gruppen durch die oben bei der Definition der Reste genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, neoPentyl, n-Hexyl, Cyclohexyl, neo-Hexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden. Unter einer Alkoxy- oder Thioalkylgruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy, 2,2,2-Trifluorethoxy, Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden.

Unter der Formulierung, dass zwei oder mehr Reste miteinander einen Ring bilden können, soll im Rahmen der vorliegenden Anmeldung unter anderem verstanden werden, dass die beiden Reste miteinander durch eine chemische Bindung verknüpft sind. Dies wird durch das folgende Schema verdeutlicht:

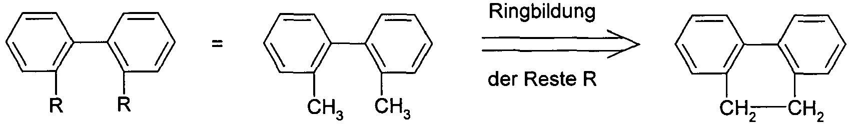

Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

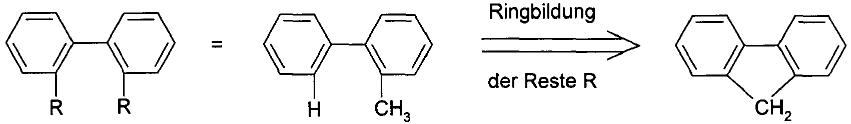

Es ist bevorzugt, dass an keine der beiden Bindungen A und A' in Formel (I) ein aromatischer oder heteroaromatischer Sechsring ankondensiert ist.

Weiterhin ist bevorzugt, dass an mindestens eine der beiden Bindungen gewählt aus Bindungen B und C und an mindestens eine der beiden Bindungen gewählt aus Bindungen B' und C' jeweils ein aromatischer oder heteroaromatischer Sechsring ankondensiert ist, besonders bevorzugt ein aromatischer Sechsring, wobei der aromatische oder heteroaromatische Sechsring mit einem oder mehreren Resten R¹ substituiert sein kann.

Es ist für Verbindungen der Formel (I) bevorzugt, dass die Gruppe X bei jedem Auftreten gleich oder verschieden gewählt ist aus BR², C(R²)₂, Si(R²)₂, O, S und NR². Besonders bevorzugt ist X gleich C(R²)₂.

Weiterhin ist es für Verbindungen der Formel (I) bevorzugt, dass nicht mehr als drei Gruppen Z pro Sechsring gleich N sind. Weiterhin bevorzugt sind nicht mehr als zwei benachbarte Gruppen Z gleich N.

Es ist für Verbindungen der Formel (I) allgemein bevorzugt, dass Z gleich CR¹ ist.

Weiterhin ist es bevorzugt, dass Ar¹ gewählt ist aus aromatischen oder heteroaromatischen Ringsystemen mit 6 bis 24 aromatischen Ringatomen, die mit einem oder mehreren Resten R¹ substituiert sein können. Besonders bevorzugt ist Ar¹ gewählt aus aromatischen Ringsystemen mit 6 bis 18 aromatischen Ringatomen, die mit einem oder mehreren Resten R¹ substituiert sein können. Ganz besonders bevorzugt ist Ar¹ gewählt aus Phenyl, Naphthyl, Biphenyl, Terphenyl, Fluorenyl, Monobenzofluorenyl, Dibenzofluorenyl, Spirobifluorenyl, Indenofluorenyl, Dibenzofuranyl, Carbazolyl und Dibenzothiophenyl, die jeweils mit einem oder mehreren Resten R¹ substituiert sein können.

Weiterhin ist es bevorzugt, dass Ar¹ keine Gruppe der Formel 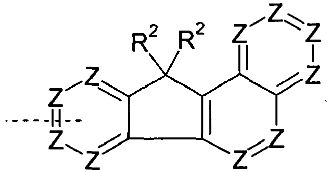 darstellt,
wobei Z, R¹ und R² definiert sind wie oben allgemein für Formel (I) angegeben; und
wobei die gestrichelte Linie die Bindung an das Stickstoffatom bezeichnet.

Bevorzugte Gruppen Ar¹ sind die im Folgenden abgebildeten Gruppen der Formeln (Ar¹-1) bis (Ar¹-34)

| | |
|---|---|
| 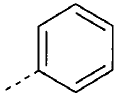 | 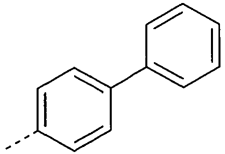 |
| Ar¹-1) | (Ar¹-2) |
| 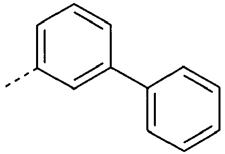 | 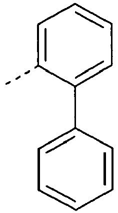 |
| (Ar¹-3) | (Ar¹-4) |
| 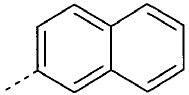 | 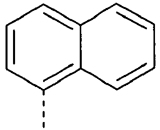 |
| (Ar¹-5) | (Ar¹-6) |
| 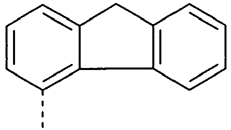 | 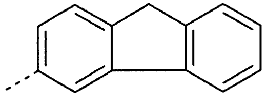 |
| (Ar¹-7) | (Ar¹-8) |
| 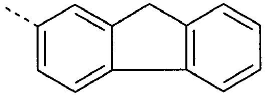 | 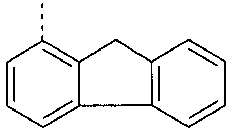 |
| (Ar¹-9) | (Ar¹-10) |
| 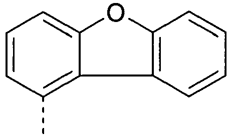 | 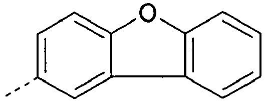 |
| (Ar¹-11) | (Ar¹-12) |
| 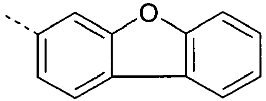 | 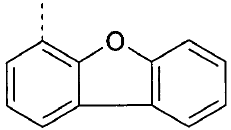 |
| (Ar¹-13) | (Ar¹-14) |
| 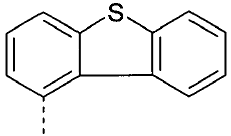 | 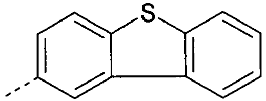 |
| (Ar¹-15) | (Ar¹-16) |
| 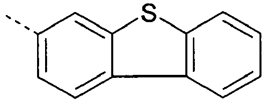 | 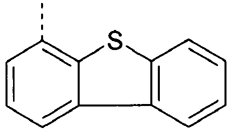 |
| (Ar¹-17) | (Ar¹-18) |
| 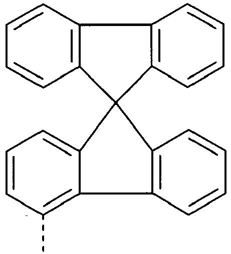 | 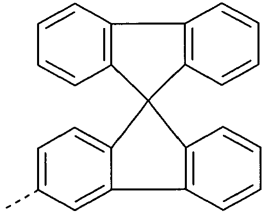 |
| (Ar¹-19) | (Ar¹-20) |
| 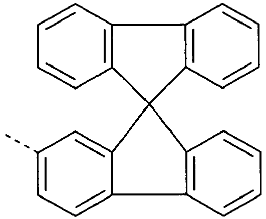 | 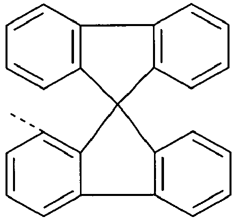 |
| (Ar¹-21) | (Ar¹-22) |
| 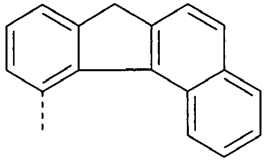 | 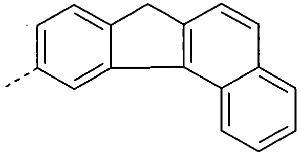 |
| (Ar¹-23) | (Ar¹-24) |
| 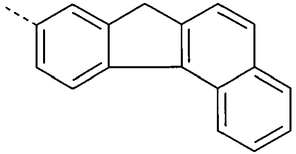 | 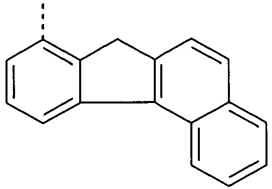 |
| (Ar¹-25) | (Ar¹-26) |
| 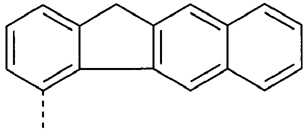 | 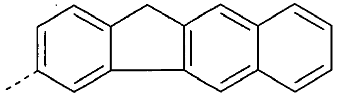 |
| (Ar¹-27) | (Ar¹-28) |
| 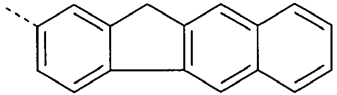 | 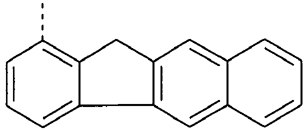 |
| (Ar¹-29) | (Ar¹-30) |
| 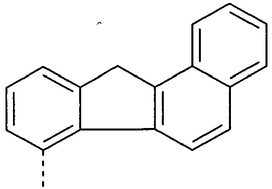 | 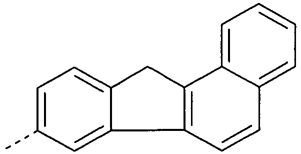 |
| (Ar¹-31) | (Ar¹-32) |
| 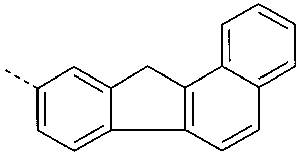 | 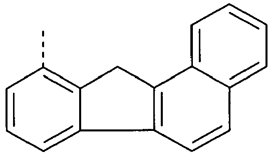 |
| (Ar¹-33) | (Ar¹-34) |

wobei die gestrichelte Linie die Bindung an den Rest der Verbindung bezeichnet und die Gruppen an freien Positionen mit einem Rest R¹ substituiert sein können.

Weiterhin bevorzugt sind R¹ und R² bei jedem Auftreten gleich oder verschieden H, D, F, CN, Si(R³)₃, eine geradkettige Alkyl- oder Alkoxygruppe mit 1 bis 20 C-Atomen oder eine verzweigte oder cyclische Alkyl- oder Alkoxygruppe mit 3 bis 20 C-Atomen, wobei die oben genannten Gruppen jeweils mit einem oder mehreren Resten R³ substituiert sein können und wobei in den oben genannten Gruppen eine oder mehrere CH₂-Gruppen durch -C≡C-, -R³C=CR³-, Si(R³)₂, C=O, C=NR³,-NR³-, -O-, -S-, -C(=O)O- oder -C(=O)NR³- ersetzt sein können, oder ein aromatisches oder heteroaromatisches Ringsystem mit 5 bis 20 aromatischen Ringatomen, das jeweils mit einem oder mehreren Resten R³ substituiert sein kann, wobei zwei oder mehr Reste R² miteinander verknüpft sein können und einen Ring bilden können.

Besonders bevorzugt ist R² gewählt aus geradkettigen Alkylgruppen mit 1 bis 10 C-Atomen oder verzweigten oder cyclischen Alkylgruppen mit 3 bis 10 C-Atomen, wobei die oben genannten Gruppen jeweils mit einem oder mehreren Resten R³ substituiert sein können, oder aus aromatischen oder heteroaromatischen Ringsystemen mit 5 bis 20 aromatischen Ringatomen, die jeweils mit einem oder mehreren Resten R³ substituiert sein können.

Weiterhin ist es bevorzugt, dass Reste R², die an dasselbe Atom der Gruppe X binden, beispielsweise die zwei Reste R² einer Gruppe C(R²)₂, miteinander zu einem Ring verbunden sind. Bevorzugt ist dies ein Fünfring oder ein Sechsring, wobei die Gruppen bevorzugt Arylgruppen oder Alkylgruppen sind. Ganz besonders bevorzugt ist die Gruppe X in diesen Fällen gewählt aus Gruppen der folgenden Formeln (X-1) bis (X-8) 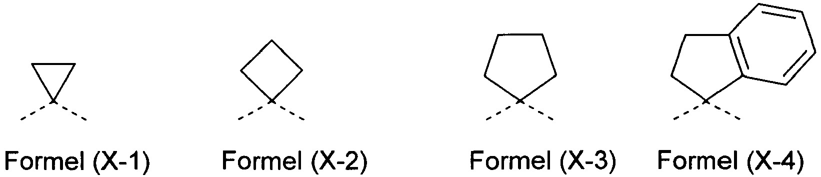 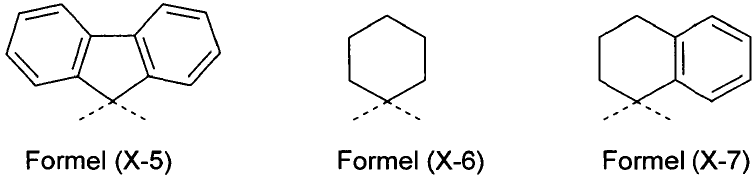 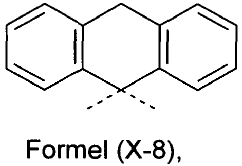 wobei die abgebildeten Gruppen in den unsubstituiert dargestellten Positionen wahlweise mit einem oder mehreren Resten R³ substituiert sein können, und wobei die gestrichelten Linien Bindungen an den Rest der Verbindung darstellen.

Weiterhin bevorzugt ist R³ bei jedem Auftreten gleich oder verschieden H, D, F, CN, Si(R⁴)₃, eine geradkettige Alkyl- oder Alkoxygruppe mit 1 bis 20 C-Atomen oder eine verzweigte oder cyclische Alkyl- oder Alkoxygruppe mit 3 bis 20 C-Atomen, wobei die oben genannten Gruppen jeweils mit einem oder mehreren Resten R⁴ substituiert sein können und wobei in den oben genannten Gruppen eine oder mehrere CH₂-Gruppen durch -C≡C-,-R⁴C=CR⁴-, Si(R⁴)₂, C=O, C=NR⁴, -NR⁴-, -O-, -S-, -C(=O)O- oder-C(=O)NR⁴- ersetzt sein können, oder ein aromatisches oder heteroaromatisches Ringsystem mit 5 bis 20 aromatischen Ringatomen, das jeweils mit einem oder mehreren Resten R⁴ substituiert sein kann.

Weiterhin ist es bevorzugt, dass die Anbindungsposition der Gruppe NAr¹ an die Fluorenylgruppen in para-Position zur direkten Bindung zwischen den beiden aromatischen Sechsringen des Fluorenylgrundkörpers liegt. Entsprechend sind Verbindungen der folgenden Formel (I-A) bevorzugt: 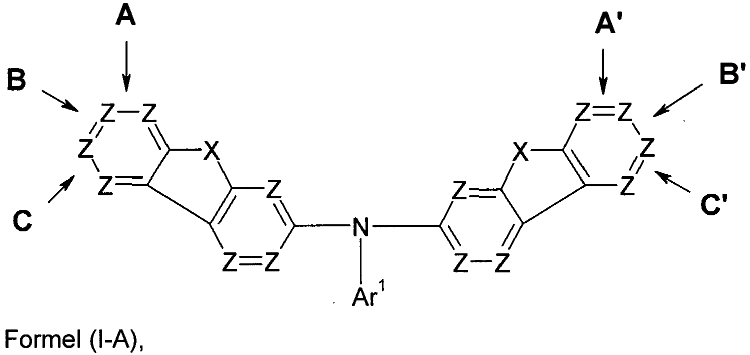 wobei die auftretenden Gruppen wie oben definiert sind,
wobei an mindestens eine der drei Bindungen gewählt aus den Bindungen A, B und C und an mindestens eine der drei Bindungen gewählt aus den Bindungen A', B' und C' ist ein aromatischer oder heteroaromatischer Sechsring ankondensiert, der mit einem oder mehreren Resten R¹ substituiert sein kann,
wobei der Fall ausgeschlossen ist, bei dem an beide Bindungen A und A' jeweils ein aromatischer oder heteroaromatischer Sechsring ankondensiert ist und kein weiterer aromatischer oder heteroaromatischer Sechsring an eine der anderen vier Bindungen gewählt aus den Bindungen B, C, B' und C' ankondensiert ist.

Es gelten auch für diese Ausführungsform die oben angegebenen bevorzugten Ausführungsformen von Gruppen als bevorzugt.

Insbesondere ist X in Verbindungen der Formel (I-A) bevorzugt bei jedem Auftreten gleich oder verschieden gewählt aus BR², C(R²)₂, Si(R²)₂, O, S und NR², besonders bevorzugt ist X gleich C(R²)₂.

Weiterhin ist es bevorzugt, dass mindestens eine Gruppe R¹ in der Verbindung gewählt ist aus geradkettigen Alkylgruppen mit 1 bis 10 C-Atomen oder verzweigten oder cyclischen Alkylgruppen mit 3 bis 10 C-Atomen, die mit einem oder mehreren Resten R³ substituiert sein können, oder aromatischen oder heteroaromatischen Ringsystemen mit 5 bis 20 aromatischen Ringatomen, bevorzugt aus aromatischen Ringsystemen mit 6 bis 20 aromatischen Ringatomen, wobei die Ringsysteme jeweils mit einem oder mehreren Resten R³ substituiert sein können.

Weiterhin ist es bevorzugt, dass mindestens eine Gruppe R¹, die an einen der äußeren Ringe der Fluorenylgruppe bindet, gewählt ist aus geradkettigen Alkylgruppen mit 1 bis 10 C-Atomen oder verzweigten oder cyclischen Alkylgruppen mit 3 bis 10 C-Atomen, die mit einem oder mehreren Resten R³ substituiert sein können, oder aromatischen oder heteroaromatischen Ringsystemen mit 6 bis 18 aromatischen Ringatomen, die jeweils mit einem oder mehreren Resten R³ substituiert sein können. Unter "äußerem Ring der Fluorenylgruppe" wird derjenige Ring von den beiden Ringen einer Fluorenylgruppe verstanden, der nicht an die Gruppe NAr¹ bindet. Dies ist besonders bevorzugt, wenn Ar¹ keine Benzofluorengruppe darstellt, insbesondere wenn Ar¹ nicht gewählt ist aus Gruppen der Formeln (Ar¹-23) bis (Ar¹-34).

Wenn Ar¹ eine Benzofluorengruppe ist, insbesondere eine Gruppe gewählt aus Gruppen der Formeln (Ar¹-23) bis (Ar¹-34) ist, kann es bevorzugt sein, dass keine Gruppe R¹ in der Verbindung vorhanden ist, die eine Alkylgruppe oder ein aromatisches oder heteroaromatisches Ringsystem darstellt. Es kann weiterhin in diesem Fall bevorzugt sein, dass alle Gruppen R¹ gleich H sind.

Durch dieses Substitutionsmuster werden besonders vorteilhafte Emissionseigenschaften der erfindungsgemäßen Verbindung erreicht.

Besonders bevorzugt ist die genannte Gruppe R¹ in para-Position zur direkten Bindung zwischen den beiden aromatischen Sechsringen des Fluorenylgrundkörpers an den Fluorenylgrundkörper gebunden, so dass die Verbindungen der Formel (I) der folgenden Struktur der Formel (I-B) entsprechen: 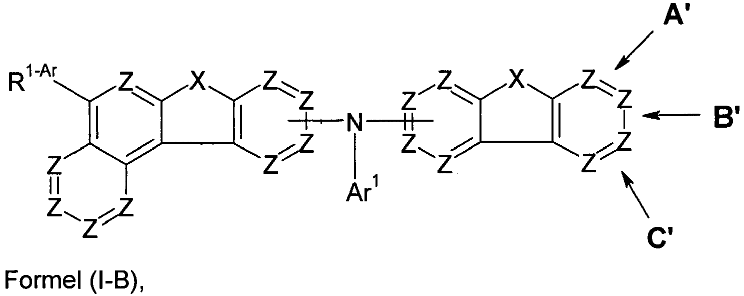 wobei die auftretenden Gruppen wie oben definiert sind und
- R^{1-Ar}: gewählt ist aus geradkettigen Alkylgruppen mit 1 bis 10 C-Atomen oder verzweigten oder cyclischen Alkylgruppen mit 3 bis 10 C-Atomen, die mit einem oder mehreren Resten R³ substituiert sein können, oder aromatischen oder heteroaromatischen Ringsystemen mit 6 bis 18 aromatischen Ringatomen, die mit einem oder mehreren Resten R³ substituiert sein können; und
wobei an mindestens eine der drei Bindungen gewählt aus den Bindungen A', B' und C' ein aromatischer oder heteroaromatischer Sechsring ankondensiert ist, der mit einem oder mehreren Resten R¹ substituiert sein kann.

Ganz besonders bevorzugte Gruppen R^{1-Ar}, wie oben definiert, sind ausgewählt aus Methyl, Ethyl, Propyl, Butyl, Isopropyl, Isobutyl, tert-Butyl, Cyclohexyl, Phenyl, Naphthyl, Biphenyl, Terphenyl, Fluorenyl, Monobenzofluorenyl, Dibenzofluorenyl, Spirobifluorenyl, Indenofluorenyl, Dibenzofuranyl, Carbazolyl und Dibenzothiophenyl, die jeweils mit einem oder mehreren Resten R³ substituiert sein können.

Bevorzugte Gruppen R^{1-Ar} sind die im Folgenden abgebildeten Gruppen der Formeln (R^{1-Ar}-1) bis (R^{1-Ar}-27)

| | |
|---|---|
| 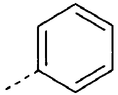 | 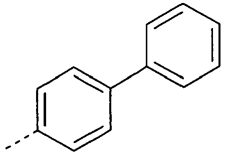 |
| (R^{1-Ar} -1) | (R^{1-Ar}-2) |
| 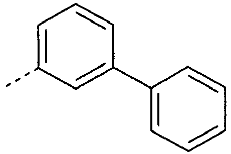 | 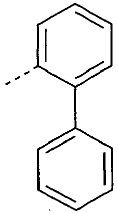 |
| (R^{1-Ar} -3) | (R^{1-Ar} -4) |
| 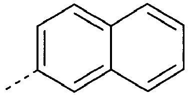 | 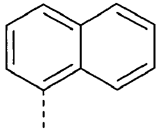 |
| (R^{1-Ar} -5) | (R^{1-Ar}-6) |
| 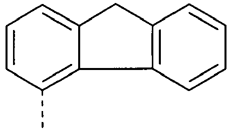 | 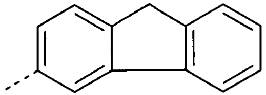 |
| (R^{1-Ar} -7) | (R^{1-Ar}-8) |
| 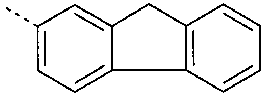 | 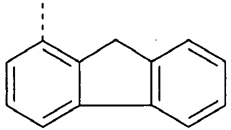 |
| (R^{1-Ar}-9) | (R^{1-Ar}-10) |
| 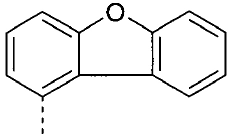 | 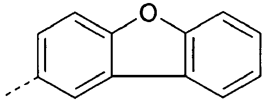 |
| (R^{1-Ar}-11) | (R^{1-Ar} -12) |
| 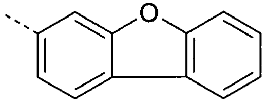 | 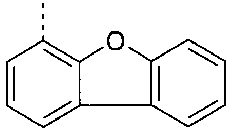 |
| (R^{1-Ar} -13) | (R^{1-Ar} -14) |
| 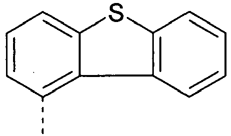 | 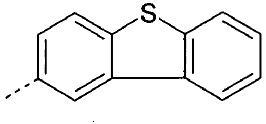 |
| (R^{1-Ar} -15) | (R^{1-Ar} -16) |
| 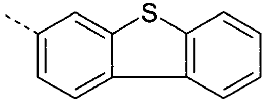 | 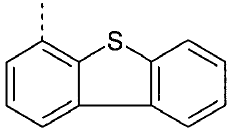 |
| (R^{1-Ar}-17) | (R^{1-Ar}-18) |
| 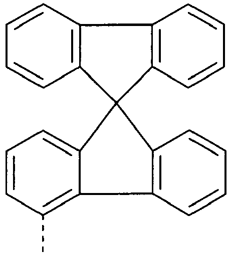 | 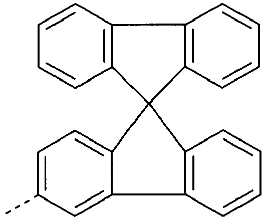 |
| (R^{1-Ar}-19) | (R^{1-Ar} -20) |
| 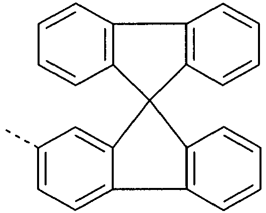 | 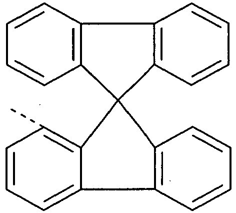 |
| (R^{1-Ar} -21) | (R^{1-Ar}-22) |
| 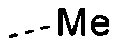 | 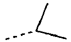 |
| (R^{1-Ar} -23) | (R^{1-Ar} -24) |
| 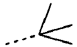 | 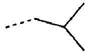 |
| (R^{1-Ar} -25) | (R^{1-Ar} -26) |
| 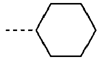 | |
| (R^{1-Ar} -27) | |

wobei die gestrichelte Linie die Bindung an den Rest der Verbindung bezeichnet und die Gruppen an freien Positionen mit einem Rest R³ substituiert sein können.

Weiterhin bevorzugt enthält die Verbindung der Formel (I) keine kondensierte Aryl- oder Heteroarylgruppe mit mehr als 18 aromatischen Ringatomen in der kondensierten Gruppe. Besonders bevorzugt enthält sie keine kondensierte Aryl- oder Heteroarylgruppe mit mehr als 14 aromatischen Ringatomen in der kondensierten Gruppe, ganz besonders bevorzugt keine kondensierte Aryl- oder Heteroarylgruppe mit mehr als 10 aromatischen Ringatomen in der kondensierten Gruppe.

Weiterhin bevorzugt enthält die Verbindung der Formel (I) zusätzlich zu der Arylaminogruppe -N(Ar¹)- keine weitere Arylaminogruppe. Unter einer Arylaminogruppe wird dabei eine Gruppe verstanden, welche mindestens eine Aryl- oder Heteroarylgruppe gebunden an ein Amino-Stickstoffatom enthält. Besonders bevorzugt enthält die Verbindung der Formel (I) zusätzlich zu der Arylaminogruppe -N(Ar¹)- keine weitere Aminogruppe.

Besonders bevorzugte Ausführungsformen von Verbindungen der Formel (I) entsprechen den folgenden Formeln (I-1) bis (I-5) 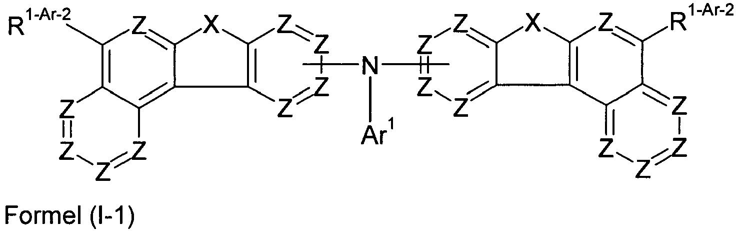 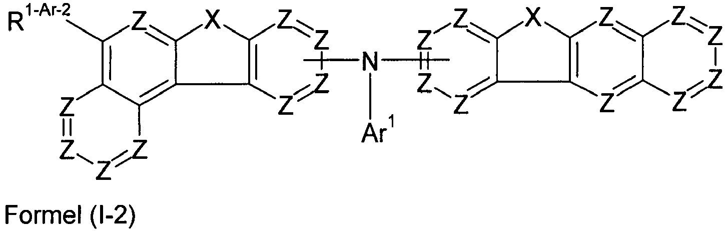 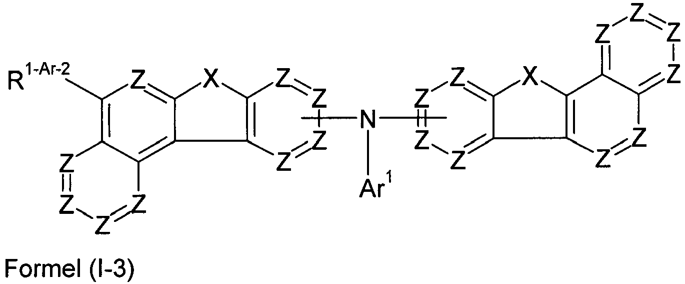 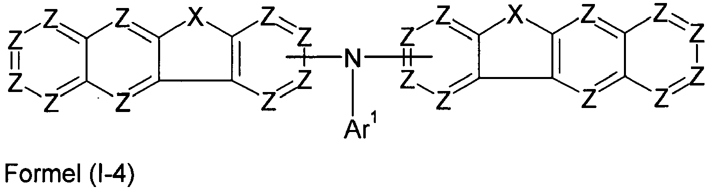 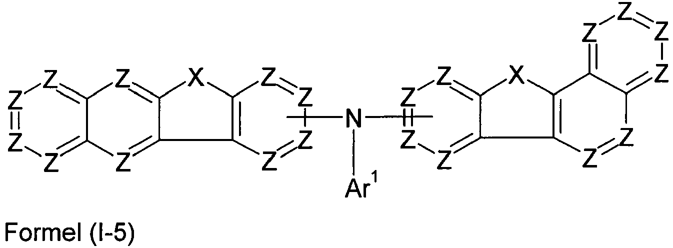 wobei die auftretenden Gruppen definiert sind wie oben, und wobei
- R^{1-Ar-2}: bei jedem Auftreten gleich oder verschieden gewählt ist aus H oder geradkettigen Alkylgruppen mit 1 bis 10 C-Atomen oder verzweigten oder cyclischen Alkylgruppen mit 3 bis 10 C-Atomen, die mit einem oder mehreren Resten R³ substituiert sein können, oder aromatischen oder heteroaromatischen Ringsystemen mit 6 bis 18 aromatischen Ringatomen, die mit einem oder mehreren Resten R³ substituiert sein können.

Ganz besonders bevorzugt ist von den obenstehenden Formeln Formel (I-1).

Es gelten für die Verbindungen der Formeln (I-1) bis (I-5) dieselben Ausführungsformen von Gruppen als bevorzugt wie oben allgemein für Verbindungen der Formel (I) angegeben. Insbesondere ist X bevorzugt C(R²)₂. Weiterhin insbesondere bevorzugt ist die Gruppe NAr¹ in para-Position zur direkten Bindung zwischen den beiden aromatischen Sechsringen des Fluorengrundkörpers gebunden, entsprechend der in Formel (I-A) gezeigten Ausführungsform.

Weiterhin ist R^{1-Ar-2} bevorzugt ausgewählt aus H und den Formeln (R^{1-Ar}-1) bis (R^{1-Ar}-27).

Besonders bevorzugte Ausführungsformen von Verbindungen der Formel (I) enstsprechen den in der folgenden Tabelle angegebenen Formeln, wobei die Grundstrukturen diejenigen der Formeln (I-1) bis (I-5) sind, und die auftretenden Gruppen definiert sind wie oben.

| Formel (I- | Grundstruktur | Ar¹ |
|---|---|---|
| 1-1) | Formel (I-1) | (Ar¹-1) |
| 1-2) | " | (Ar¹-2) |
| 1-3) | " | (Ar¹-3) |
| 1-4) | " | (Ar¹-4) |
| 1-5) | " | (Ar¹-5) |
| 1-6) | " | (Ar¹-6) |
| 1-7) | " | (Ar¹-7) |
| 1-8) | " | (Ar¹-8) |
| 1-9) | " | (Ar¹-9) |
| 1-10) | " | (Ar¹-10) |
| 1-11) | " | (Ar¹-11) |
| 1-12) | " | (Ar¹-12) |
| 1-13) | " | (Ar¹-13) |
| 1-14) | " | (Ar¹-14) |
| 1-15) | " | (Ar¹-15) |
| 1-16) | " | (Ar¹-16) |
| 1-17) | " | (Ar¹-17) |
| 1-18) | " | (Ar¹-18) |
| 1-19) | " | (Ar¹-19) |
| 1-20) | " | (Ar¹-20) |
| 1-21) | " | (Ar¹-21) |
| 1-22) | " | (Ar¹-22) |
| 1-23) | " | (Ar¹-23) |
| 1-24) | " | (Ar¹-24) |
| 1-25) | " | (Ar¹-25) |
| 1-26) | " | (Ar¹-26) |
| 1-27) | " | (Ar¹-27) |
| 1-28) | " | (Ar¹-28) |
| 1-29) | " | (Ar¹-29) |
| 1-30) | " | (Ar¹-30) |
| 1-31) | " | (Ar¹-31) |
| 1-32) | " | (Ar¹-32) |
| 1-33) | " | (Ar¹-33) |
| 1-34) | " | (Ar¹-34) |
| 2-1) | Formel (I-2) | (Ar¹-1) |
| 2-2) | " | (Ar¹-2) |
| 2-3) | " | (Ar¹-3) |
| 2-4) | " | (Ar¹-4) |
| 2-5) | " | (Ar¹-5) |
| 2-6) | " | (Ar¹-6) |
| 2-7) | " | (Ar¹-7) |
| 2-8) | " | (Ar¹-8) |
| 2-9) | " | (Ar¹-9) |
| 2-10) | " | (Ar¹-10) |
| 2-11) | " | (Ar¹-11) |
| 2-12) | " | (Ar¹-12) |
| 2-13) | " | (Ar¹-13) |
| 2-14) | " | (Ar¹-14) |
| 2-15) | " | (Ar¹-15) |
| 2-16) | " | (Ar¹-16) |
| 2-17) | " | (Ar¹-17) |
| 2-18) | " | (Ar¹-18) |
| 2-19) | " | (Ar¹-19) |
| 2-20) | " | (Ar¹-20) |
| 2-21) | " | (Ar¹-21) |
| 2-22) | " | (Ar¹-22) |
| 2-23) | " | (Ar¹-23) |
| 2-24) | " | (Ar¹-24) |
| 2-25) | " | (Ar¹-25) |
| 2-26) | " | (Ar¹-26) |
| 2-27) | " | (Ar¹-27) |
| 2-28) | " | (Ar¹-28) |
| 2-29) | " | (Ar¹-29) |
| 2-30) | " | (Ar¹-30) |
| 2-31) | " | (Ar¹-31) |
| 2-32 | " | (Ar¹-32) |
| 2-33) | " | (Ar¹-33) |
| 2-34) | " | (Ar¹-34) |
| 3-1) | Formel (I-3) | (Ar¹-1) |
| 3-2) | " | (Ar¹-2) |
| 3-3) | " | (Ar¹-3) |
| 3-4) | " | (Ar¹-4) |
| 3-5) | " | (Ar¹-5) |
| 3-6) | " | (Ar¹-6) |
| 3-7) | " | (Ar¹-7) |
| 3-8) | " | (Ar¹-8) |
| 3-9) | " | (Ar¹-9) |
| 3-10) | " | (Ar¹-10) |
| 3-11) | " | (Ar¹-11) |
| 3-12) | " | (Ar¹-12) |
| 3-13) | " | (Ar¹-13) |
| 3-14) | " | (Ar¹-14) |
| 3-15) | " | (Ar¹-15) |
| 3-16) | " | (Ar¹-16) |
| 3-17) | " | (Ar¹-17) |
| 3-18) | " | (Ar¹-18) |
| 3-19) | " | (Ar¹-19) |
| 3-20) | " | (Ar¹-20) |
| 3-21 ) | " | (Ar¹-21) |
| 3-22) | " | (Ar¹-22) |
| 3-23) | " | (Ar¹-23) |
| 3-24 ) | " | (Ar¹-24) |
| 3-25) | " | (Ar¹-25) |
| 3-26) | " | (Ar¹-26) |
| 3-27) | " | (Ar¹-27) |
| 3-28) | " | (Ar¹-28) |
| 3-29) | " | (Ar¹-29) |
| 3-30) | " | (Ar¹-30) |
| 3-31 ) | " | (Ar¹-31) |
| 3-32) | " | (Ar¹-32) |
| 3-33) | " | (Ar¹-33) |
| 3-34) | " | (Ar¹-34) |
| 4-1) | Formel (I-4) | (Ar¹-1) |
| 4-2) | " | (Ar¹-2) |
| 4-3) | " | (Ar¹-3) |
| 4-4) | " | (Ar¹-4) |
| 4-5) | " | (Ar¹-5) |
| 4-6) | " | (Ar¹-6) |
| 4-7) | " | (Ar¹-7) |
| 4-8) | " | (Ar¹-8) |
| 4-9) | " | (Ar¹-9) |
| 4-10) | " | (Ar¹-10) |
| 4-11) | " | (Ar¹⁻11) |
| 4-12) | " | (Ar¹-12) |
| 4-13) | " | (Ar¹-13) |
| 4-14) | " | (Ar¹-14) |
| 4-15) | " | (Ar¹-15) |
| 4-16) | " | (Ar¹-16) |
| 4-17) | " | (Ar¹-17) |
| 4-18) | " | (Ar¹-18) |
| 4-19) | " | (Ar¹-19) |
| 4-20) | " | (Ar¹-20) |
| 4-21) | " | (Ar¹-21) |
| 4-22) | " | (Ar¹-22) |
| 4-23) | " | (Ar¹-23) |
| 4-24) | " | (Ar¹-24) |
| 4-25 | " | (Ar¹-25) |
| 4-26) | " | (Ar¹-26) |
| 4-27) | " | (Ar¹-27) |
| 4-28) | " | (Ar¹-28) |
| 4-29) | " | (Ar¹-29) |
| 4-30) | " | (Ar¹-30) |
| 4-31) | " | (Ar¹-31) |
| 4-32) | " | (Ar¹-32) |
| 4-33) | " | (Ar¹-33) |
| 4-34) | " | (Ar¹-34) |
| 5-1) | Formel (I-5) | (Ar¹-1) |
| 5-2) | " | (Ar¹-2) |
| 5-3) | " | (Ar¹-3) |
| 5-4) | " | (Ar¹-4) |
| 5-5) | " | (Ar¹-5) |
| 5-6) | " | (Ar¹-6) |
| 5-7) | " | (Ar¹-7) |
| 5-8) | " | (Ar¹-8) |
| 5-9) | " | (Ar¹-9) |
| 5-10) | " | (Ar¹-10) |
| 5-11) | " | (Ar¹-11) |
| 5-12) | " | (Ar¹-12) |
| 5-13) | " | (Ar¹-13) |
| 5-14) | " | (Ar¹-14) |
| 5-15) | " | (Ar¹-15) |
| 5-16) | " | (Ar¹-16) |
| 5-17) | " | (Ar¹-17) |
| 5-18) | " | (Ar¹-18) |
| 5-19) | " | (Ar¹-19) |
| 5-20) | " | (Ar¹-20) |
| 5-21) | " | (Ar¹-21) |
| 5-22) | " | (Ar¹-22) |
| 5-23) | " | (Ar¹-23) |
| 5-24) | " | (Ar¹-24) |
| 5-25) | " | (Ar¹-25) |
| 5-26) | " | (Ar¹-26) |
| 5-27) | " | (Ar¹-27) |
| 5-28) | " | (Ar¹-28) |
| 5-29) | " | (Ar¹-29) |
| 5-30) | " | (Ar¹-30) |
| 5-31) | " | (Ar¹-31) |
| 5-32) | " | (Ar¹-32) |
| 5-33) | " | (Ar¹-33) |
| 5-34) | " | (Ar¹-34) |

Bevorzugt ist für Verbindungen der obenstehenden Tabelle, dass R^{1-Ar-2} ausgewählt ist aus H und den Formeln (R^{1-Ar}-1) bis (R^{1-Ar}-27).

Folgende Verbindungen sind Beispiele für Verbindungen nach Formel (I):

| | |
|---|---|
| 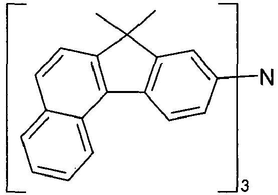 | 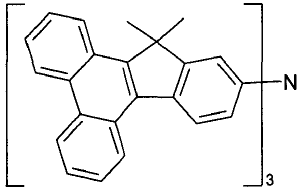 |
| 1 | 2 |
| 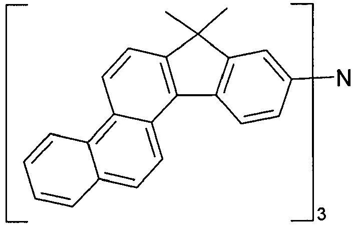 | 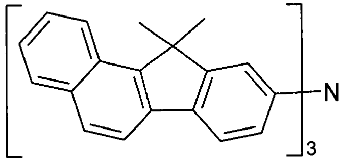 |
| 3 | 4 |
| 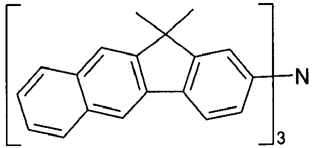 | 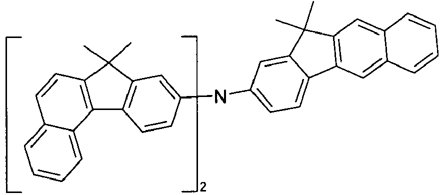 |
| 5 | 6 |
| 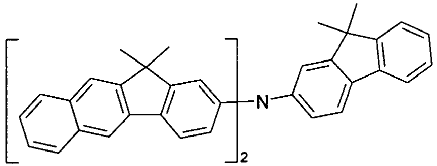 | 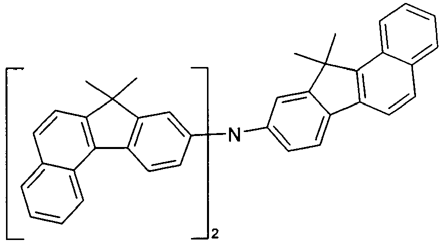 |
| 7 | 8 |
| 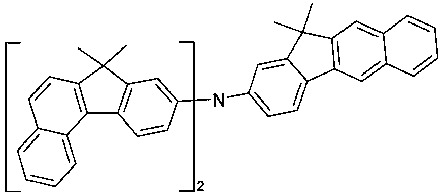 | 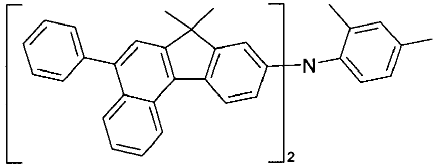 |
| 9 | 10 |
| 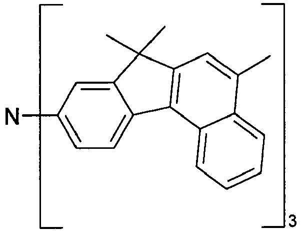 | 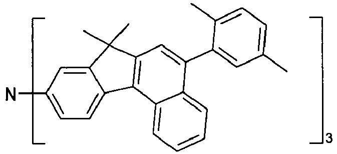 |
| 11 | 12 |
| 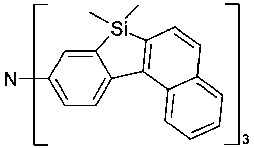 | 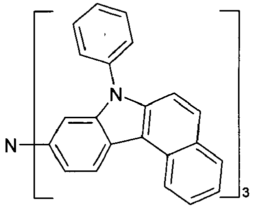 |
| 13 | 14 |
| 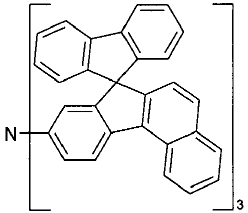 | 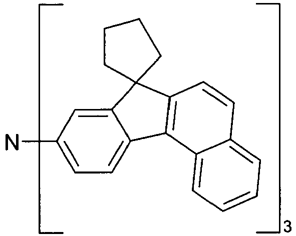 |
| 15 | 16 |
| 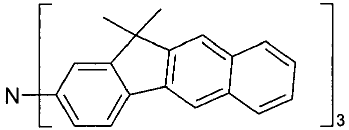 | 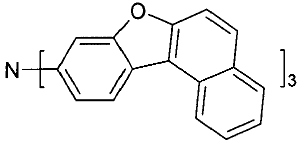 |
| 17 | 18 |
| 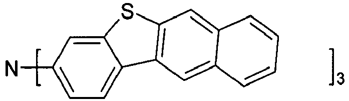 | 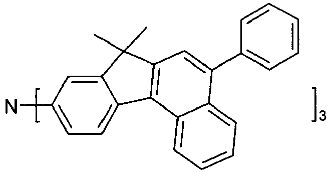 |
| 19 | 20 |
| 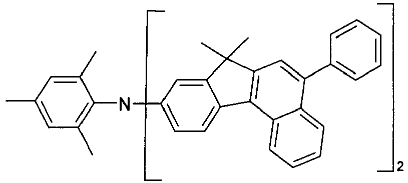 | 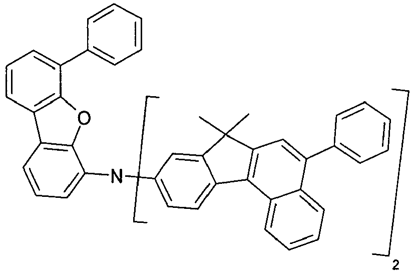 |
| 21 | 22 |
| 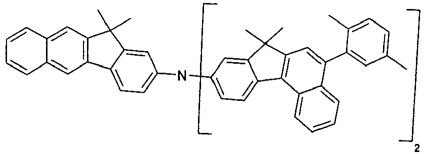 | 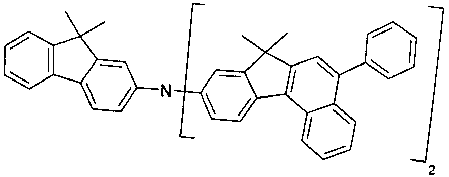 |
| 23 | 24 |
| 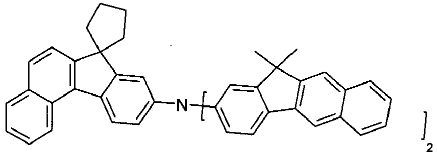 | 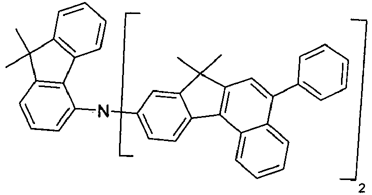 |
| 25 | 26 |
| 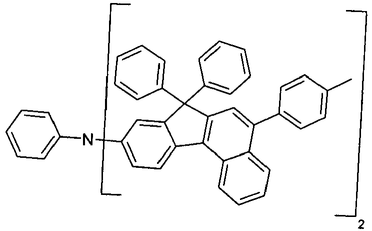 | 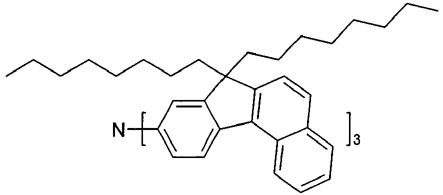 |
| 27 | 28 |
| 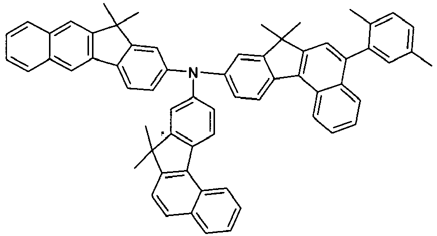 | 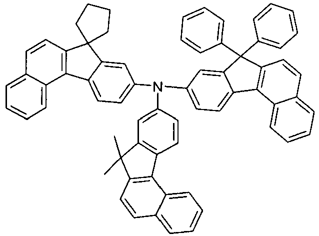 |
| 29 | 30 |
| 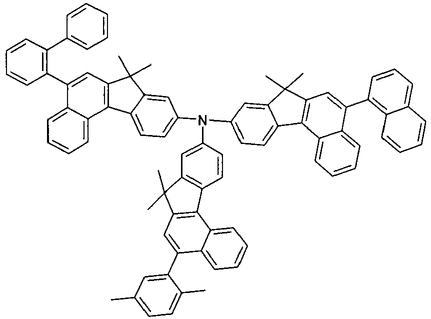 | 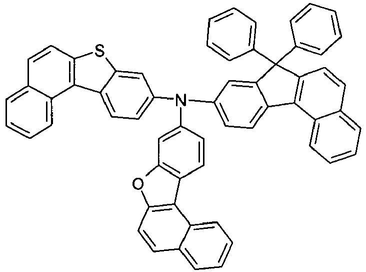 |
| 31 | 32 |
| 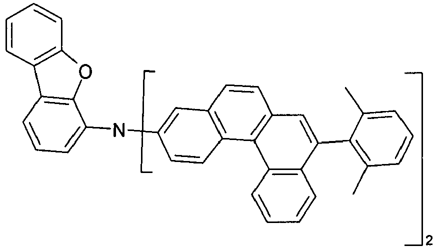 | 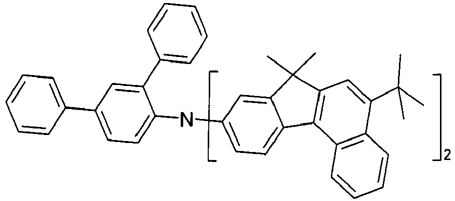 |
| 33 | 34 |
| 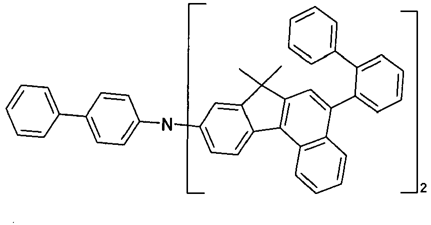 | 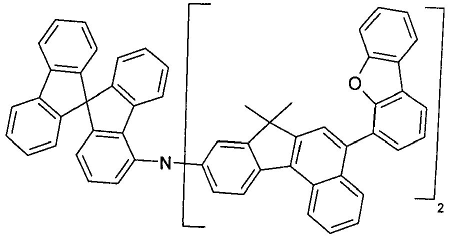 |
| 35 | 36 |
| 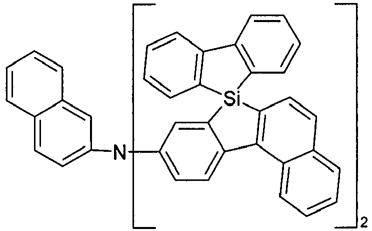 | 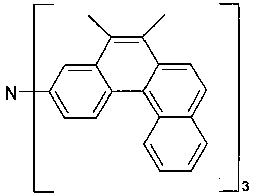 |
| 37 | 38 |
| 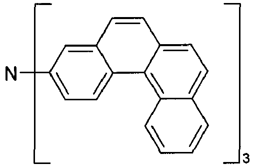 | 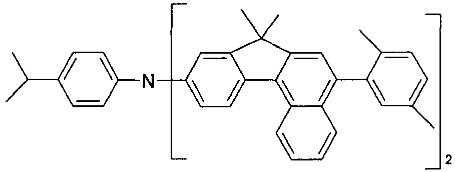 |
| 39 | 40 |
| 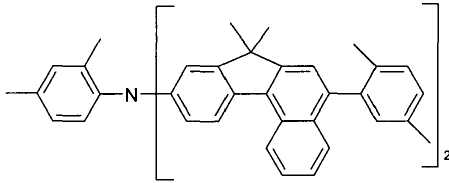 | 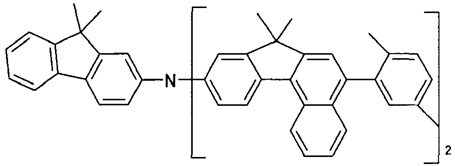 |
| 41 | 42 |
| 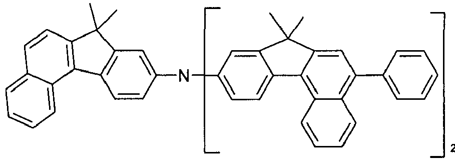 | 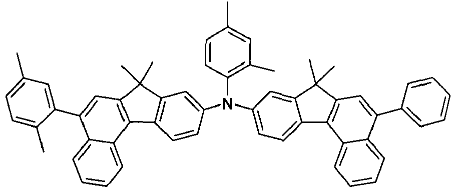 |
| 43 | 44 |

Gegenstand der vorliegenden Anmeldung ist weiterhin ein Verfahren zur Herstellung einer erfindungsgemäßen Verbindung.

Bevorzugt ist ein Verfahren zur Herstellung einer erfindungsgemäßen Verbindung, dadurch gekennzeichnet, dass es mindestens eine übergangsmetallkatalysierte Kupplungsreaktion umfasst. Bevorzugt ist die Kupplungsreaktion ausgewählt aus Buchwald-Kupplungsreaktionen und Suzuki-Kupplungsreaktionen. Weiterhin und bevorzugt in Kombination mit der oben genannten bevorzugten Ausführungform des Verfahrens umfasst das Verfahren eine oder mehrere Ringschlussreaktionen, besonders bevorzugt säure-induzierte Ringschlussreaktionen von tertiären Alkoholen zu Methylenbrücken.

Besonders bevorzugte Ausführungsformen von Verfahren zur Herstellung von erfindungsgemäßen Verbindungen sind im Folgenden dargestellt. Dadurch werden dem Fachmann mögliche Syntheseverfahren vorgeschlagen, mit denen er die erfindungsgemäßen Verbindungen herstellen kann. Der Fachmann ist an die vorgestellten Verfahren jedoch nicht gebunden. Es kann sie nach Bedarf im Rahmen seines allgemeinen Fachwissens abwandeln und anpassen, sofern dies erforderlich ist.

Die erfindungsgemäßen Verbindungen können mit bekannten Syntheseschritten der organischen Chemie hergestellt werden. Bevorzugte Reaktionen sind dabei Cyclisierungsreaktionen, Buchwald-Kupplungen und Suzuki-Kupplungen.

In den folgenden Schemata 1 bis 3 sind drei verschiedene Wege zur Herstellung von erfindungsgemäßen Verbindungen dargestellt. Das Verfahren nach Schema 1 eignet sich insbesondere zur Synthese von erfindungsgemäßen Verbindungen, die eine Aryl-Gruppe gebunden an die Benzofluorenyl-Gruppe enthalten. Das Verfahren nach Schema 3 eignet sich insbesondere zur Herstellung von erfindungsgemäßen Verbindungen, die drei Benzofluoren-Gruppen enthalten.

In den der Schemata 1 bis 3 bezeichnet Ar eine beliebige Aryl- oder Heteroarylgruppe, und Hal bezeichnet eine beliebige reaktive Gruppe, bevorzugt eine Halogengruppe, besonders bevorzugt Brom. Alle gezeigten Verbindungen können wahlweise mit einem oder mehreren organischen Resten substituiert sein.

In dem Syntheseverfahren nach Schema 1 wird zunächst eine Phenyl-Naphthyl-Verbindung hergestellt durch Suzuki-Kupplung eines Phenyl-Derivats mit einem Naphthyl-Derivat, das eine Carbonsäureester-Gruppe trägt, die Vorstufe zur verbrückenden Methylengruppe des Fluorens. Die Carbonsäreester-Gruppe wird durch Addition einer metallorganischen Verbindung zum tertiären Alkohol umgesetzt, der die Ringschlussreaktion zur Methylen-Brücke des Fluorens eingeht. Die weiteren Schritte sind Halogenierung, Kupplung einer Arylgruppe am äußeren Ring des Fluoren-Derivats, erneute Halogenierung und folgende Buchwald-Kupplung. Bei der Buchwald-Kupplung werden je zwei Fluoren-Derivate mit einem Arylamin gekuppelt, wobei die erfindungsgemäße Verbindung entsteht.

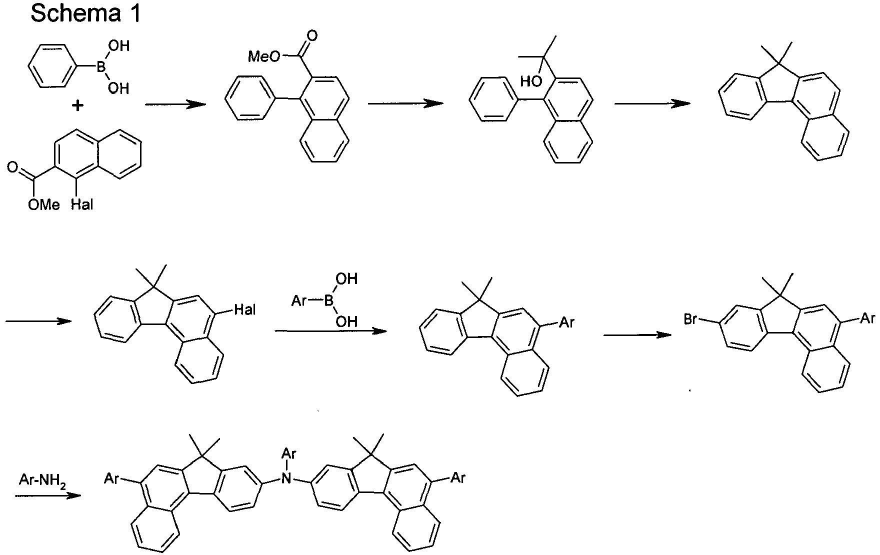

Im Verfahren gemäß Schema 2 wird über eine Suzuki-Kupplung bereits an der Arylamino-Verbindung die Phenyl-Naphthyl-Verbindung hergestellt, die eine Carbonsäureestergruppe als Vorstufe der Methylenbrücke des Benzofluorens enthält. Addition eines metallorganischen Nukleophils und anschließender Ringschluss zur Bildung einer Methylenbrücke führen zur gewünschten erfindungsgemäßen Verbindung.

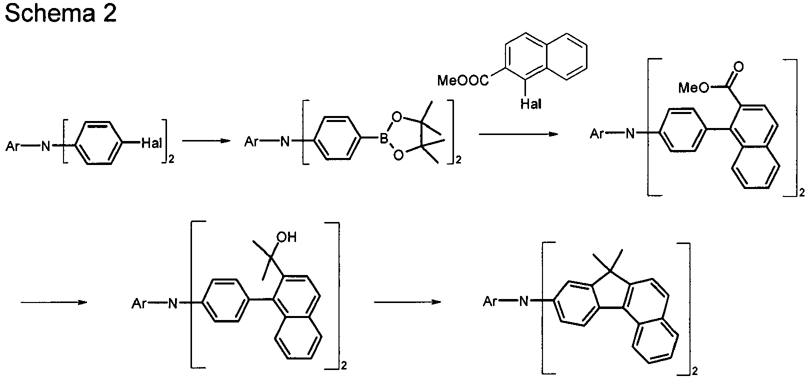

Im Verfahren gemäß Schema 3 wird weitgehend analog zu Schema 2 vorgegangen, mit dem Unterschied, dass das eingesetzte Amin statt zwei drei reaktive Phenylgruppen enthält, so dass insgesamt drei Benzofluorenylgruppen an das zentrale Stickstoffatom gebunden entstehen.

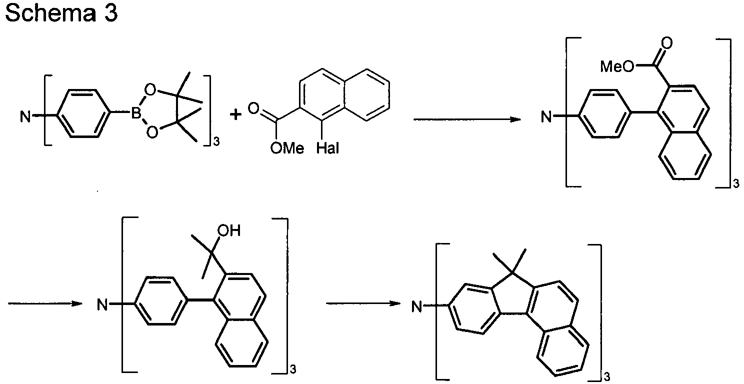

An die oben genannten Syntheseverfahren nach Schemata 1 bis 3 können sich weitere Funktionalisierungsreaktionen anschließen, in denen die erhaltenen erfindungsgemäßen Verbindungen weiter umgesetzt werden.

Die oben beschriebenen erfindungsgemäßen Verbindungen, insbesondere Verbindungen, welche mit reaktiven Abgangsgruppen, wie Brom, lod, Chlor, Boronsäure oder Boronsäureester, substituiert sind, können als Monomere zur Erzeugung entsprechender Oligomere, Dendrimere oder Polymere Verwendung finden. Geeignete reaktive Abgangsgruppen sind beispielsweise Brom, Iod, Chlor, Boronsäuren, Boronsäureester, Amine, Alkenyl- oder Alkinylgruppen mit endständiger C-C-Doppelbindung bzw. C-C-Dreifachbindung, Oxirane, Oxetane, Gruppen, die eine Cycloaddition, beispielsweise eine 1,3-dipolare Cycloaddition, eingehen, wie beispielsweise Diene oder Azide, Carbonsäurederivate, Alkohole und Silane.

Weiterer Gegenstand der Erfindung sind daher Oligomere, Polymere oder Dendrimere enthaltend eine oder mehrere Verbindungen gemäß Formel (I), wobei die Bindung(en) zum Polymer, Oligomer oder Dendrimer an beliebigen, in Formel (I) mit R¹ oder R² substituierten Positionen lokalisiert sein können. Je nach Verknüpfung der Verbindung gemäß Formel (I) ist die Verbindung Bestandteil einer Seitenkette des Oligomers oder Polymers oder Bestandteil der Hauptkette. Unter einem Oligomer im Sinne dieser Erfindung wird eine Verbindung verstanden, welche aus mindestens drei Monomereinheiten aufgebaut ist. Unter einem Polymer im Sinne der Erfindung wird eine Verbindung verstanden, die aus mindestens zehn Monomereinheiten aufgebaut ist. Die erfindungsgemäßen Polymere, Oligomere oder Dendrimere können konjugiert, teilkonjugiert oder nichtkonjugiert sein. Die erfindungsgemäßen Oligomere oder Polymere können linear, verzweigt oder dendritisch sein. In den linear verknüpften Strukturen können die Einheiten gemäß Formel (I) direkt miteinander verknüpft sein oder sie können über eine bivalente Gruppe, beispielsweise über eine substituierte oder unsubstituierte Alkylengruppe, über ein Heteroatom oder über eine bivalente aromatische oder heteroaromatische Gruppe miteinander verknüpft sein. In verzweigten und dendritischen Strukturen können beispielsweise drei oder mehrere Einheiten gemäß Formel (I) über eine trivalente oder höhervalente Gruppe, beispielsweise über eine trivalente oder höhervalente aromatische oder heteroaromatische Gruppe, zu einem verzweigten bzw. dendritischen Oligomer oder Polymer verknüpft sein.

Für die Wiederholeinheiten gemäß Formel (I) in Oligomeren, Dendrimeren und Polymeren gelten dieselben Bevorzugungen wie oben für Verbindungen gemäß Formel (I) beschrieben.

Zur Herstellung der Oligomere oder Polymere werden die erfindungsgemäßen Monomere homopolymerisiert oder mit weiteren Monomeren copolymerisiert. Geeignete und bevorzugte Comonomere sind gewählt aus Fluorenen (z. B. gemäß EP 842208 oder WO 2000/22026), Spirobifluorenen (z. B. gemäß EP 707020, EP 894107 oder WO 2006/061181), Paraphenylenen (z. B. gemäß WO 1992/18552), Carbazolen (z. B. gemäß WO 2004/070772 oder WO 2004/113468), Thiophenen (z. B. gemäß EP 1028136), Dihydrophenanthrenen (z. B. gemäß WO 2005/014689 oder WO 2007/006383), cis- und trans-Indenofluorenen (z. B. gemäß WO 2004/041901 oder WO 2004/113412), Ketonen (z. B. gemäß WO 2005/040302), Phenanthrenen (z. B. gemäß WO 2005/104264 oder WO 2007/017066) oder auch mehreren dieser Einheiten. Die Polymere, Oligomere und Dendrimere enthalten üblicherweise noch weitere Einheiten, beispielsweise emittierende (fluoreszierende oder phosphoreszierende) Einheiten, wie z. B. Vinyltriarylamine (z. B. gemäß WO 2007/068325) oder phosphoreszierende Metallkomplexe (z. B. gemäß WO 2006/003000), und/oder Ladungstransporteinheiten, insbesondere solche basierend auf Triarylaminen.

Die erfindungsgemäßen Polymere, Oligomere und Dendrimere weisen vorteilhafte Eigenschaften, insbesondere hohe Lebensdauern, hohe Effizienzen und gute Farbkoordinaten auf.

Die erfindungsgemäßen Polymere und Oligomere werden in der Regel durch Polymerisation von einer oder mehreren Monomersorten hergestellt, von denen mindestens ein Monomer im Polymer zu Wiederholungseinheiten der Formel (I) führt. Geeignete Polymerisationsreaktionen sind dem Fachmann bekannt und in der Literatur beschrieben. Besonders geeignete und bevorzugte Polymerisationsreaktionen, die zu C-C- bzw. C-N-Verknüpfungen führen, sind folgende:
(A) SUZUKI-Polymerisation;
(B) YAMAMOTO-Polymerisation;
(C) STILLE-Polymerisation; und
(D) HARTWIG-BUCHWALD-Polymerisation.

Wie die Polymerisation nach diesen Methoden durchgeführt werden kann und wie die Polymere dann vom Reaktionsmedium abgetrennt und aufgereinigt werden können, ist dem Fachmann bekannt und in der Literatur, beispielsweise in WO 2003/048225, WO 2004/037887 und WO 2004/037887, im Detail beschrieben.

Gegenstand der vorliegenden Erfindung ist somit auch ein Verfahren zur Herstellung der erfindungsgemäßen Polymere, Oligomere und Dendrimere, das dadurch gekennzeichnet ist, dass sie durch Polymerisation gemäß SUZUKI, Polymerisation gemäß YAMAMOTO, Polymerisation gemäß STILLE oder Polymerisation gemäß HARTWIG-BUCHWALD hergestellt werden. Die erfindungsgemäßen Dendrimere können gemäß dem Fachmann bekannten Verfahren oder in Analogie dazu hergestellt werden. Geeignete Verfahren sind in der Literatur beschrieben, wie z. B. in Frechet, Jean M. J.; Hawker, Craig J., "Hyperbranched polyphenylene and hyperbranched polyesters: new soluble, three-dimensional, reactive polymers", Reactive & Functional Polymers (1995), 26(1-3), 127-36; Janssen, H. M.; Meijer, E. W., "The synthesis and characterization of dendritic molecules", Materials Science and Technology (1999), 20 (Synthesis of Polymers), 403-458; Tomalia, Donald A., "Dendrimer molecules", Scientific American (1995), 272(5), 62-6; WO 2002/067343 A1 und WO 2005/026144 A1.

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethylenglycolbutylmethylether, Triethylenglycolbutylmethyl-ether, Diethylenglycoldibutylether, Triethylenglycoldimethylether, Diethylenglycolmonobutylether, Tripropylenglycoldimethylether, Tetraethylenglycoldimethylether, 2-Isopropylnaphthalin, Pentylbenzol, Hexylbenzol, Heptylbenzol, Octylbenzol, 1,1-Bis(3,4-Dimethylphenyl)ethan oder Mischungen dieser Lösemittel.

Gegenstand der Erfindung ist daher weiterhin eine Formulierung, insbesondere eine Lösung, Dispersion oder Emulsion, enthaltend mindestens eine Verbindung gemäß Formel (I) oder mindestens ein Polymer, Oligomer oder Dendrimer enthaltend mindestens eine Einheit gemäß Formel (I) sowie mindestens ein Lösungsmittel, bevorzugt ein organisches Lösungsmittel. Wie solche Lösungen hergestellt werden können, ist dem Fachmann bekannt und beispielsweise in WO 2002/072714, WO 2003/019694 und der darin zitierten Literatur beschrieben.

Die erfindungsgemäßen Verbindungen gemäß Formel (I) eignen sich für den Einsatz in elektronischen Vorrichtungen, insbesondere in organischen Elektrolumineszenzvorrichtungen (OLEDs). Abhängig von der Substitution werden die Verbindungen in unterschiedlichen Funktionen und Schichten eingesetzt.

Weiterer Gegenstand der Erfindung ist daher die Verwendung einer Verbindung gemäß Formel (I) in einer elektronischen Vorrichtung. Dabei ist die elektronische Vorrichtung bevorzugt ausgewählt aus der Gruppe bestehend aus organischen integrierten Schaltungen (OICs), organischen Feld-Effekt-Transistoren (OFETs), organischen Dünnfilmtransistoren (OTFTs), organischen lichtemittierenden Transistoren (OLETs), organischen Solarzellen (OSCs), organischen optischen Detektoren, organischen Photorezeptoren, organischen Feld-Quench-Devices (OFQDs), organischen lichtemittierenden elektrochemischen Zellen (OLECs), organischen Laserdioden (O-Laser) und besonders bevorzugt organischen Elektrolumineszenzvorrichtungen (OLEDs).

Weiterer Gegenstand der Erfindung ist eine elektronische Vorrichtung enthaltend mindestens eine Verbindung der Formel (I). Bevorzugt ist die elektronische Vorrichtung gewählt aus den oben angegebenen Vorrichtungen. Besonders bevorzugt ist eine organische Elektrolumineszenzvorrichtung, enthaltend Anode, Kathode und mindestens eine emittierende Schicht, dadurch gekennzeichnet, dass mindestens eine organische Schicht mindestens eine Verbindung gemäß Formel (I) enthält.

Außer Kathode, Anode und der emittierenden Schicht kann die organische Elektrolumineszenzvorrichtung noch weitere Schichten enthalten. Diese sind beispielsweise gewählt aus jeweils einer oder mehreren Lochinjektionsschichten, Lochtransportschichten, Lochblockierschichten, Elektronentransportschichten, Elektroneninjektionsschichten, Elektronenblockierschichten, Excitonenblockierschichten, Zwischenschichten (Interlayers), Ladungserzeugungsschichten (Charge-Generation Layers) (IDMC 2003, Taiwan; Session 21 OLED (5), T. Matsumoto, T. Nakada, J. Endo, K. Mori, N. Kawamura, A. Yokoi, J. Kido, Multiphoton Organic EL Device Having Charge Generation Layer) und/oder organischen oder anorganischen p/n-Übergängen. Es sei aber darauf hingewiesen, dass nicht notwendigerweise jede dieser Schichten vorhanden sein muss und die Wahl der Schichten immer von den verwendeten Verbindungen abhängt und insbesondere auch von der Tatsache, ob es sich um eine fluoreszierende oder phosphoreszierende Elektrolumineszenzvorrichtung handelt.

Die Abfolge der Schichten der organischen Elektrolumineszenzvorrichtung ist bevorzugt die folgende: Anode-Lochinjektionsschicht-Lochtransportschicht-emittierende Schicht-Elektronentransportschicht-Elektroneninjektionsschicht-Kathode.

Dabei soll erneut darauf hingewiesen werden, dass nicht alle der genannten Schichten vorhanden sein müssen, und/oder dass zusätzlich weitere Schichten vorhanden sein können.

Die erfindungsgemäße organische Elektrolumineszenzvorrichtung kann mehrere emittierende Schichten enthalten. Besonders bevorzugt weisen diese Emissionsschichten in diesem Fall insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die blaues oder gelbes oder orangefarbenes oder rotes Licht emittieren. Insbesondere bevorzugt sind Dreischichtsysteme, also Systeme mit drei emittierenden Schichten, wobei bevorzugt mindestens eine dieser Schichten mindestens eine Verbindung gemäß Formel (I) enthält und wobei die drei Schichten blaue, grüne und orange oder rote Emission zeigen (für den prinzipiellen Aufbau siehe z. B. WO 2005/011013). Es soll angemerkt werden, dass sich für die Erzeugung von weißem Licht anstelle mehrerer farbig emittierender Emitterverbindungen auch eine einzeln verwendete Emitterverbindung eignen kann, welche in einem breiten Wellenlängenbereich emittiert.

Alternativ und/oder zusätzlich können die erfindungsgemäßen Verbindungen auch in der Lochtransportschicht oder in einer anderen Schicht vorhanden sein.

Es ist bevorzugt, wenn die Verbindung gemäß Formel (I) in einer emittierenden Schicht eingesetzt wird. Insbesondere eignet sich die Verbindung gemäß Formel (I) zur Verwendung als emittierendes Material (Emitterverbindung).

Die erfindungsgemäße Verbindung eignet sich besonders zur Verwendung als blau emittierende Emitterverbindung. Dabei kann die betreffende elektronische Vorrichtung eine einzige emittierende Schicht enthaltend die erfindungsgemäße Verbindung enthalten, oder sie kann zwei oder mehr emittierende Schichten enthalten. Die weiteren emittierenden Schichten können dabei eine oder mehrere erfindungsgemäße Verbindungen oder alternativ andere Verbindungen enthalten.

Wenn die erfindungsgemäße Verbindung als emittierendes Material in einer emittierenden Schicht eingesetzt wird, wird sie bevorzugt in Kombination mit einem oder mehreren Matrixmaterialien eingesetzt. Der Anteil der erfindungsgemäßen Verbindung in der Mischung der emittierenden Schicht beträgt in diesem Fall bevorzugt zwischen 0.1 und 50.0 Vol.-%, besonders bevorzugt zwischen 0.5 und 20.0 Vol.-%, ganz besonders bevorzugt zwischen 1.0 und 10.0 Vol.-%. Entsprechend beträgt der Anteil des Matrixmaterials bzw. der Matrixmaterialien bevorzugt zwischen 50.0 und 99.9 Vol.-%, besonders bevorzugt zwischen 80.0 und 99.5 Vol.-%, ganz besonders bevorzugt zwischen 90.0 und 99.0 Vol.-%.

Bevorzugte Matrixmaterialien zur Verwendung in Kombination mit den erfindungsgemäßen Verbindungen als Emitter sind ausgewählt aus den Klassen der Oligoarylene (z. B. 2,2',7,7'-Tetraphenylspirobifluoren gemäß EP 676461 oder Dinaphthylanthracen), insbesondere der Oligoarylene enthaltend kondensierte aromatische Gruppen, der Oligoarylenvinylene (z. B. DPVBi oder Spiro-DPVBi gemäß EP 676461), der polypodalen Metallkomplexe (z. B. gemäß WO 2004/081017), der lochleitenden Verbindungen (z. B. gemäß WO 2004/058911), der elektronenleitenden Verbindungen, insbesondere Ketone, Phosphinoxide, Sulfoxide, etc. (z. B. gemäß WO 2005/084081 und WO 2005/084082), der Atropisomere (z. B. gemäß WO 2006/048268), der Boronsäurederivate (z. B. gemäß WO 2006/117052) oder der Benzanthracene (z. B. gemäß WO 2008/145239). Besonders bevorzugte Matrixmaterialien sind ausgewählt aus den Klassen der Oligoarylene, enthaltend Naphthalin, Anthracen, Benzanthracen und/oder Pyren oder Atropisomere dieser Verbindungen, der Oligoarylenvinylene, der Ketone, der Phosphinoxide und der Sulfoxide. Ganz besonders bevorzugte Matrixmaterialien sind ausgewählt aus den Klassen der Oligoarylene, enthaltend Anthracen, Benzanthracen, Benzphenanthren und/oder Pyren oder Atropisomere dieser Verbindungen. Unter einem Oligoarylen im Sinne dieser Erfindung soll eine Verbindung verstanden werden, in der mindestens drei Aryl- bzw. Arylengruppen aneinander gebunden sind.

Bevorzugte Matrixmaterialien zur Verwendung in Kombination mit der Verbindung der Formel (I) in der emittierenden Schicht sind in der folgenden Tabelle abgebildet.

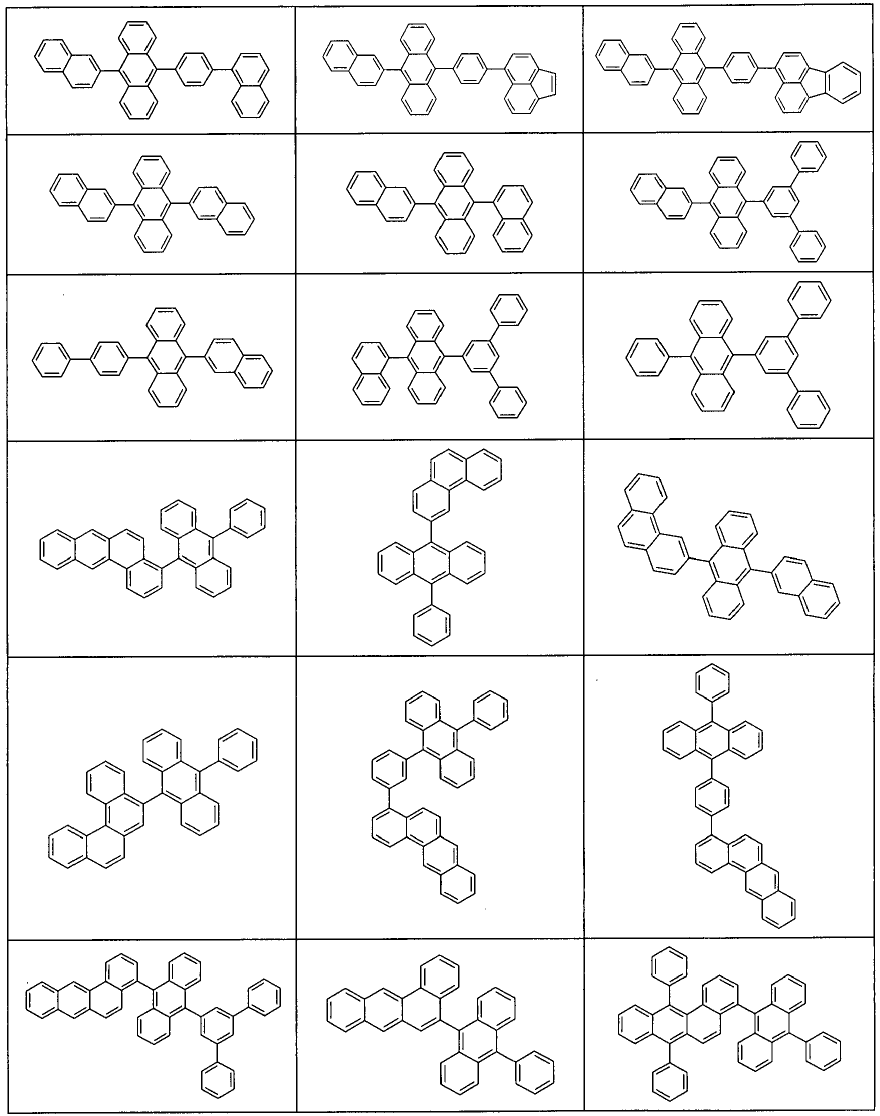

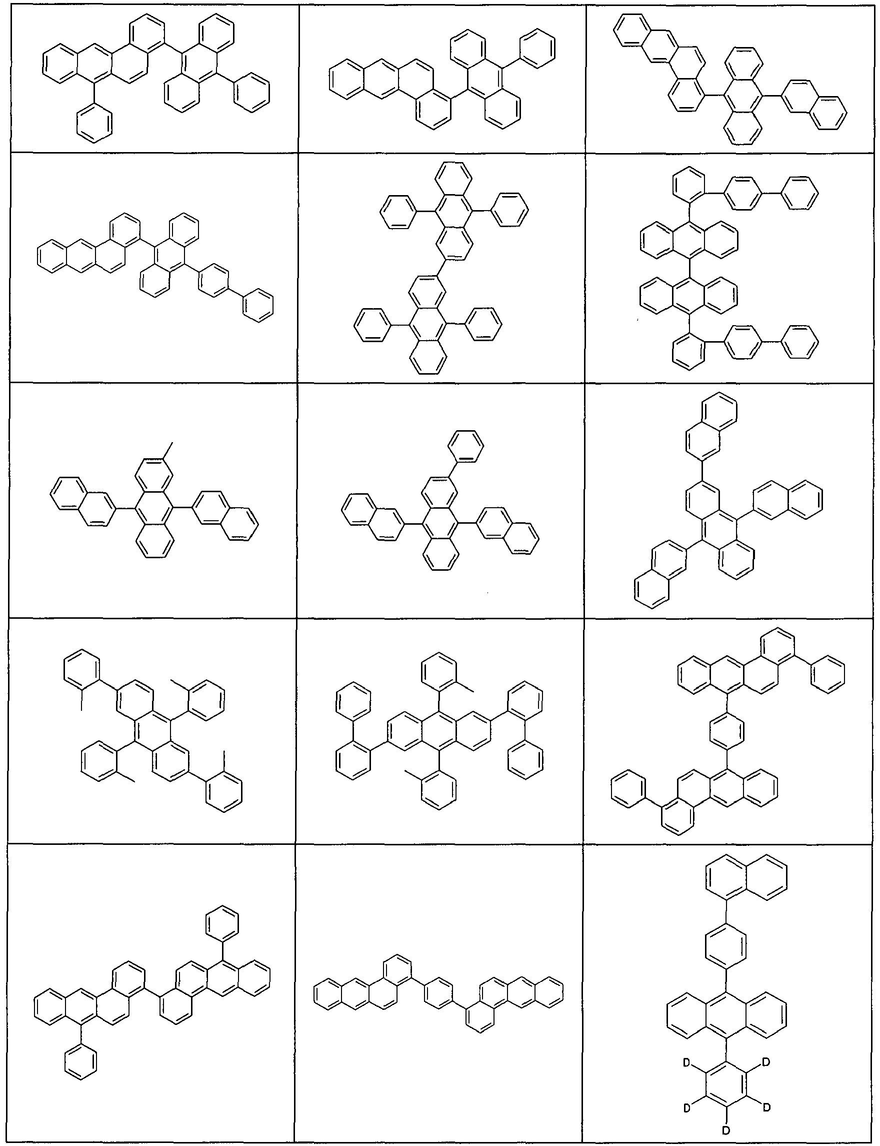

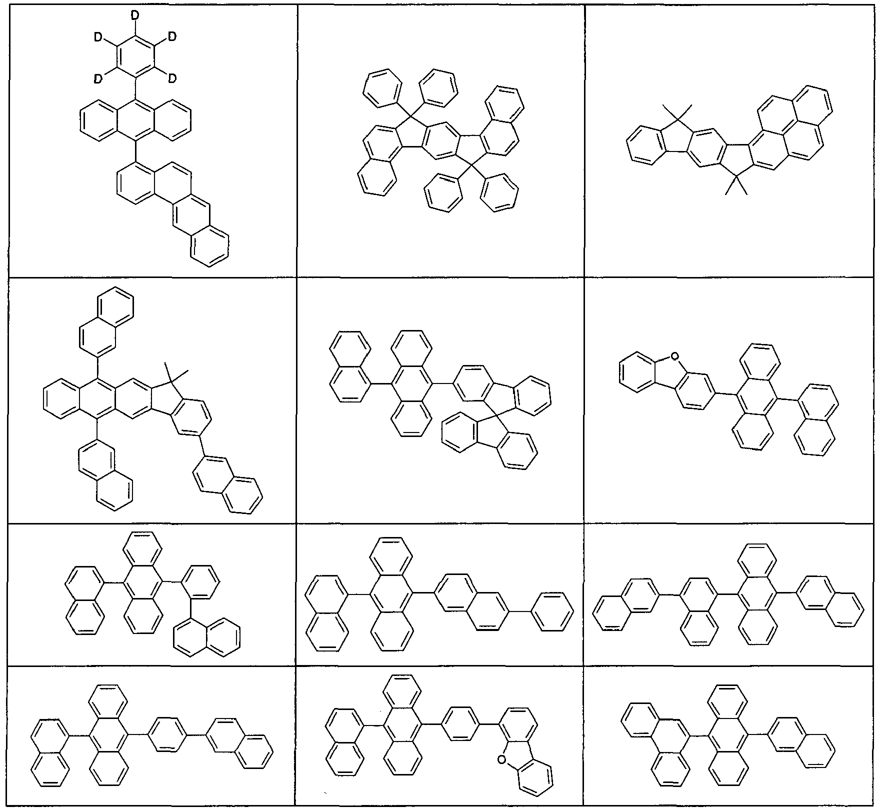

Die erfindungsgemäßen Verbindungen können auch in anderen Schichten eingesetzt werden, beispielsweise als Lochtransportmaterialien in einer Lochinjektions- oder Lochtransportschicht oder Elektronenblockierschicht oder als Matrixmaterialien in einer emittierenden Schicht, bevorzugt als Matrixmaterialien für phosphoreszierende Emitter.

Wird die Verbindung gemäß Formel (I) als Lochtransportmaterial in einer Lochtransportschicht, einer Lochinjektionsschicht oder einer Elektronenblockierschicht eingesetzt, so kann die Verbindung als Reinmaterial, d.h. in einem Anteil von 100 %, in der Lochtransportschicht eingesetzt werden, oder sie kann in Kombination mit einer oder mehreren weiteren Verbindungen eingesetzt werden. Gemäß einer bevorzugten Ausführungsform enthält die organische Schicht enthaltend die Verbindung der Formel (I) dann zusätzlich einen oder mehrere p-Dotanden. Als p-Dotanden werden gemäß der vorliegenden Erfindung bevorzugt solche organischen Elektronenakzeptorverbindungen eingesetzt, die eine oder mehrere der anderen Verbindungen der Mischung oxidieren können.

Besonders bevorzugte Ausführungsformen von p-Dotanden sind die in WO 2011/073149, EP 1968131, EP 2276085, EP 2213662, EP 1722602, EP 2045848, DE 102007031220, US 8044390, US 8057712, WO 2009/003455, WO 2010/094378, WO 2011/120709, US 2010/0096600 und WO 2012/095143 offenbarten Verbindungen.

Wenn die Verbindung der Formel (I) als Matrixmaterial in Kombination mit einem phosphoreszierenden Emitter in einer emittierenden Schicht eingesetzt wird, ist der phosphoreszierende Emitter bevorzugt ausgewählt aus den unten aufgeführten Klassen und Ausführungsformen von phosphoreszierenden Emittern. Weiterhin sind in diesem Fall in der emittierenden Schicht bevorzugt ein oder mehrere weitere Matrixmaterialien vorhanden.

Solche sogenannten Mixed-Matrix-Systeme umfassen bevorzugt zwei oder drei verschiedene Matrixmaterialien, besonders bevorzugt zwei verschiedene Matrixmaterialien. Bevorzugt stellt dabei eines der beiden Materialien ein Material mit lochtransportierenden Eigenschaften und das andere Material ein Material mit elektronentransportierenden Eigenschaften dar. Bevorzugt stellt die Verbindung der Formel (I) das Material mit lochtransportierenden Eigenschaften dar.

Die gewünschten elektronentransportierenden und lochtransportierenden Eigenschaften der Mixed-Matrix-Komponenten können jedoch auch hauptsächlich oder vollständig in einer einzigen Mixed-Matrix-Komponente vereinigt sein, wobei die weitere bzw. die weiteren Mixed-Matrix-Komponenten andere Funktionen erfüllen. Die beiden unterschiedlichen Matrixmaterialien können dabei in einem Verhältnis von 1:50 bis 1:1, bevorzugt 1:20 bis 1:1, besonders bevorzugt 1:10 bis 1:1 und ganz besonders bevorzugt 1:4 bis 1:1 vorliegen. Bevorzugt werden Mixed-Matrix-Systeme in phosphoreszierenden organischen Elektrolumineszenzvorrichtungen eingesetzt. Genauere Angaben zu Mixed-Matrix-Systemen sind unter anderem in der Anmeldung WO 2010/108579 enthalten.

Besonders geeignete Matrixmaterialien, welche in Kombination mit den erfindungsgemäßen Verbindungen als Matrixkomponenten eines Mixed-Matrix-Systems verwendet werden können, sind ausgewählt aus den unten angegebenen bevorzugten Matrixmaterialien für phosphoreszierende Emitter oder den bevorzugten Matrixmaterialien für fluoreszierende Emitter, je nachdem welche Art von Emitterverbindung im mixed-Matrix-System eingesetzt wird.

Im folgenden sind allgemein bevorzugte Materialklassen zur Verwendung als entsprechende Funktionsmaterialien in den erfindungsgemäßen organischen Elektrolumineszenzvorrichtungen aufgeführt.

Als phosphoreszierende Emitter eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als phosphoreszierende Emitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten.

Dabei werden im Sinne der vorliegenden Erfindung alle lumineszierenden Iridium-, Platin- oder Kupferkomplexe als phosphoreszierende Verbindungen angesehen.

Beispiele der oben beschriebenen phosphoreszierenden Emitter können den Anmeldungen WO 2000/70655, WO 2001/41512, WO 2002/02714, WO 2002/15645, EP 1191613, EP 1191612, EP 1191614, WO 2005/033244, WO 2005/019373 und US 2005/0258742 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenzvorrichtungen bekannt sind, zur Verwendung in den erfindungsgemäßen Vorrichtungen. Auch kann der Fachmann ohne erfinderisches Zutun weitere phosphoreszierende Komplexe in Kombination mit den erfindungsgemäßen Verbindungen in OLEDs einsetzen.

Bevorzugte fluoreszierende Emitter sind neben den erfindungsgemäßen Verbindungen ausgewählt aus der Klasse der Arylamine. Unter einem Arylamin bzw. einem aromatischen Amin im Sinne dieser Erfindung wird eine Verbindung verstanden, die drei substituierte oder unsubstituierte aromatische oder heteroaromatische Ringsysteme direkt an den Stickstoff gebunden enthält. Bevorzugt ist mindestens eines dieser aromatischen oder heteroaromatischen Ringsysteme ein kondensiertes Ringsystem, besonders bevorzugt mit mindestens 14 aromatischen Ringatomen. Bevorzugte Beispiele hierfür sind aromatische Anthracenamine, aromatische Anthracendiamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 9-Position. Unter einem aromatischen Anthracendiamin wird eine Verbindung verstanden, in der zwei Diarylaminogruppen direkt an eine Anthracengruppe gebunden sind, vorzugsweise in 9,10-Position. Aromatische Pyrenamine, Pyrendiamine, Chrysenamine und Chrysendiamine sind analog dazu definiert, wobei die Diarylaminogruppen am Pyren bevorzugt in 1-Position bzw. in 1,6-Position gebunden sind.

Bevorzugte Matrixmaterialien zur Verwendung mit fluoreszierenden Emitterverbindungen sind obenstehend aufgeführt.

Bevorzugte Matrixmaterialien für phosphoreszierende Emitter sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Biscarbazolylbiphenyl) oder die in WO 2005/039246, US 2005/0069729, JP 2004/288381, EP 1205527 oder WO 2008/086851 offenbarten Carbazolderivate, Indolocarbazolderivate, z. B. gemäß WO 2007/063754 oder WO 2008/056746, Indenocarbazolderivate, z. B. gemäß WO 2010/136109, WO 2011/000455 oder WO 2013/041176, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2010/015306, WO 2007/063754 oder WO 2008/056746, Zinkkomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, überbrückte CarbazolDerivate, z. B. gemäß US 2009/0136779, WO 2010/050778, WO 2011/042107, WO 2011/088877 oder WO 2012/143080, Triphenylenderivaten, z. B. gemäß WO 2012/048781, oder Lactame, z. B. gemäß WO 2011/116865 oder WO 2011/137951.

Geeignete Ladungstransportmaterialien, wie sie in der Lochinjektions- bzw. Lochtransportschicht bzw. Elektronenblockierschicht oder in der Elektronentransportschicht der erfindungsgemäßen elektronischen Vorrichtung verwendet werden können, sind neben den erfindungsgemäßen Verbindungen beispielsweise die in Y. Shirota et al., Chem. Rev. 2007, 107(4), 953-1010 offenbarten Verbindungen oder andere Materialien, wie sie gemäß dem Stand der Technik in diesen Schichten eingesetzt werden.

Als Materialien für die Elektronentransportschicht können alle Materialien verwendet werden, wie sie gemäß dem Stand der Technik als Elektronentransportmaterialien in der Elektronentransportschicht verwendet werden. Insbesondere eignen sich Aluminiumkomplexe, beispielsweise Alq₃, Zirkoniumkomplexe, beispielsweise Zrq₄, Lithiumkomplexe, beispielsweise Liq, Benzimidazolderivate, Triazinderivate, Pyrimidinderivate, Pyridinderivate, Pyrazinderivate, Chinoxalinderivate, Chinolinderivate, Oxadiazolderivate, aromatische Ketone, Lactame, Borane, Diazaphospholderivate und Phosphinoxidderivate. Weiterhin geeignete Materialien sind Derivate der oben genannten Verbindungen, wie sie in JP 2000/053957, WO 2003/060956, WO 2004/028217, WO 2004/080975 und WO 2010/072300 offenbart werden.

Bevorzugt sind als Lochtransportmaterialien, die in einer Lochtransport-, Lochinjektions- oder Elektronenblockierschicht in der erfindungsgemäßen Elektrolumineszenzvorrichtung verwendet werden können, Indenofluorenamin-Derivate (z. B. gemäß WO 06/122630 oder WO 06/100896), die in EP 1661888 offenbarten Aminderivate, Hexaazatriphenylenderivate (z. B. gemäß WO 01/049806), Aminderivate mit kondensierten Aromaten (z. B. gemäß US 5,061,569), die in WO 95/09147 offenbarten Aminderivate, Monobenzoindenofluorenamine (z. B. gemäß WO 08/006449), Dibenzoindenofluorenamine (z. B. gemäß WO 07/140847), Spirobifluoren-Amine (z. B. gemäß WO 2012/034627 oder WO 2013/120577), Fluoren-Amine (z. B. gemäß den noch nicht offengelegten Anmeldungen EP 12005369.9, EP 12005370.7 und EP 12005371.5), Spiro-Dibenzopyran-Amine (z. B. gemäß WO 2013/083216) und Dihydroacridin-Derivate (z. B. gemäß WO 2012/150001). Auch die erfindungsgemäßen Verbindungen können als Lochtransportmaterialien verwendet werden.

Als Kathode der organischen Elektrolumineszenzvorrichtung sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, AI, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag oder Al, wobei dann in der Regel Kombinationen der Metalle, wie beispielsweise Ca/Ag, Mg/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, Li₂O, BaF₂, MgO, NaF, CsF, Cs₂CO₃, etc.). Weiterhin kann dafür Lithiumchinolinat (LiQ) verwendet werden. Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/NiOₓ, Al/PtOₓ) bevorzugt sein. Für einige Anwendungen muss mindestens eine der Elektroden transparent oder teiltransparent sein, um entweder die Bestrahlung des organischen Materials (organische Solarzelle) oder die Auskopplung von Licht (OLED, O-LASER) zu ermöglichen. Bevorzugte Anodenmaterialien sind hier leitfähige gemischte Metalloxide. Besonders bevorzugt sind Indium-ZinnOxid (ITO) oder Indium-Zink Oxid (IZO). Bevorzugt sind weiterhin leitfähige, dotierte organische Materialien, insbesondere leitfähige dotierte Polymere.

Die Vorrichtung wird entsprechend (je nach Anwendung) strukturiert, kontaktiert und schließlich versiegelt, da sich die Lebensdauer der erfindungsgemäßen Vorrichtungen bei Anwesenheit von Wasser und/oder Luft verkürzt.

In einer bevorzugten Ausführungsform ist die erfindungsgemäße organische Elektrolumineszenzvorrichtung dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Dabei ist es jedoch auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Nozzle Printing oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen gemäß Formel (I) nötig. Hohe Löslichkeit lässt sich durch geeignete Substitution der Verbindungen erreichen.

Weiterhin bevorzugt ist es, dass zur Herstellung einer erfindungsgemäßen organischen Elektrolumineszenzvorrichtung eine oder mehrere Schichten aus Lösung und eine oder mehrere Schichten durch ein Sublimationsverfahren aufgetragen werden.

Erfindungsgemäß können die elektronischen Vorrichtungen enthaltend eine oder mehrere erfindungsgemäße Verbindungen in Displays, als Lichtquellen in Beleuchtungsanwendungen sowie als Lichtquellen in medizinischen und/oder kosmetischen Anwendungen (z.B. Lichttherapie) eingesetzt werden.

### Ausführungsbeispiele

### A) Synthesebeispiele

### A-1) Variante I

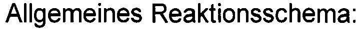

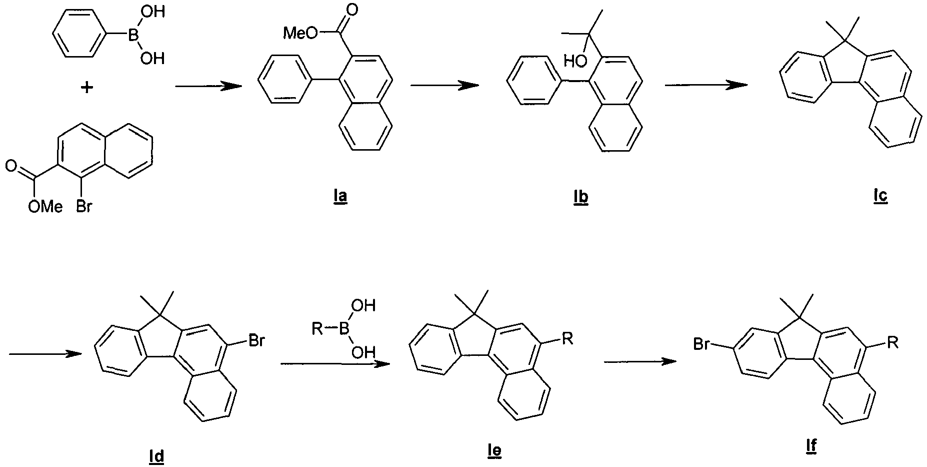

### A-1-1) Synthese der Bausteine (If-1) bis (If-5)

### 1-Phenyl-naphthalen-2-carboxylsäuremethylester (Ia)

1-Brom-naphthalen-2-carboxylsäuremethylester (70.0 g, 264 mmol), Phenylboronsäure (38.6 g, 317 mmol) und Kaliumphosphatmonohydrat (182 g, 792 mmol) werden in einer Mischung von 0.2 L Toluol, 0.2 L Dioxan und 0.2 L Wasser gelöst und mit Palladiumacetat (1.18 g, 5.3 mmol) und Tri-orthotolyl-Phosphin (3.2 g, 10.6 mmol) versetzt. Der Ansatz wird über Nacht unter Rückfluss erhitzt, auf Raumtemperatur abgekühlt und mit dest. Wasser erweitert. Nach Phasentrennung wird die wässrige Phase mehrmals mit Toluol extrahiert. Die vereinigten organischen Phasen werden mit dest. Wasser gewaschen, über Magnesiumsulfat getrocknet und über Aluminiumoxid filtriert. Die organische Phase wird bis zu einem orangen Öl eingeengt. Es werden 69 g Produkt erhalten (99% d. Th.).

### 2-(1-Phenyl-naphthalen-2-yl)-propan-2-ol (Ib)

(la) (69 g, 264 mmol) und Cerium(III) Chlorid (71 g, 291 mmol) werden in 500 mL THF gelöst und bei 0°C mit Methylmagnesiumchlorid (3 M Lösung in THF) (308 mL, 925 mmol) versetzt. Man lässt die Reaktionslösung über Nacht im Eisbad auf Raumtemperatur erwärmen. Der Ansatz wird vorsichtig mit gesättigter NH₄Cl-Lösung hydrolysiert und mit 4%iger Salzsäure neutralisiert. Die Mischung wird mit dest. Wasser erweitert und gründlich mit Toluol extrahiert. Die vereinigten organischen Phasen werden mehrmals mit dest. Wasser und einmal mit NatriumhydrogencarbonatLösung gewaschen und über Magnesiumsulfat getrocknet. Nach Entfernen des Lösemittels im Vakuum erhält man einen hellbeigen Feststoff. Dieser wird in einem Heptan/ Toluol-Gemisch umkristallisiert. Man isoliert 52 g (75% d. Th.) als farblosen Feststoff.

### 7,7-Dimethyl-7H-benzo[c]fluorene (Ic)

(Ib) (52 g, 198 mmol) wird mit 500 mL DCM gelöst und bei 0°C mit Methansulfonsäure (64 mL, 991 mmol) und Polyphosphorsäure (77 g, 793 mmol) versetzt. Man lässt die Reaktionslösung über Nacht auf Raumtemperatur erwärmen. Die Mischung wird mit Ethanol erweitert und eingeengt. Der Rückstand wird in Toluol gelöst, mit NaOH-Lösung und dest. Wasser gewaschen und über Magnesiumsulfat getrocknet. Nach Entfernen des Lösemittels im Vakuum wird der Feststoff in Ethanol umkristallisiert. Man isoliert 44 g eines gelben Feststoffes. (91% d. Th.).

### 5-Bromo-7,7-dimethyl-7H-benzo[c]fluorene (Id)

(Ic) (38.2 g, 156 mmol) wird in 0.3 L Chloroform gelöst und auf 0°C gekühlt. Unter Rühren wird eine Dibrom-Lösung (117 g, 660 mmol) in 0.2 L Chloroform zugetropft, so dass die Reaktionstemperatur nicht über 5 °C steigt. Die Reaktion wird über Nacht im Eisbad auf Raumtemperatur erwärmt. 200 mL einer 10%igen Natriumthiosulfat-Lösung werden hinzu gegeben und die Phasen getrennt. Die wässrige Phase wird mit DCM mehrmals extrahiert. Die organische Phase wird mit dest. Wasser gewaschen, getrocknet und vom Lösungsmittel befreit. Der erhaltene Feststoff wird in Toluol/Heptan umkristallisiert. Man erhält 50 g eines farblosen Feststoffes (99% d. Th.).

### 7,7-Dimethyl-5-Phenyl-7H-benzo[c]fluoren (le-1)

(Id) (28.5 g, 88 mmol), Phenylboronsäure (13.2 g, 106 mmol) und Kaliumcarbonat (30.5 g, 220 mmol) werden in einer Mischung von 150 mL Toluol und 150 mL Wasser gelöst und mit Tetrakis-(triphenylphosphin)-palladium (1.02 g, 0.9 mmol) versetzt. Der Ansatz wird über Nacht unter Rückfluss erhitzt, auf Raumtemperatur abgekühlt und mit dest. Wasser erweitert. Nach Phasentrennung wird die wässrige Phase mehrmals mit Toluol extrahiert. Die vereinigten organischen Phasen werden mit dest. Wasser gewaschen, über Magnesiumsulfat getrocknet und über AlOx und Kieselgel filtriert. Die organische Phase wird eingeengt und der resultierende Feststoff mit Ethanol gewaschen. Es werden 25.9 g (92% d. Th.) Produkt erhalten.

Analog dazu werden folgende Verbindungen hergestellt:

| | Brom-Aren | R | Produkt | Ausbeute |
|---|---|---|---|---|
| (Ie-1) | 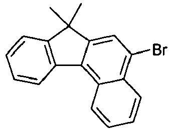 | 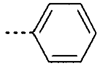 | 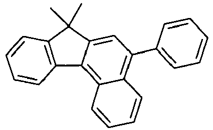 | 92% |
| (Ie-2) | 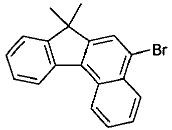 | 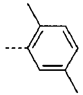 | 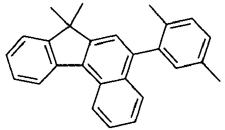 | 89% |
| (Ie-3) | 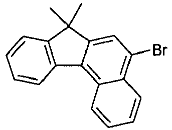 | 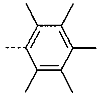 | 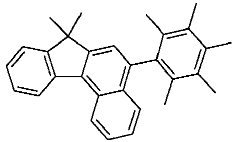 | 82% |
| (Ie-4) | 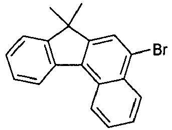 | 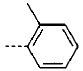 | 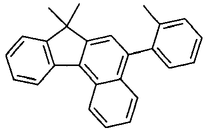 | 91% |
| (Ie-5) | 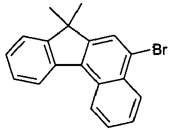 | 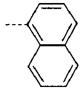 | 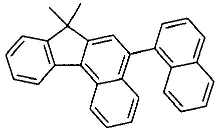 | 80% |
| (Ie-8) | 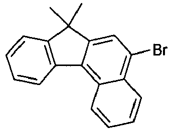 | 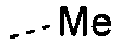 | 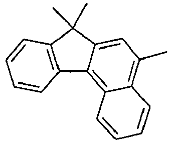 | 85% |
| (Ie-9) | 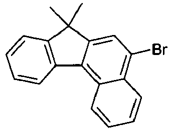 | 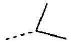 | 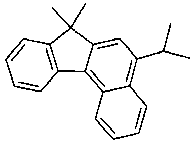 | 78% |
| (Ie-10) | 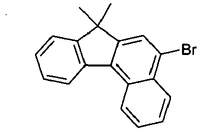 | 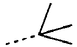 | 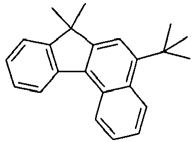 | 65% |
| (Ie-11) | 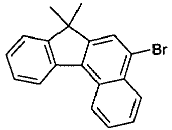 | 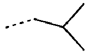 | 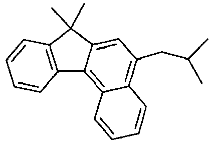 | 82% |
| (Ie-12) | 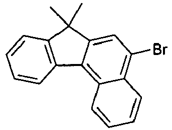 | 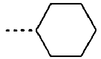 | 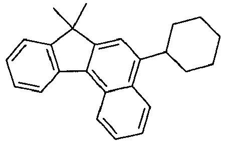 | 72% |

### 9-Bromo-7,7-dimethyl-5-Phenyl-7H-benzo[c]fluoren (If-1)

(Ie-1) (25.8 g, 81 mmol) wird in 0.15 L Chloroform gelöst und auf 0 °C gekühlt. Unter Rühren wird eine Dibrom-Lösung (13.6 g, 85 mmol) in 0.1 L Chloroform zugetropft, so dass die Reaktionstemperatur nicht über 5 °C steigt. Die Reaktion wird über Nacht im Eisbad auf Raumtemperatur erwärmt. 100 mL einer 10%igen Natriumthiosulfat-Lösung werden hinzu gegeben und die Phasen getrennt. Die wässrige Phase wird mit DCM mehrmals extrahiert. Die organische Phase wird mit dest. Wasser gewaschen, getrocknet und vom Lösungsmittel befreit. Der erhaltene Feststoff wird in Toluol/Heptan umkristallisiert. Es werden 22 g eines farblosen Feststoffs (62% d. Th.) erhalten.

Analog dazu werden folgende Verbindungen hergestellt:

| | Edukt | Produkt | Ausbeute |
|---|---|---|---|
| (If-1) | 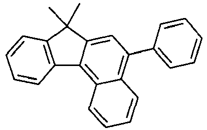 | 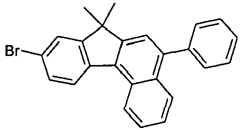 | 62% |
| (If-2) | 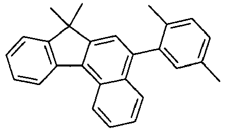 | 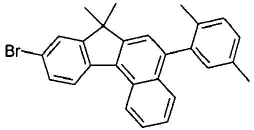 | 75% |
| (If-3) | 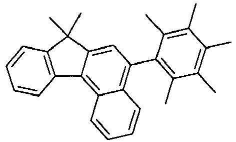 | 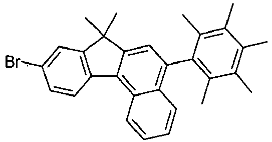 | 80% |
| (If-4) | 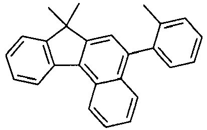 | 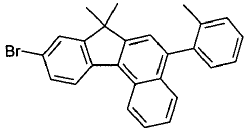 | 78% |
| (If-5) | 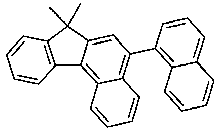 | 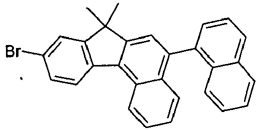 | 77% |
| (If-8) | 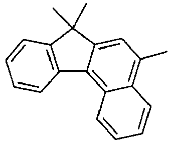 | 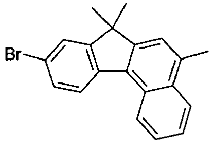 | 80% |
| (If-9) | 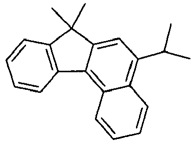 | 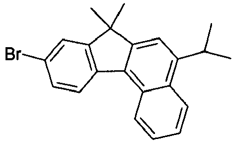 | 75% |
| (If-10) | 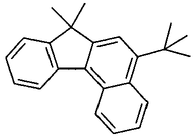 | 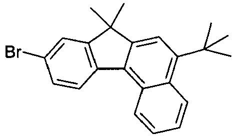 | 83% |
| (If-11) | 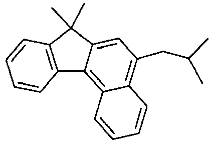 | 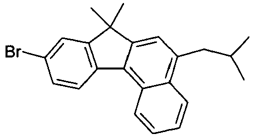 | 85% |
| (If-12) | 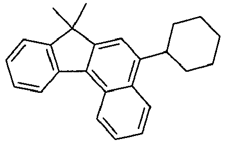 | 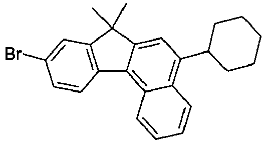 | 89% |

### A-1-2) Synthese des Bausteins (If-6)

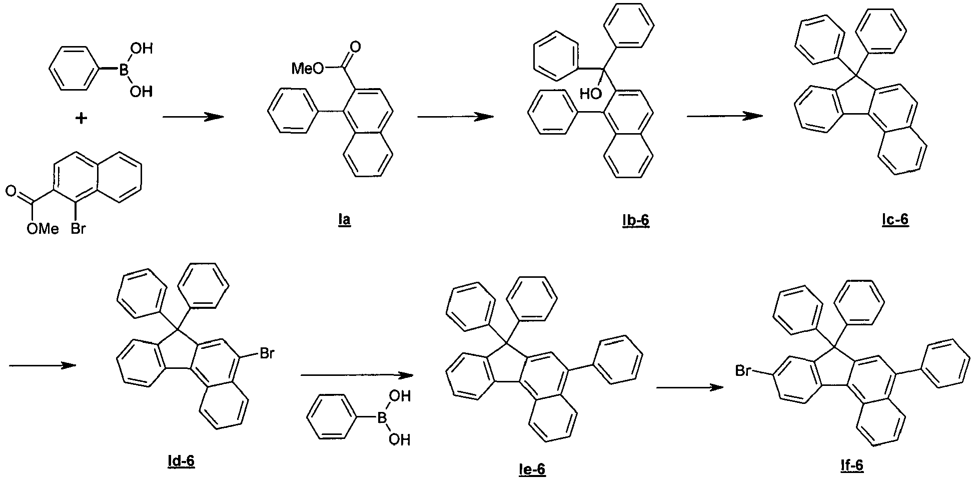

### Diphenyl-(1-Phenyl-naphthalen-2-yl)-methanol (Ib-6)

(la) (35 g, 133 mmol) und Cerium(III) Chlorid (36 g, 146 mmol) werden in 250 mL THF gelöst und bei 0°C mit Phenylmagnesiumchlorid (3 M Lösung in THF) (150 mL, 450 mmol) versetzt. Man lässt die Reaktionslösung über Nacht im Eisbad auf Raumtemperatur erwärmen. Der Ansatz wird vorsichtig mit gesättigter NH₄Cl-Lösung hydrolysiert und mit 4%iger Salzsäure neutralisiert. Die Mischung wird mit dest. Wasser erweitert und gründlich mit Toluol extrahiert. Die vereinigten organischen Phasen werden mehrmals mit dest. Wasser und einmal mit NatriumhydrogencarbonatLösung gewaschen und über Magnesiumsulfat getrocknet. Nach Entfernen des Lösemittels im Vakuum erhält man einen hellbeigen Feststoff. Dieser wird in Heptan/Toluol umkristallisiert. Man isoliert 41 g (80% d. Th.) als farblosen Feststoff.

Der Baustein (lc-6) wird analog zu (Ic) synthetisiert, mit einer Ausbeute von 88%.

Der Baustein (ld-6) wird analog zu (Id) synthetisiert, mit einer Ausbeute von 95%.

Der Baustein (Ie-6) wird analog zu (Ie-1) synthetisiert, mit einer Ausbeute von 82%.

Der Baustein (If-6) wird analog zu (If-1) synthetisiert, mit einer Ausbeute von 68%.

### A-1-3) Synthese des Bausteins (Id-7)

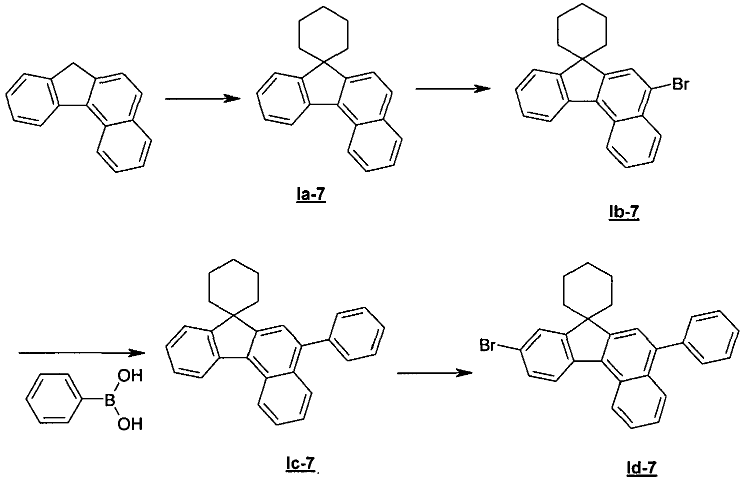

7H-Benzo[c]fluoren wurde gemäß folgender Literaturvorschrift synthetisiert: Organic Letters, 2009, Vol. 11, No. 20, 4588-4591.

### Synthese von (Ia-7):

7H-Benzo[c]fluoren (38 g, 176 mmol), 1,5-Dibrompentan (40.5 g, 176 mmol) und Tetrabutylammoniumbromid (32,3 g, 100 mmol) werden in 0.5 L Toluol gelöst. 0.5 L 3M NaOH Lösung wird zugegeben, und die Reaktion wird über Nacht auf Rückfluss gekocht. Die Reaktion wird auf Raumtemperatur abgekühlt, die Phasen getrennt, die wässrige Phase wird mit Toluol drei Mal extrahiert. Die organische Phase wird mit dest. Wasser gewaschen, getrocknet und vom Lösungsmittel befreit. Der erhaltene Feststoff wird in Toluol Heptan umkristallisiert. 31 g eines farblosen Feststoffes (62% d. Th.) werden isoliert.

Der Baustein (Ib-7) wird analog zu (Id) synthetisiert, mit einer Ausbeute von 87%.

Der Baustein (Ic-7) wird analog zu (Ie-1) synthetisiert, mit einer Ausbeute von 89%.

Der Baustein (Id-7) wird analog zu (If-1) synthetisiert, mit einer Ausbeute von 65%.

### A-1-4) Synthese der Zielverbindungen (I-1) bis (I-13)

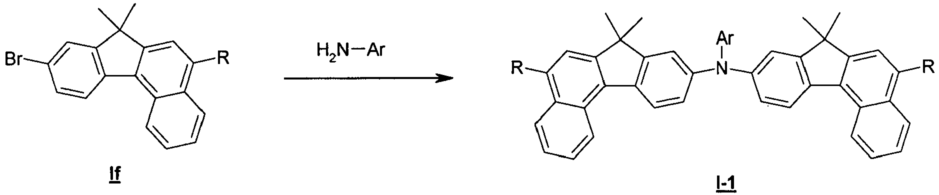

### Bis-(7,7-dimethyl-5-phenyl-7H-benzo[c]fluoren-9-yl)-p-tolyl-amin (I-1)

(If-1) (14 g, 35 mmol) und p-Tolylamin (1.7 g, 16 mmol) werden in 200 mL Toluol gelöst. Anschließend wird die Reaktionslösung mit 1M Tritertbutylphosphin-Lösung in Toluol (3.6 mL, 3.6 mmol), Palladium(II)-Acetat (5.0 g, 17.4 mmol) und Natrium-tert-butoxid (10 g, 104 mmol) versetzt und über Nacht unter Rückfluss erhitzt. Die Mischung wird bei Raumtemperatur mit Toluol und dest. Wasser erweitert, die organische Phase abgetrennt und die wässrige Phase mehrmals mit Toluol extrahiert. Die org. Phase wird mit Magnesiumsulfat getrocknet, über AlOx und Kieselgel filtriert und eingeengt. Der Rückstand wird in Heptan/Toluol umkristallisiert. Verbindung (I-1) wird als hell gelber Feststoff isoliert (6 g, 23% d. Th.).

Analog dazu werden folgende Verbindungen hergestellt:

| Beispiel Verbindung | Benzfluoren Derivat | Ar-NH₂ | Produkt | Ausbeute |
|---|---|---|---|---|
| I-1 | If-1 | 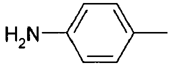 | 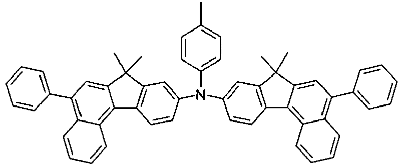 | 23% |
| I-2 | If-1 | 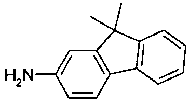 | 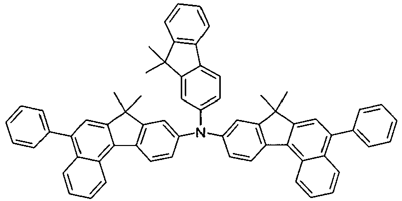 | 20% |
| I-3 | If-1 | 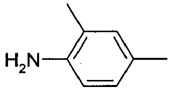 | 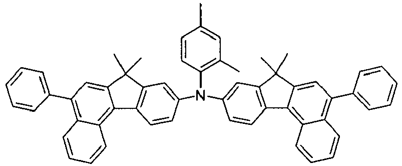 | 25% |
| I-4 | If-1 | 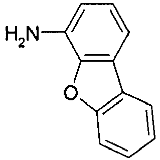 | 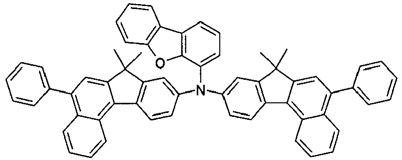 | 17% |
| I-5 | If-1 | 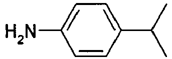 | 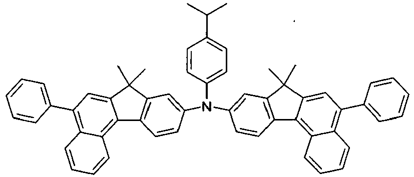 | 28% |
| I-6 | If-1 | 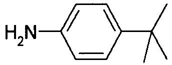 | 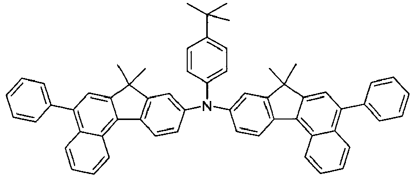 | 33% |
| I-7 | If-1 | 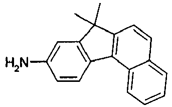 | 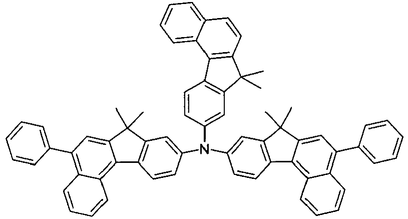 | 25% |
| I-8 | If-2 | 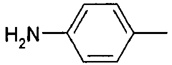 | 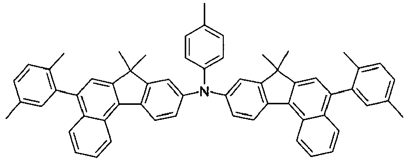 | 26% |
| I-9 | If-2 | 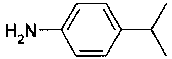 | 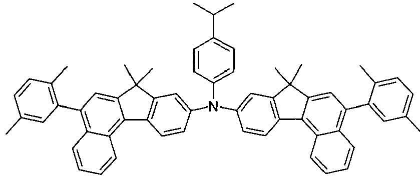 | 44% |
| I-10 | If-2 | 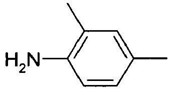 | 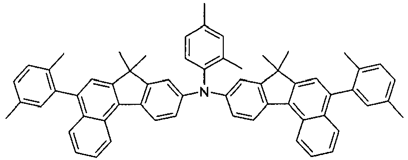 | 40% |
| I-11 | If-2 | 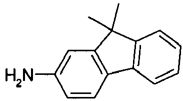 | 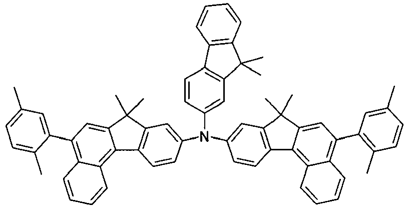 | 21% |
| I-12 | If-3 | 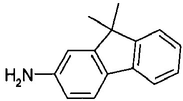 | 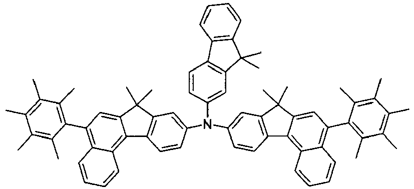 | 29% |
| I-13 | If-4 | 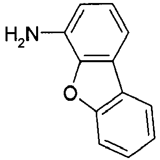 | 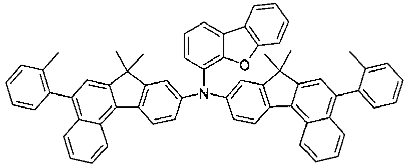 | 15% |
| I-14 | If-5 | 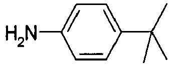 | 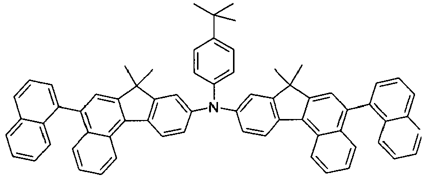 | 26% |
| I-15 | If-6 | 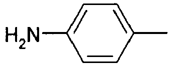 | 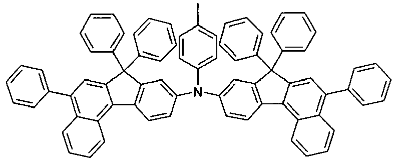 | 31% |
| I-16 | Id-7 | 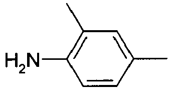 | 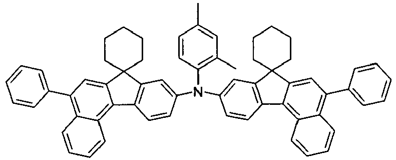 | 29% |
| I-17 | If-8 | 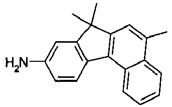 | 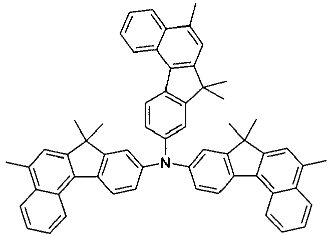 | 30% |
| I-18 | If-9 | 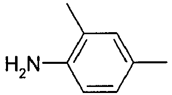 | 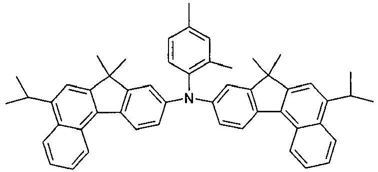 | 28% |
| I-19 | If-10 | 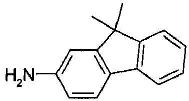 | 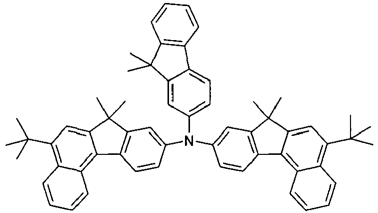 | 22% |
| I-20 | If-11 | 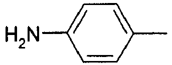 | 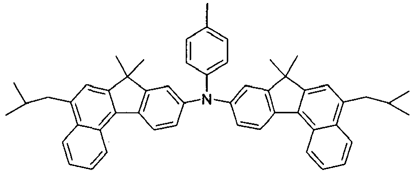 | 32% |
| I-21 | If-12 | 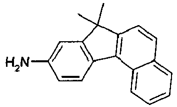 | 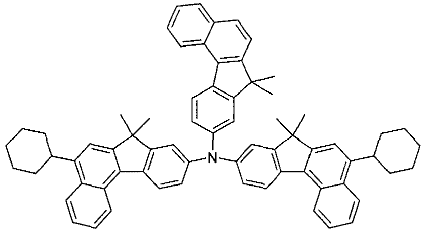 | 39% |

### A-2) Variante II:

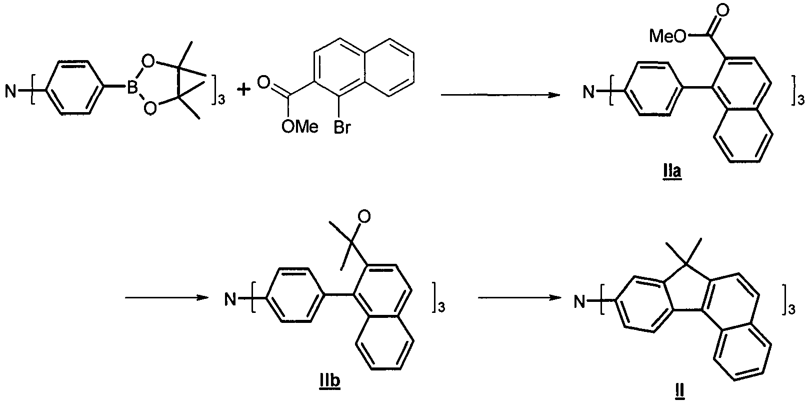

Die Synthese vom Edukt Tris-[4-(4,4,5,5-tetramethyl-[1,3,2]dioxaborolan-2-yl)-phenyl]-amin wird in folgender Publikation beschrieben: Chem. Mater. 2011, 23, 4428-4434.

### Tris-[4-(2-carboxylsäuremethylester-napht-1-yl)-phenyl]-amin (IIa)

1-Brom-naphthalen-2-carboxylsäuremethylester (40 g, 150 mmol), Tris-[4-(4,4,5,5-tetramethyl-[1,3,2]dioxaborolan-2-yl)-phenyl]-amin (23.5 g, 37.5 mmol) und Kaliumphosphat-Monohydrat (130 g, 0.56 mol) werden in einer Mischung von 0.75 L Toluol, 0.75 L Dioxan und 0.75 L Wasser gelöst und mit Paladiumacetat (0.67 g, 3 mmol) und tri-orthotolyl Phosphin (5.5 g, 18 mmol) versetzt. Der Ansatz wird über Nacht unter Rückfluss erhitzt, auf Raumtemperatur abgekühlt und mit dest. Wasser erweitert. Nach Phasentrennung wird die wässrige Phase mehrmals mit Toluol extrahiert. Die vereinigten organischen Phasen werden mit dest. Wasser gewaschen, über Magnesiumsulfat getrocknet und über AlOx filtriert. Die organische Phase wird eingeengt. (IIa) wird als hell gelber Feststoff isoliert: 19.5 g (65% d. Th.)

### Tris-[4-(2-(propan-2-ol-2yl)-napht-1-yl)-phenyl]-amin (IIb)

(IIa) (19.5 g, 25.4 mmol) und Cerium(III) Chlorid (39 g, 81,9 mmol) werden in 1 L THF gelöst und bei 0°C mit Methylmagnesiumchlorid (3 M Lösung in THF) (330 mL, 975 mmol) versetzt. Man lässt die Reaktionslösung über Nacht im Eisbad auf Raumtemperatur erwärmen. Der Ansatz wird vorsichtig mit gesättigter NH₄Cl-Lösung hydrolysiert und mit 4%iger Salzsäure neutralisiert. Die Mischung wird mit dest. Wasser erweitert und gründlich mit Toluol extrahiert. Die vereinigten organischen Phasen werden mehrmals mit dest. Wasser und einmal mit NatriumhydrogencarbonatLösung gewaschen und über Magnesiumsulfat getrocknet. Nach Entfernen des Lösemittels wird der Feststoff in einem Heptan/ Toluol-Gemisch umkristallisiert. Man isoliert 15,6 g (80% d. Th.) als farblosen Feststoff.

### Tris-(7,7-Dimethyl-7H-benzo[c]fluoren-9-yl)-amin (II)

(IIb) (15.6 g, 19.5 mmol) wird mit 600 mL DCM gelöst und bei 0°C mit Methansulfonsäure (20 mL, 0.3 mol) und Polyphosphorsäure (23 g, 230 mmol) versetzt. Man lässt die Reaktionslösung über Nacht auf Raumtemperatur erwärmen. Die Mischung wird mit Ethanol erweitert und eingeengt. Der Rückstand wird in Toluol gelöst, mit NaOH-Lösung und dest. Wasser gewaschen und über Magnesiumsulfat getrocknet. Nach Entfernen des Lösemittels im Vakuum wird der Feststoff über eine Kieselgelsäule aufgereinigt (Heptan/Ethylacetat). Verbindung (II) wird als gelber Feststoff isoliert: 5,46 g (40% d. Th.).

### A-3) Variante III:

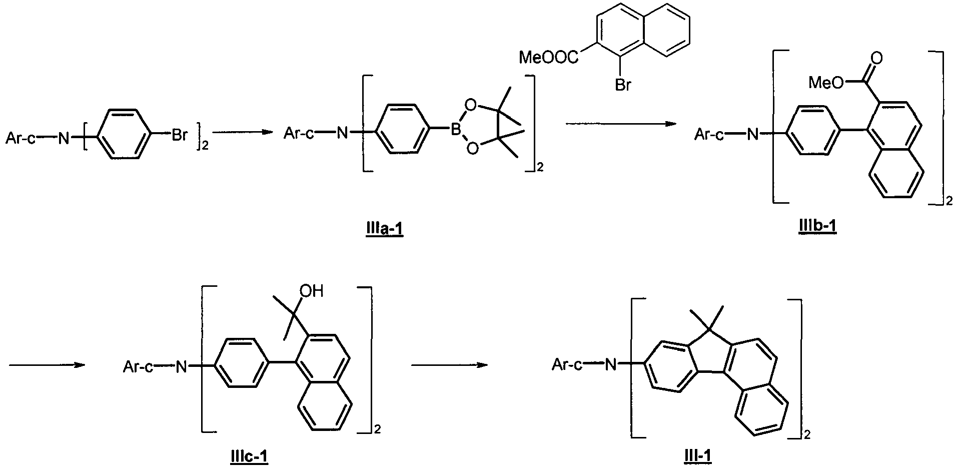

Die Edukte Bis-(4-bromo-phenyl)-(4-tert-butyl-phenyl)-amin und Bis-(4-bromo-phenyl)-(4-sec-butyl-phenyl)-amin werden nach der Methode aus folgender Publikation synthetisiert: Journal of Polymer Science, Part A: Polymer Chemistry, 2011 , vol. 49, 2, 352 - 360. Die Synthese von Biphenyl-4-yl-bis-(4-bromo-phenyl)-amin wird gemäß WO 2009/139358 durchgeführt.

### Bis-[4-(4,4,5,5-tetramethyl-[1,3,2]dioxaborolan-2-yl)-phenyl]-(4-tert-butyl-phenyl)-amin (IIIa-1)

Bis-(4-bromo-phenyl)-(4-tert-butyl-phenyl)-amin (27,6 g, 60 mmol) und Bispinacolato-diboran (38.1 g, 150 mmol) werden in 0.5 L THF gelöst. Dann werden Kaliumacetat (49 g, 500 mmol) und 1,1'-Bis(diphenylphosphino)ferrocen-dichloropalladium(II)*DCM (1.45 g, 1.8 mmol) zu der Reaktionslösung hinzugegeben und über Nacht unter Rückfluss erhitzt. Nach dem Abkühlen auf Raumtemperatur wird der Ansatz mit DCM und dest. Wasser erweitert und die wässrige Phase wirdmehrmals mit DCM extrahiert. Die vereinigten organischen Phasen werden mit dest. Wasser gewaschen und nach Trocknen mit Magnesiumsulfat über AlOx filtriert. Das Lösemittel wird bei Normaldruck entfernt. Der erhaltene Feststoff wird in Toluol-Heptan Gemisch umkristallisiert. Es werden 23.7 g eines hellgrauen Pulvers erhalten (72% d. Th.).

Analog dazu werden folgende Verbindungen hergestellt:

| Beispiel Verbindung | Ar-c | Produkt | Ausbeute |
|---|---|---|---|
| IIIa-1 | 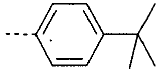 | 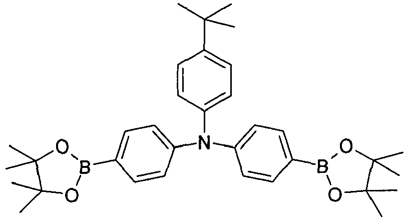 | 72% |
| IIIa-2 | 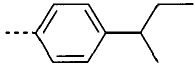 | 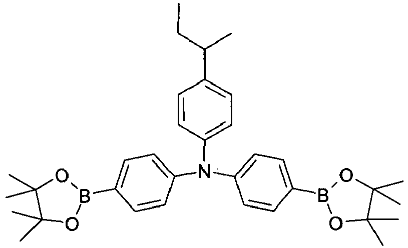 | 81% |
| IIIa-3 | 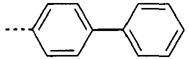 | 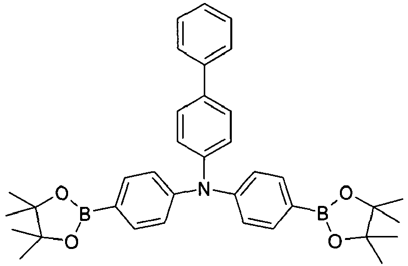 | 71% |

### Bis-[4-(2-carboxylsäuremethylester-napht-1-yl)-phenyl]-(4-tert-butylphenyl)-amin (IIIb-1)

(IIIa-1) (23.7 g, 42.1 mmol), 1-Brom-naphthalen-2-carboxylsäure-methylester (40.0 g, 151 mmol) und Kaliumphosphat-Monohydrat (130 g, 565 mmol) werden in einer Mischung von 0.75 L Toluol, 0.75 L Dioxan und 0.75 L Wasser gelöst und mit Paladiumacetat (675 mg, 3,0 mmol) und Triorthotolylphosphin (5.5 g, 18 mmol) versetzt. Der Ansatz wird über Nacht unter Rückfluss erhitzt, auf Raumtemperatur abgekühlt und mit dest. Wasser erweitert. Nach Phasentrennung wird die wässrige Phase mehrmals mit Toluol extrahiert. Die vereinigten organischen Phasen werden mit dest. Wasser gewaschen, über Magnesiumsulfat getrocknet und über AlOx filtriert. Die organische Phase wird eingeengt. Verbindung (IIIb-1) wird als hell gelber Feststoff isoliert 20.6 g (73% d. Th.)

Analog dazu werden folgende Verbindungen hergestellt:

| Beispiel Verbindung | Ar-c | Produkt | Ausbeute |
|---|---|---|---|
| IIIb-1 | 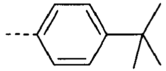 | 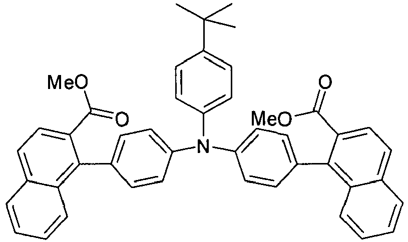 | 55% |
| IIIb-2 | 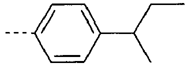 | 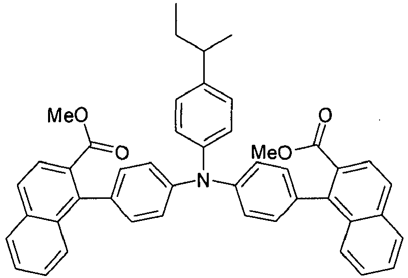 | 58% |
| IIIb-3 | 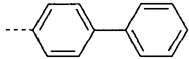 | 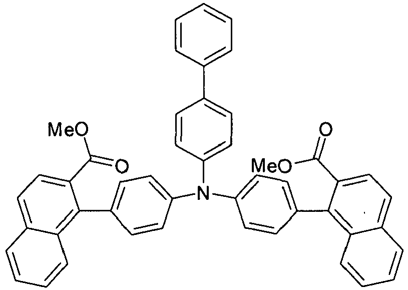 | 65% |

### Bis-[4-(2-(propan-2-ol-2yl)-napht-1-yl)-phenyl]-(4-tert-butyl-phenyl)-amin (IIIc-1)

(IIIb-1) (20.5 g, 26.7 mmol) und Cerium(III) Chlorid (41 g, 86 mmol) werden in 1 L THF gelöst und bei 0°C mit Methylmagnesiumchlorid (3 M Lösung in THF) (350 mL, 1.05 mol) versetzt. Man lässt die Reaktionslösung über Nacht im Eisbad auf Raumtemperatur erwärmen. Der Ansatz wird vorsichtig mit gesättigter NH₄Cl-Lösung hydrolysiert und mit 4%iger Salzsäure neutralisiert. Die Mischung wird mit dest. Wasser erweitert und gründlich mit Toluol extrahiert. Die vereinigten organischen Phasen werden mehrmals mit dest. Wasser und einmal mit NatriumhydrogencarbonatLösung gewaschen und über Magnesiumsulfat getrocknet. Nach Entfernen des Lösemittels wird der Feststoff in einem Heptan/ Toluol-Gemisch umkristallisiert. Man isoliert 16,4 g (80% d. Th.) als farblosen Feststoff.

Analog dazu werden folgende Verbindungen hergestellt:

| Beispiel Verbindung | Ar-c | Produkt | Ausbeute |
|---|---|---|---|
| IIIc-1 | 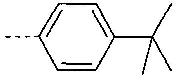 | 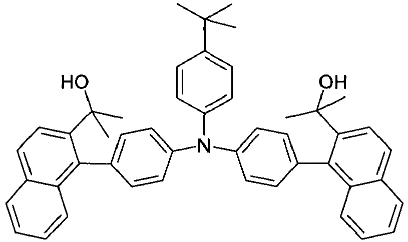 | 36% |
| IIIc-2 | 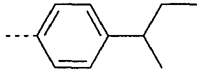 | 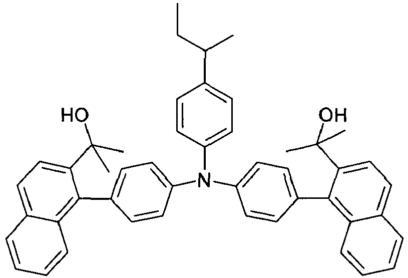 | 43% |
| IIIc-3 | 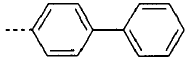 | 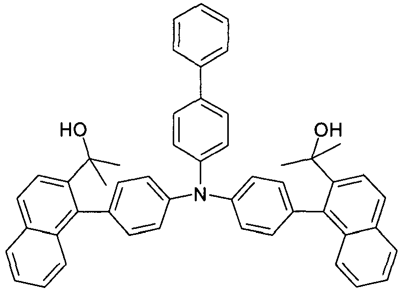 | 41% |

### Bis-(7,7-Dimethyl-7H-benzo[c]fluoren-9-yl)-(4-tert-butyl-phenyl)-amin (III-1)

(IIIc-1) (16.2 g, 20.3 mmol) wird mit 600 mL DCM gelöst und bei 0°C mit Methansulfonsäure (20.0 mL, 300 mmol) und Polyphosphorsäure (24.4 g, 24,4 mmol) versetzt. Man lässt die Reaktionslösung über Nacht auf Raumtemperatur erwärmen. Die Mischung wird mit Ethanol erweitert und eingeengt. Der Rückstand wird in Toluol gelöst, mit NaOH-Lösung und dest. Wasser gewaschen und über Magnesiumsulfat getrocknet. Nach Entfernen des Lösemittels im Vakuum wird der Feststoff über eine Kieselgelsäule aufgereinigt (Heptan/Ethylacetat) und danach in Toluol-Heptan Gemisch umkristallisiert. (III-1) wird als gelber Feststoff isoliert: 5,8 g (45% d. Th.).

Analog dazu werden folgende Verbindungen hergestellt:

| Beispiel Verbindung | Ar-c | Produkt | Ausbeute |
|---|---|---|---|
| III-1 | 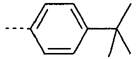 | 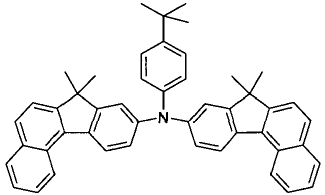 | 45% |
| III-2 | 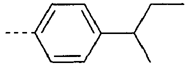 | 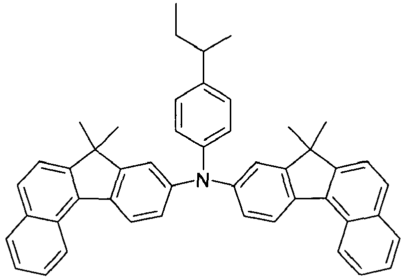 | 47% |
| III-3 | 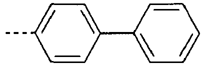 | 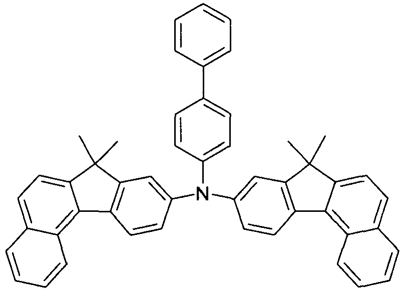 | 42% |

### B) Device-Beispiele: Herstellung von OLEDs

Die Herstellung von erfindungsgemäßen OLEDs sowie OLEDs nach dem Stand der Technik erfolgt nach einem allgemeinen Verfahren gemäß WO 04/058911, das auf die hier beschriebenen Gegebenheiten (Schichtdickenvariation, Materialien) angepasst wird.

In den folgenden Beispielen (siehe Tabellen 1 bis 3) werden die Daten verschiedener OLEDs vorgestellt. Als Substrate werden Glasplättchen verwendet, die mit strukturiertem ITO (Indium Zinn Oxid) der Dicke 50 nm beschichtet sind. Die OLEDs haben prinzipiell folgenden Schichtaufbau: Substrat / Buffer (20nm) / Lochinjektionsschicht (HIL, 160nm)/ Lochtransportschicht (HTL, 20nm) / Emissionsschicht (EML, 20nm) / Elektronentransportschicht (ETL, 30nm) / Elektroneninjektionsschicht (LiQ 1nm) und abschließend eine Kathode. Die Kathode wird durch eine 100 nm dicke Aluminiumschicht gebildet. Als Buffer wird eine 20nm dicke Schicht Clevios P VP AI 4083 (bezogen von Heraeus Clevios GmbH, Leverkusen) durch Spincoating aufgebracht. Alle restlichen Materialien werden in einer Vakuumkammer thermisch aufgedampft. Der Aufbau der EML der OLEDs und die in dieser Schicht verwendeten Materialien sind in Tabelle 1 gezeigt. Die Strukturen aller in der OLED verwendeten Materialien sind in Tabelle 3 gezeigt, wobei HIL das Material der Lochinjektionsschicht, HTL das Material der Lochtransportschicht, ETL das Material der Elektronentransportschicht und LiQ das Material der Elektroneninjektionsschicht darstellt.

Die Emissionsschicht (EML) besteht immer aus mindestens einem Matrixmaterial (Host=H) und einer emittierenden Verbindung (Dotand=D), der dem Matrixmaterial durch Coverdampfung in einem bestimmten Volumenanteil beigemischt wird. Eine Angabe wie H1:D1 (95%:5%) bedeutet hierbei, dass das Matrixmaterial H1 in einem Volumenanteil von 95% und die emittierende Verbindung D1 in einem Anteil von 5% in der Schicht vorliegt.

Die OLEDs werden standardmäßig charakterisiert. Hierfür werden die Elektrolumineszenzspektren aufgenommen, die Stromeffizienz (gemessen in cd/A) und die externe Quanteneffizienz (EQE, gemessen in Prozent) in Abhängigkeit der Leuchtdichte unter Annahme einer lambertschen Abstrahlcharakteristik aus Strom-Spannungs-Leuchtdichte-Kennlinien (IUL-Kennlinien) berechnet und abschließend die Lebensdauer der Bauteile bestimmt. Die Elektrolumineszenzspektren werden bei einer Leuchtdichte von 1000 cd/m² aufgenommen und daraus die CIE 1931 x und y Farbkoordinaten berechnet. Die Angabe EQE @ 1000 cd/m² bezeichnet die externe Quanteneffizienz bei einer Betriebsleuchtdichte von 1000 cd/m². Die Lebensdauer LD50 @ 60mA/cm² ist die Zeit, die vergeht, bis die Starthelligkeit (cd/m²) bei einer Stromdichte von 60mA/cm² auf die Hälfte gesunken ist. Die erhaltenen Daten für die verschiedenen OLEDs sind in Tabelle 2 zusammengefasst.

### Verwendung der erfindungsgemäßen Verbindungen als fluoreszierende Emitter in OLEDs

Die erfindungsgemäßen Verbindungen eignen sich insbesondere als blau emittierende fluoreszierende Emitter. Hierzu wurden OLEDs enthaltend die Verbindungen D2, D3, D4 und D5 hergestellt (Beispiele E3 bis E7).

Die Vergleichsbeispiele V1 und V2, in denen der Emitter V-D1 gemäß dem Stand der Technik anstelle der erfindungsgemäßen Verbindungen verwendet wird, verdeutlichen, dass ein einzelner Benzofluorensubstituent an einem Amin nicht zu zufriedenstellenden Ergebnissen führt (schlechtere Werte für externe Quanteneffizienz und Lebensdauer).

Hingegen werden mit den erfindungsgemäßen Verbindungen als fluoreszierende Emitter, wie die Beispiele E3 bis E7 zeigen, hervorragende Werte für externe Quanteneffizienz erzielt, bei tiefblauer Emission und verbesserter Lebensdauer (LD50).

| **Tabelle 1: Aufbau der OLEDs** | |
|---|---|
| ***Bsp.*** | ***EML*** |
| | *Dicke* / *nm* |
| *V1* | *H1(95%): V-D1(5%) 20 nm* |
| *V2* | *H2(95%):V-D1(5%)20 nm* |
| *E3* | *H2(95%):D2(5%) 20 nm* |
| *E4* | *H1(95%):D3(5%)20 nm* |
| *E5* | *H2 (95%):D3(5%)20 nm* |
| *E6* | *H2(95%):D4(5%) 20 nm* |
| *E7* | *H2(95%):D5(5%) 20 nm* |

| **Tabelle 2: Daten der OLEDs** | | | | |
|---|---|---|---|---|
| ***Bsp.*** | ***EQE* @ *1000 cd*/*m2*** | ***LD50* @ *60mA*/*cm²*** | ***CIE*** | |
| | *%* | *[h]* | *x* | y |
| *V1* | *2.1* | *80* | 0.15 | 0.09 |
| *V2* | *2.3* | *80* | 0.16 | 0.09 |
| *E3* | *5.8* | *230* | 0.15 | 0.13 |
| *E4* | *6.0* | *290* | 0.15 | 0.11 |
| *E5* | *6.3* | *310* | *0.15* | *0.12* |
| *E6* | *7* | *320* | 0.15 | 0.11 |
| *E7* | *7.4* | *340* | 0.15 | 0.12 |

| **Tabelle 3: Strukturen der verwendeten Materialien** | |
|---|---|
| 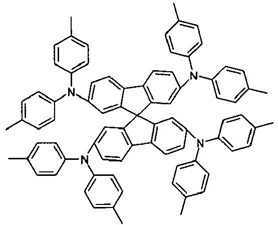 | 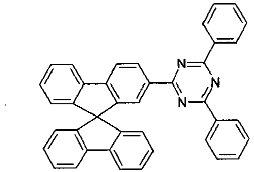 |
| HIL | ETL |
| 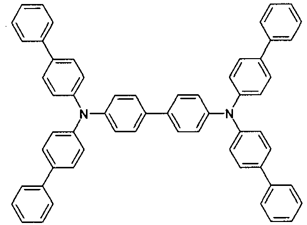 | 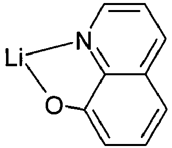 |
| HTL | LiQ |
| 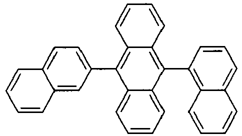 | 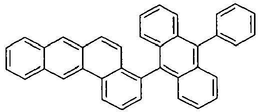 |
| H1 | H2 |
| 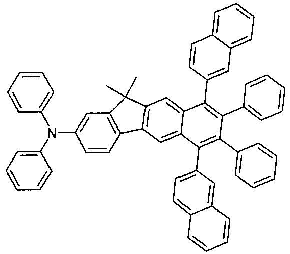 | 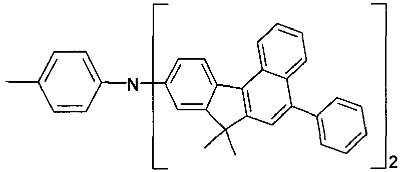 |
| V-D1 | D2 (Verbindung I-1 der Synthesebsp.) |
| 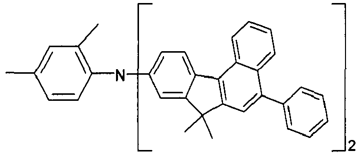 | 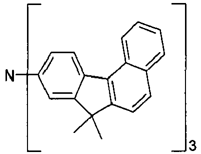 |
| D3 (Verbindung I-3 der Synthesebsp.) | D4 (Verbindung II der Synthesebsp.) |
| 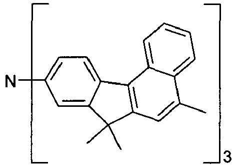 | |
| D5 (Verbindung I-17 der Synthesebeispiele) | |

## Patentansprüche

1. Verbindung einer Formel (I) 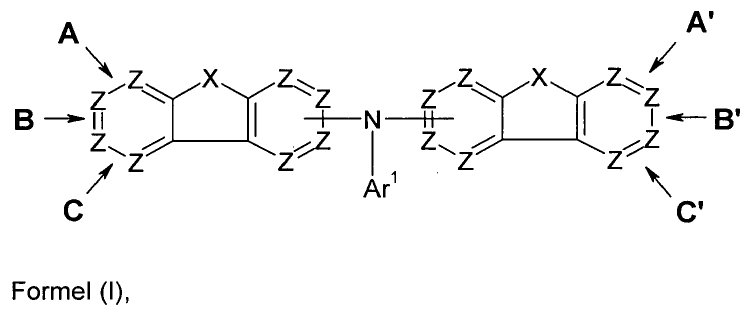 wobei gilt:
an mindestens eine der drei Bindungen gewählt aus den Bindungen A, B und C und an mindestens eine der drei Bindungen gewählt aus den Bindungen A', B' und C' ist ein aromatischer oder heteroaromatischer Sechsring ankondensiert, der mit einem oder mehreren Resten R¹ substituiert sein kann,
Z ist bei jedem Auftreten gleich oder verschieden CR¹ oder N;
X ist bei jedem Auftreten gleich oder verschieden BR², C(R²)₂, C(R²)₂-C(R²)₂, -R²C=CR²-, -R²C=N-, Si(R²)₂, Si(R²)₂-Si(R²)₂, C=O, O, S, S=O, SO₂, NR², PR² oder P(=O)R²;
Ar¹ ist ein aromatisches oder heteroaromatisches Ringsystem mit 6 bis 40 aromatischen Ringatomen, das mit einem oder mehreren Resten R¹ substituiert sein kann;
R¹ ist bei jedem Auftreten gleich oder verschieden H, D, F, Cl, Br, I, C(=O)R³, CN, Si(R³)₃, P(=O)(R³)₂, S(=O)R³, S(=O)₂R³, eine geradkettige Alkyl- oder Alkoxygruppe mit 1 bis 20 C-Atomen oder eine verzweigte oder cyclische Alkyl- oder Alkoxygruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R³ substituiert sein können und wobei eine oder mehrere CH₂-Gruppen in den oben genannten Gruppen durch -R³C=CR³-, -C≡C-, Si(R³)₂, C=O, C=NR³, -C(=O)O-, -C(=O)NR³-, NR³, P(=O)(R³), -O-, -S-, SO oder SO₂ ersetzt sein können und wobei ein oder mehrere H-Atome in den oben genannten Gruppen durch D, F, Cl, Br, I oder CN ersetzt sein können, oder ein aromatisches oder heteroaromatisches Ringsystem mit 5 bis 30 aromatischen Ringatomen, das jeweils durch einen oder mehrere Reste R³ substituiert sein kann, oder eine Aryloxy- oder Heteroaryloxy-gruppe mit 5 bis 30 aromatischen Ringatomen, die durch einen oder mehrere Reste R³ substituiert sein kann;
R² ist bei jedem Auftreten gleich oder verschieden H, D, F, Cl, Br, I, C(=O)R³, CN, Si(R³)₃, N(R³)₂, P(=O)(R³)₂, S(=O)R³, S(=O)₂R³, eine geradkettige Alkyl- oder Alkoxygruppe mit 1 bis 20 C-Atomen oder eine verzweigte oder cyclische Alkyl- oder Alkoxygruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R³ substituiert sein können und wobei eine oder mehrere CH₂-Gruppen in den oben genannten Gruppen durch -R³C=CR³-, -C≡C-, Si(R³)₂, C=O, C=NR³, -C(=O)O-, -C(=O)NR³-, NR³, P(=O)(R³), -O-, -S-, SO oder SO₂ ersetzt sein können und wobei ein oder mehrere H-Atome in den oben genannten Gruppen durch D, F, Cl, Br, I oder CN ersetzt sein können, oder ein aromatisches oder heteroaromatisches Ringsystem mit 5 bis 30 aromatischen Ringatomen, das jeweils durch einen oder mehrere Reste R³ substituiert sein kann, oder eine Aryloxy- oder Heteroaryloxy-gruppe mit 5 bis 30 aromatischen Ringatomen, die durch einen oder mehrere Reste R³ substituiert sein kann, wobei zwei oder mehr Reste R² miteinander verknüpft sein können und einen Ring bilden können;
R³ ist bei jedem Auftreten gleich oder verschieden H, D, F, Cl, Br, I, C(=O)R⁴, CN, Si(R⁴)₃, N(R⁴)₂, P(=O)(R⁴)₂, S(=O)R⁴, S(=O)₂R⁴, eine geradkettige Alkyl- oder Alkoxygruppe mit 1 bis 20 C-Atomen oder eine verzweigte oder cyclische Alkyl- oder Alkoxygruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R⁴ substituiert sein können und wobei eine oder mehrere CH₂-Gruppen in den oben genannten Gruppen durch -R⁴C=CR⁴-, -C≡C-, Si(R⁴)₂, C=O, C=NR⁴, -C(=O)O-, -C(=O)NR⁴-, NR⁴, P(=O)(R⁴), -O-, -S-, SO oder SO₂ ersetzt sein können und wobei ein oder mehrere H-Atome in den oben genannten Gruppen durch D, F, Cl, Br, I oder CN ersetzt sein können, oder ein aromatisches oder heteroaromatisches Ringsystem mit 5 bis 30 aromatischen Ringatomen, das jeweils durch einen oder mehrere Reste R⁴ substituiert sein kann, oder eine Aryloxy- oder Heteroaryloxy-gruppe mit 5 bis 30 aromatischen Ringatomen, die durch einen oder mehrere Reste R⁴ substituiert sein kann;
R⁴ ist bei jedem Auftreten gleich oder verschieden H, D, F oder ein aliphatischer, aromatischer oder heteroaromatischer organischer Rest mit 1 bis 20 C-Atomen, in dem auch ein oder mehrere H-Atome durch D oder F ersetzt sein können;
wobei der Fall ausgeschlossen ist, bei dem an beide Bindungen A und A' jeweils ein aromatischer oder heteroaromatischer Sechsring ankondensiert ist und kein weiterer aromatischer oder heteroaromatischer Sechsring an eine der anderen vier Bindungen gewählt aus den Bindungen B, C, B' und C' ankondensiert ist.

2. Verbindung nach Anspruch 1, **dadurch gekennzeichnet, dass** an keine der beiden Bindungen A und A' in Formel (I) ein aromatischer oder heteroaromatischer Sechsring ankondensiert ist.

3. Verbindung nach Anspruch 1 oder 2, **dadurch gekennzeichnet, dass** an mindestens eine der beiden Bindungen gewählt aus Bindungen B und C und an mindestens eine der beiden Bindungen gewählt aus Bindungen B' und C' jeweils ein aromatischer oder heteroaromatischer Sechsring ankondensiert ist, wobei der aromatische oder heteroaromatische Sechsring mit einem oder mehreren Resten R¹ substituiert sein kann.

4. Verbindung nach einem oder mehreren der Ansprüche 1 bis 3, **dadurch gekennzeichnet, dass** die Gruppe X bei jedem Auftreten gleich oder verschieden gewählt ist aus BR², C(R²)₂, Si(R²)₂, O, S und NR².

5. Verbindung nach einem oder mehreren der Ansprüche 1 bis 4, **dadurch gekennzeichnet, dass** die Gruppe Z gleich CR¹ ist.

6. Verbindung nach einem oder mehreren der Ansprüche 1 bis 5, **dadurch gekennzeichnet, dass** Ar¹ gewählt ist aus Phenyl, Naphthyl, Biphenyl, Terphenyl, Fluorenyl, Monobenzofluorenyl, Dibenzofluorenyl, Spirobifluorenyl, Indenofluorenyl, Dibenzofuranyl, Carbazolyl und Dibenzothiophenyl, die jeweils mit einem oder mehreren Resten R¹ substituiert sein können.

7. Verbindung nach einem oder mehreren der Ansprüche 1 bis 6, **dadurch gekennzeichnet, dass** sie der folgenden Formel (I-A) entspricht 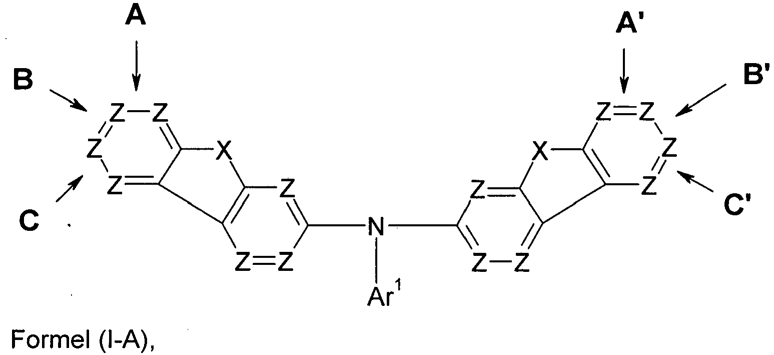
wobei die auftretenden Gruppen wie in einem oder mehreren der Ansprüche 1 bis 6 definiert sind,
wobei an mindestens eine der drei Bindungen gewählt aus den Bindungen A, B und C und an mindestens eine der drei Bindungen gewählt aus den Bindungen A', B' und C' ist ein aromatischer oder heteroaromatischer Sechsring ankondensiert, der mit einem oder mehreren Resten R¹ substituiert sein kann, und
wobei der Fall ausgeschlossen ist, bei dem an beide Bindungen A und A' jeweils ein aromatischer oder heteroaromatischer Sechsring ankondensiert ist und kein weiterer aromatischer oder heteroaromatischer Sechsring an eine der anderen vier Bindungen gewählt aus den Bindungen B, C, B' und C' ankondensiert ist.

8. Verbindung nach einem oder mehreren der Ansprüche 1 bis 7, **dadurch gekennzeichnet, dass** die Verbindung der Formel (I) zusätzlich zu der Arylaminogruppe -N(Ar¹)- keine weitere Arylaminogruppe enthält.

9. Verbindung nach einem oder mehreren der Ansprüche 1 bis 8, **dadurch gekennzeichnet, dass** sie einer der folgenden Formeln (I-1) bis (I-5) entspricht 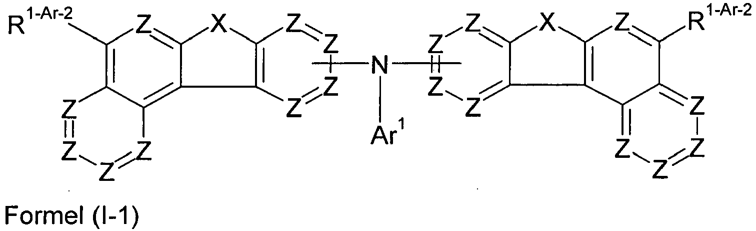 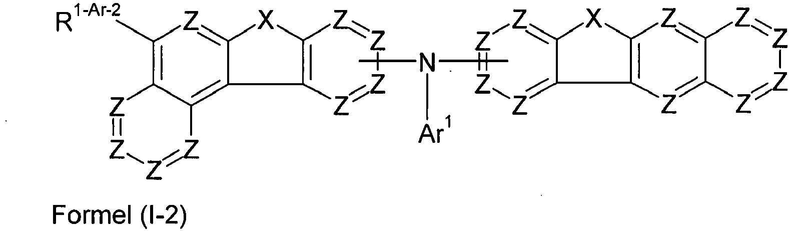 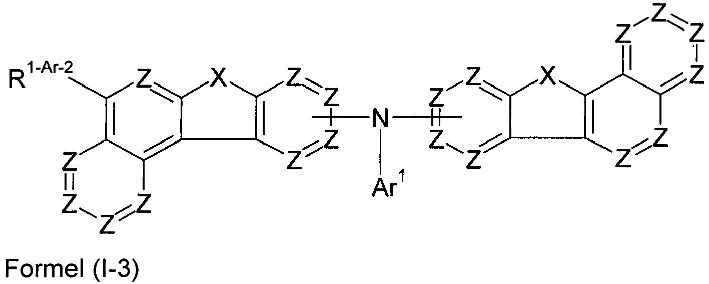 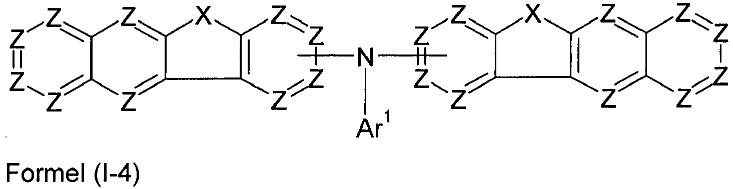 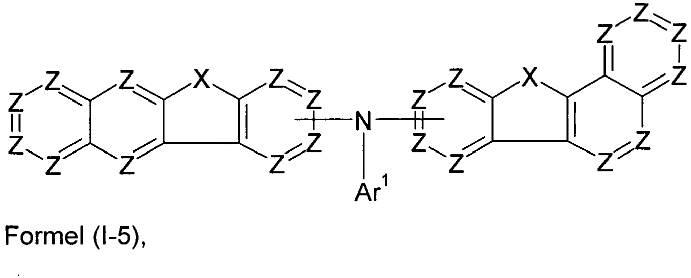 wobei die auftretenden Gruppen definiert sind wie in einem oder mehreren der Ansprüche 1 bis 8, und wobei
R^{1-Ar-2} bei jedem Auftreten gleich oder verschieden gewählt ist aus H oder geradkettigen Alkylgruppen mit 1 bis 10 C-Atomen oder verzweigten oder cyclischen Alkylgruppen mit 3 bis 10 C-Atomen, die mit einem oder mehreren Resten R³ substituiert sein können, oder aromatischen oder heteroaromatischen Ringsystemen mit 6 bis 18 aromatischen Ringatomen, die mit einem oder mehreren Resten R³ substituiert sein können.

10. Verbindung nach Anspruch 9, **dadurch gekennzeichnet, dass** die Gruppe X gleich C(R²)₂ ist.

11. Oligomer, Polymer oder Dendrimer enthaltend eine oder mehrere Verbindungen nach einem oder mehreren der Ansprüche 1 bis 10, wobei die Bindung(en) zum Polymer, Oligomer oder Dendrimer an beliebigen, in Formel (I) mit R¹ oder R² substituierten Positionen lokalisiert sein können.

12. Formulierung, enthaltend mindestens eine Verbindung nach einem oder mehreren der Ansprüche 1 bis 10 oder mindestens ein Polymer, Oligomer oder Dendrimer nach Anspruch 11 sowie mindestens ein Lösungsmittel.

13. Elektronische Vorrichtung, ausgewählt aus der Gruppe bestehend aus organischen integrierten Schaltungen (OICs), organischen Feld-Effekt-Transistoren (OFETs), organischen Dünnfilmtransistoren (OTFTs), organischen lichtemittierenden Transistoren (OLETs), organischen Solarzellen (OSCs), organischen optischen Detektoren, organischen Photorezeptoren, organischen Feld-Quench-Devices (OFQDs), organischen lichtemittierenden elektrochemischen Zellen (OLECs), organischen Laserdioden (O-Laser) und organischen Elektrolumineszenzvorrichtungen (OLEDs), enthaltend mindestens eine Verbindung nach einem oder mehreren der Ansprüche 1 bis 10.

14. Elektronische Vorrichtung nach Anspruch 13, ausgewählt aus organischen Elektrolumineszenzvorrichtungen, **dadurch gekennzeichnet, dass** die Verbindung nach einem oder mehreren der Ansprüche 1 bis 10 in mindestens einer organischen Schicht gewählt aus Lochtransportschicht, Lochinjektionsschicht, Elektronenblockierschicht und emittierender Schicht enthalten ist.

15. Verwendung einer Verbindung nach einem oder mehreren der Ansprüche 1 bis 10 in einer elektronischen Vorrichtung.

## Claims

1. Compound of a formula (I) 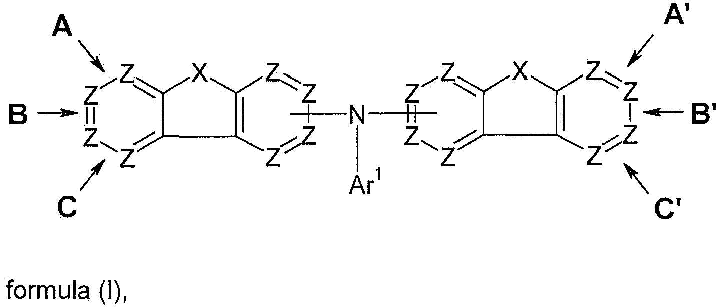 where:
an aromatic or heteroaromatic six-membered ring, which may be substituted by one or more radicals R¹, is condensed onto at least one of the three bonds selected from bonds A, B and C and onto at least one of the three bonds selected from bonds A', B' and C',
Z is on each occurrence, identically or differently, CR¹ or N;
X is on each occurrence, identically or differently, BR², C(R²)₂, C(R²)₂-C(R²)₂, -R²C=CR²-, -R²C=N-, Si(R²)₂, Si(R²)₂-Si(R²)₂, C=O, O, S, S=O, SO₂, NR², PR² or P(O)R²;
Ar¹ is an aromatic or heteroaromatic ring system having 6 to 40 aromatic ring atoms, which may be substituted by one or more radicals R¹;
R¹ is on each occurrence, identically or differently, H, D, F, Cl, Br, I, C(=O)R³, CN, Si(R³)₃, P(=O)(R³)₂, S(O)R³, S(=O)₂R³, a straight-chain alkyl or alkoxy group having 1 to 20 C atoms or a branched or cyclic alkyl or alkoxy group having 3 to 20 C atoms or an alkenyl or alkynyl group having 2 to 20 C atoms, where the above-mentioned groups may in each case be substituted by one or more radicals R³ and where one or more CH₂ groups in the above-mentioned groups may be replaced by -R³C=CR³-, -C≡C-, Si(R³)₂, C=O, C=NR³, -C(=O)O-, -C(=O)NR³-, NR³, P(=O)(R³), -O-, -S-, SO or SO₂ and where one or more H atoms in the above-mentioned groups may be replaced by D, F, Cl, Br, I or CN, or an aromatic or heteroaromatic ring system having 5 to 30 aromatic ring atoms, which may in each case be substituted by one or more radicals R³, or an aryloxy or heteroaryloxy group having 5 to 30 aromatic ring atoms, which may be substituted by one or more radicals R³;
R² is on each occurrence, identically or differently, H, D, F, Cl, Br, I, C(=O)R³, CN, Si(R³)₃, N(R³)₂, P(=O)(R³)₂, S(=O)R³, S(=O)₂R³, a straight-chain alkyl or alkoxy group having 1 to 20 C atoms or a branched or cyclic alkyl or alkoxy group having 3 to 20 C atoms or an alkenyl or alkynyl group having 2 to 20 C atoms, where the above-mentioned groups may in each case be substituted by one or more radicals R³ and where one or more CH₂ groups in the above-mentioned groups may be replaced by -R³C=CR³-, -C≡C-, Si(R³)₂, C=O, C=NR³, -C(=O)O-, -C(=O)NR³-, NR³, P(=O)(R³), -O-, -S-, SO or SO₂ and where one or more H atoms in the above-mentioned groups may be replaced by D, F, Cl, Br, I or CN, or an aromatic or heteroaromatic ring system having 5 to 30 aromatic ring atoms, which may in each case be substituted by one or more radicals R³, or an aryloxy or heteroaryloxy group having 5 to 30 aromatic ring atoms, which may be substituted by one or more radicals R³, where two or more radicals R² may be linked to one another and may form a ring;
R³ is on each occurrence, identically or differently, H, D, F, Cl, Br, I, C(=O)R⁴, CN, Si(R⁴)₃, N(R⁴)₂, P(=O)(R⁴)₂, S(=O)R⁴, S(=O)₂R⁴, a straight-chain alkyl or alkoxy group having 1 to 20 C atoms or a branched or cyclic alkyl or alkoxy group having 3 to 20 C atoms or an alkenyl or alkynyl group having 2 to 20 C atoms, where the above-mentioned groups may in each case be substituted by one or more radicals R⁴ and where one or more CH₂ groups in the above-mentioned groups may be replaced by -R⁴C=CR⁴-, -C=C-, Si(R⁴)₂, C=O, C=NR⁴, -C(=O)O-, -C(=O)NR⁴-, NR⁴, P(=O)(R⁴), -O-, -S-, SO or SO₂ and where one or more H atoms in the above-mentioned groups may be replaced by D, F, Cl, Br, I or CN, or an aromatic or heteroaromatic ring system having 5 to 30 aromatic ring atoms, which may in each case be substituted by one or more radicals R⁴, or an aryloxy or heteroaryloxy group having 5 to 30 aromatic ring atoms, which may be substituted by one or more radicals R⁴;
R⁴ is on each occurrence, identically or differently, H, D, F or an aliphatic, aromatic or heteroaromatic organic radical having 1 to 20 C atoms, in which, in addition, one or more H atoms may be replaced by D or F;
where the case in which an aromatic or heteroaromatic six-membered ring is condensed onto each of the two bonds A and A' and no further aromatic or heteroaromatic six-membered ring is condensed onto one of the other four bonds selected from bonds B, C, B' and C' is excluded.

2. Compound according to Claim 1, **characterised in that** an aromatic or heteroaromatic six-membered ring is not condensed onto either of the two bonds A and A' in formula (I).

3. Compound according to Claim 1 or 2, **characterised in that** an aromatic or heteroaromatic six-membered ring is in each case condensed onto at least one of the two bonds selected from bonds B and C and onto at least one of the two bonds selected from bonds B' and C', where the aromatic or heteroaromatic six-membered ring may be substituted by one or more radicals R¹.

4. Compound according to one or more of Claims 1 to 3, **characterised in that** the group X is selected on each occurrence, identically or differently, from BR², C(R²)₂, Si(R²)₂, O, S and NR².

5. Compound according to one or more of Claims 1 to 4, **characterised in that** the group Z is equal to CR¹.

6. Compound according to one or more of Claims 1 to 5, **characterised in that** Ar¹ is selected from phenyl, naphthyl, biphenyl, terphenyl, fluorenyl, monobenzofluorenyl, dibenzofluorenyl, spirobifluorenyl, indenofluorenyl, dibenzofuranyl, carbazolyl and dibenzothiophenyl, each of which may be substituted by one or more radicals R¹.

7. Compound according to one or more of Claims 1 to 6, **characterised in that** it conforms to the following formula (I-A) 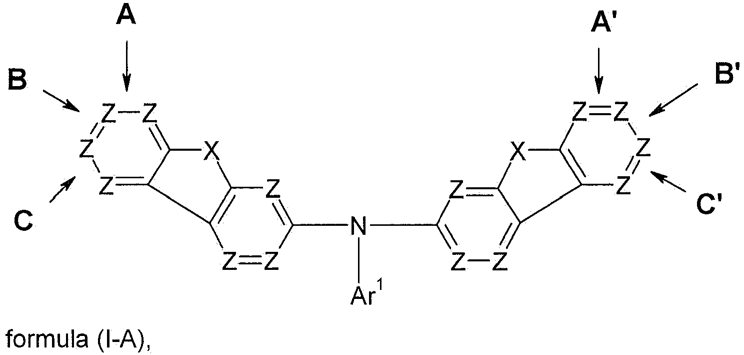
where the groups occurring are as defined in one or more of Claims 1 to 6,
where an aromatic or heteroaromatic six-membered ring, which may be substituted by one or more radicals R¹, is condensed onto at least one of the three bonds selected from bonds A, B and C and onto at least one of the three bonds selected from bonds A', B' and C', and where the case in which an aromatic or heteroaromatic six-membered ring is condensed onto each of the two bonds A and A' and no further aromatic or heteroaromatic six-membered ring is condensed onto one of the other four bonds selected from bonds B, C, B' and C' is excluded.

8. Compound according to one or more of Claims 1 to 7, **characterised in that** the compound of the formula (I) contains no further arylamino group in addition to the arylamino group -N(Ar¹)-.

9. Compound according to one or more of Claims 1 to 8, **characterised in that** it conforms to one of the following formulae (I-1) to (I-5) 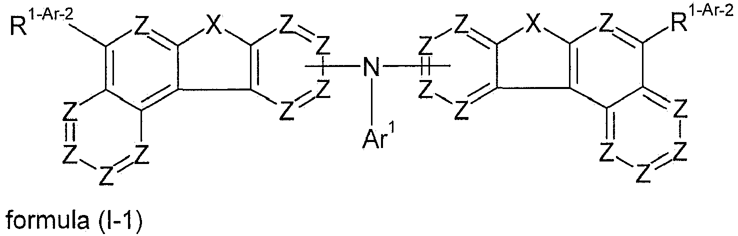 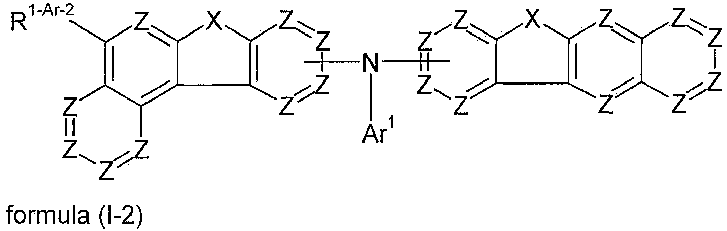 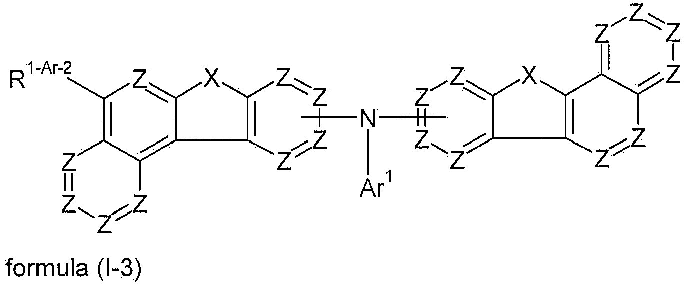 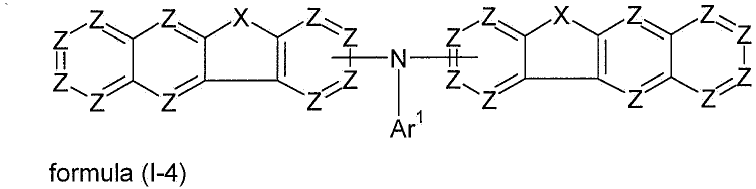 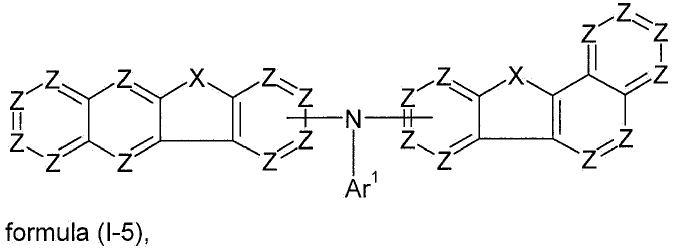 where the groups occurring are defined as in one or more of Claims 1 to 8, and where
R^{1-Ar-2} is selected on each occurrence, identically or differently, from H or straight-chain alkyl groups having 1 to 10 C atoms or branched or cyclic alkyl groups having 3 to 10 C atoms, which may be substituted by one or more radicals R³, or aromatic or heteroaromatic ring systems having 6 to 18 aromatic ring atoms, which may be substituted by one or more radicals R³.

10. Compound according to Claim 9, **characterised in that** the group X is equal to C(R²)₂.

11. Oligomer, polymer or dendrimer containing one or more compounds according to one or more of Claims 1 to 10, where the bond(s) to the polymer, oligomer or dendrimer may be localised at any desired positions in formula (I) that are substituted by R¹ or R².

12. Formulation comprising at least one compound according to one or more of Claims 1 to 10 or at least one polymer, oligomer or dendrimer according to Claim 11 and at least one solvent.

13. Electronic device selected from the group consisting of organic integrated circuits (OICs), organic field-effect transistors (OFETs), organic thin-film transistors (OTFTs), organic light-emitting transistors (OLETs), organic solar cells (OSCs), organic optical detectors, organic photoreceptors, organic field-quench devices (OFQDs), organic light-emitting electrochemical cells (OLECs), organic laser diodes (O-lasers) and organic electroluminescent devices (OLEDs) comprising at least one compound according to one or more of Claims 1 to 10.

14. Electronic device according to Claim 13, selected from organic electroluminescent devices, **characterised in that** the compound according to one or more of Claims 1 to 10 is present in at least one organic layer selected from hole-transport layer, hole-injection layer, electron-blocking layer and emitting layer.

15. Use of a compound according to one or more of Claims 1 to 10 in an electronic device.

## Revendications

1. Composé d'une formule (I) : 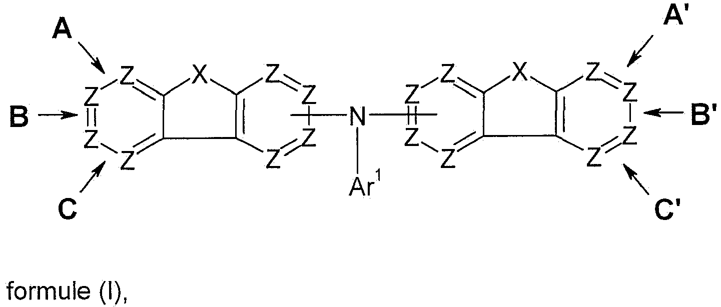 dans laquelle :
un cycle à six éléments aromatique ou hétéroaromatique, lequel peut être substitué par un radical ou par plusieurs radicaux R¹, est condensé sur au moins l'une des trois liaisons qui sont sélectionnées parmi les liaisons A, B et C et sur au moins l'une des trois liaisons qui sont sélectionnées parmi les liaisons A', B' et C' ;
Z est pour chaque occurrence, de manière identique ou différente, CR¹ ou N ;
X est pour chaque occurrence, de manière identique ou différente, BR², C(R²)₂, C(R²)₂-C(R²)₂, -R²C=CR²-, -R²C=N-, Si(R²)₂, Si(R²)₂-Si(R²)₂, C=O, O, S, S=O, SO₂, NR², PR² ou P(=O)R² ;
Ar¹ est un système de cycle aromatique ou hétéroaromatique qui comporte de 6 à 40 atomes de cycle aromatique, lequel peut être substitué par un radical ou par plusieurs radicaux R¹ ;
R¹ est pour chaque occurrence, de manière identique ou différente, H, D, F, Cl, Br, I, C(=O)R³, CN, Si(R³)₃, P(=O)(R³)₂, S(=O)R³, S(=O)₂R³, un groupe alkyle ou alcoxy en chaîne droite qui comporte de 1 à 20 atome(s) de C ou un groupe alkyle ou alcoxy ramifié ou cyclique qui comporte de 3 à 20 atomes de C ou un groupe alkényle ou alkynyle qui comporte de 2 à 20 atomes de C, où les groupes qui ont été mentionnés ci-avant peuvent dans chaque cas être substitués par un radical ou par plusieurs radicaux R³ et où un ou plusieurs groupe(s) CH₂ dans les groupes qui ont été mentionnés ci-avant peut/peuvent être remplacé(s) par -R³C=CR³-, -C≡C-, Si(R³)₂, C=O, C=NR³, -C(=O)O-, -C(=O)NR³-, NR³, P(=O)(R³), -O-, -S-, SO ou SO₂ et où un ou plusieurs atome(s) de H dans les groupes qui ont été mentionnés ci-avant peut/peuvent être remplacé(s) par D, F, Cl, Br, I ou CN, ou un système de cycle aromatique ou hétéroaromatique qui comporte de 5 à 30 atomes de cycle aromatique, lequel peut dans chaque cas être substitué par un radical ou par plusieurs radicaux R³, ou un groupe aryloxy ou hétéroaryloxy qui comporte de 5 à 30 atomes de cycle aromatique, lequel peut être substitué par un radical ou par plusieurs radicaux R³ ;
R² est pour chaque occurrence, de manière identique ou différente, H, D, F, Cl, Br, I, C(=O)R³, CN, Si(R³)₃, N(R³)₂, P(=O)(R³)₂, S(=O)R³, S(=O)₂R³, un groupe alkyle ou alcoxy en chaîne droite qui comporte de 1 à 20 atome(s) de C ou un groupe alkyle ou alcoxy ramifié ou cyclique qui comporte de 3 à 20 atomes de C ou un groupe alkényle ou alkynyle qui comporte de 2 à 20 atomes de C, où les groupes qui ont été mentionnés ci-avant peuvent dans chaque cas être substitués par un radical ou par plusieurs radicaux R³ et où un ou plusieurs groupe(s) CH₂ dans les groupes qui ont été mentionnés ci-avant peut/peuvent être remplacé(s) par -R³C=CR³-, -C≡C-, Si(R³)₂, C=O, C=NR³, -C(=O)O-, -C(=O)NR³-, NR³, P(=O)(R³), -O-, -S-, SO ou SO₂ et où un ou plusieurs atome(s) de H dans les groupes qui ont été mentionnés ci-avant peut/peuvent être remplacé(s) par D, F, Cl, Br, I ou CN, ou un système de cycle aromatique ou hétéroaromatique qui comporte de 5 à 30 atomes de cycle aromatique, lequel peut dans chaque cas être substitué par un radical ou par plusieurs radicaux R³, ou un groupe aryloxy ou hétéroaryloxy qui comporte de 5 à 30 atomes de cycle aromatique, lequel peut être substitué par un radical ou par plusieurs radicaux R³, où deux radicaux R² ou plus peuvent être liés l'un à l'autre ou les uns aux autres et peuvent former un cycle ;
R³ est pour chaque occurrence, de manière identique ou différente, H, D, F, Cl, Br, I, C(=O)R⁴, CN, Si(R⁴)₃, N(R⁴)₂, P(=O)(R⁴)₂, S(=O)R⁴, S(=O)₂R⁴, un groupe alkyle ou alcoxy en chaîne droite qui comporte de 1 à 20 atome(s) de C ou un groupe alkyle ou alcoxy ramifié ou cyclique qui comporte de 3 à 20 atomes de C ou un groupe alkényle ou alkynyle qui comporte de 2 à 20 atomes de C, où les groupes qui ont été mentionnés ci-avant peuvent dans chaque cas être substitués par un radical ou par plusieurs radicaux R⁴ et où un ou plusieurs groupe(s) CH₂ dans les groupes qui ont été mentionnés ci-avant peut/peuvent être remplacé(s) par -R⁴C=CR⁴-, -C≡C-, Si(R⁴)₂, C=O, C=NR⁴, -C(=O)O-, -C(=O)NR⁴-, NR⁴, P(=O)(R⁴), -O-, -S-, SO ou SO₂ et où un ou plusieurs atome(s) de H dans les groupes qui ont été mentionnés ci-avant peut/peuvent être remplacé(s) par D, F, Cl, Br, I ou CN, ou un système de cycle aromatique ou hétéroaromatique qui comporte de 5 à 30 atomes de cycle aromatique, lequel peut dans chaque cas être substitué par un radical ou par plusieurs radicaux R⁴, ou un groupe aryloxy ou hétéroaryloxy qui comporte de 5 à 30 atomes de cycle aromatique, lequel peut être substitué par un radical ou par plusieurs radicaux R⁴ ;
R⁴ est pour chaque occurrence, de manière identique ou différente, H, D, F ou un radical organique aliphatique, aromatique ou hétéroaromatique qui comporte de 1 à 20 atome(s) de C, où, en outre, un ou plusieurs atome(s) de H peut/peuvent être remplacé(s) par D ou F ;
où le cas dans lequel un cycle à six éléments aromatique ou hétéroaromatique est condensé su chacune des deux liaisons A et A' et aucun autre cycle à six éléments aromatique ou hétéroaromatique n'est condensé sur l'une des quatre autres liaisons qui sont sélectionnées parmi les liaisons B, C, B' et C' est exclus.

2. Composé selon la revendication 1, **caractérisé en ce qu'**un cycle à six éléments aromatique ou hétéroaromatique n'est pas condensé sur l'une ou l'autre des deux liaisons A et A' dans la formule (I).

3. Composé selon la revendication 1 ou 2, **caractérisé en ce qu'**un cycle à six éléments aromatique ou hétéroaromatique est dans chaque cas condensé sur au moins l'une des deux liaisons qui sont sélectionnées parmi les liaisons B et C et sur au moins l'une des deux liaisons qui sont sélectionnées parmi les liaisons B' et C', où le cycle à six éléments aromatique ou hétéroaromatique peut être substitué par un radical ou par plusieurs radicaux R¹.

4. Composé selon une ou plusieurs des revendications 1 à 3, **caractérisé en ce que** le groupe X est sélectionné pour chaque occurrence, de manière identique ou différente, parmi BR², C(R²)₂, Si(R²)₂, O, S et NR².

5. Composé selon une ou plusieurs des revendications 1 à 4, **caractérisé en ce que** le groupe Z est égal à CR¹.

6. Composé selon une ou plusieurs des revendications 1 à 5, **caractérisé en ce que** Ar¹ est sélectionné parmi phényle, naphtyle, biphényle, terphényle, fluorényle, monobenzofluorényle, dibenzofluorényle, spirobifluorényle, indénofluorényle, dibenzofuranyle, carbazolyle et dibenzothiophényle, dont chacun peut être substitué par un radical ou par plusieurs radicaux R¹.

7. Composé selon une ou plusieurs des revendications 1 à 6, **caractérisé en ce qu'**il est conforme à la formule (I-A) qui suit : 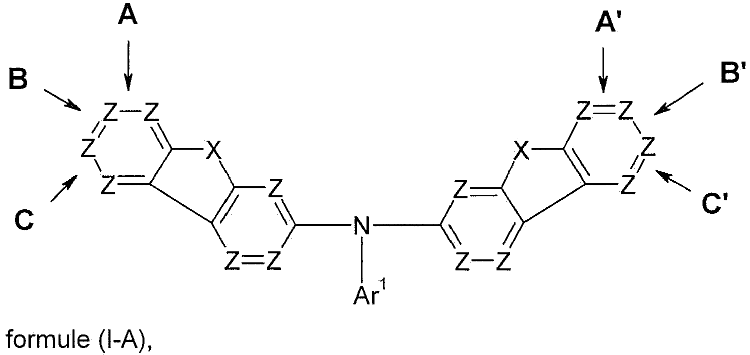
dans laquelle les groupes qui sont rencontrés sont tel que défini selon une ou plusieurs des revendications 1 à 6 ;
où un cycle à six éléments aromatique ou hétéroaromatique, lequel peut être substitué par un radical ou par plusieurs radicaux R¹, est condensé sur au moins l'une des trois liaisons qui sont sélectionnées parmi les liaisons A, B et C et sur au moins l'une des trois liaisons qui sont sélectionnées parmi les liaisons A', B' et C' ; et
où le cas dans lequel un cycle à six éléments aromatique ou hétéroaromatique est condensé sur chacune des deux liaisons A et A' et aucun autre cycle à six éléments aromatique ou hétéroaromatique n'est condensé sur l'une des quatre autres liaisons qui sont sélectionnées parmi les liaisons B, C, B' et C' est exclus.

8. Composé selon une ou plusieurs des revendications 1 à 7, **caractérisé en ce que** le composé de la formule (I) ne contient pas d'autre groupe arylamino en plus du groupe arylamino -N(Ar¹)-.

9. Composé selon une ou plusieurs des revendications 1 à 8, **caractérisé en ce qu'**il est conforme à l'une des formules (I-1) à (I-5) qui suivent : 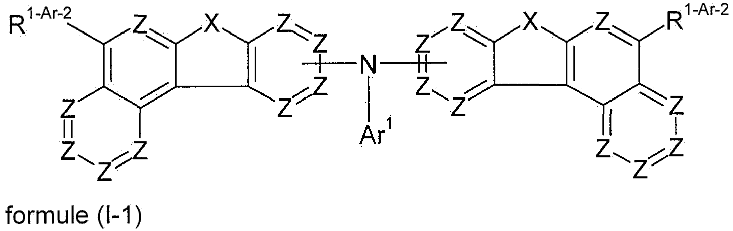 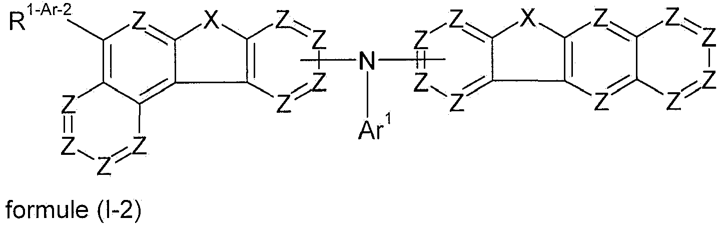 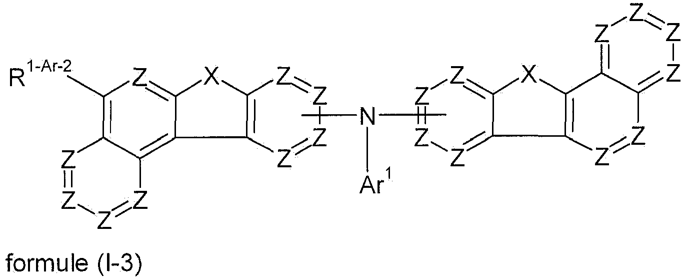 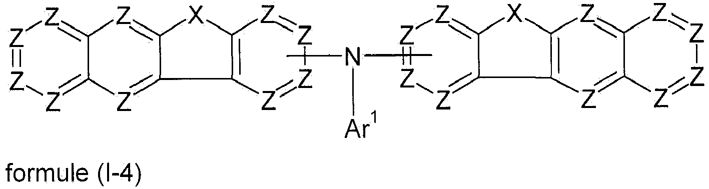 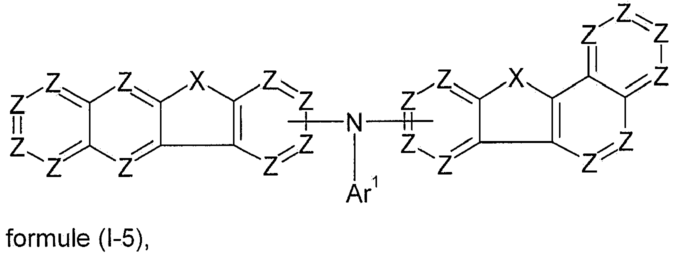 dans lesquelles les groupes qui surviennent sont définis tel que selon une ou plusieurs des revendications 1 à 8 ; et dans lesquelles :
R^{1-Ar-2} est sélectionné pour chaque occurrence, de manière identique ou différente, parmi H ou des groupes alkyle en chaîne droite qui comportent de 1 à 10 atome(s) de C ou des groupes alkyle ramifiés ou cycliques qui comportent de 3 à 10 atomes de C, lesquels peuvent être substitués par un radical ou par plusieurs radicaux R³, ou des systèmes de cycle aromatique ou hétéroaromatique qui comportent de 6 à 18 atomes de cycle aromatique, lesquels peuvent être substitués par un radical ou par plusieurs radicaux R³.

10. Composé selon la revendication 9, **caractérisé en ce que** le groupe X est égal à C(R²)₂.

11. Oligomère, polymère ou dendrimère qui contient un ou plusieurs composé(s) selon une ou plusieurs des revendications 1 à 10, où la/les liaison(s) sur le polymère, sur l'oligomère ou sur le dendrimère peut/peuvent être localisée(s) au niveau de n'importe quelles positions souhaitées de la formule (I) qui sont substituées par R¹ ou par R².

12. Formulation comprenant au moins un composé selon une ou plusieurs des revendications 1 à 10 ou au moins un polymère, un oligomère ou un dendrimère selon la revendication 11 et au moins un solvant.

13. Dispositif électronique qui est sélectionné parmi le groupe qui est constitué par les circuits intégrés organiques (OIC), les transistors à effet de champ organiques (OFET), les transistors à film mince organiques (OTFT), les transistors à émission de lumière/électroluminescents organiques (OLET), les cellules solaires organiques (OSC), les détecteurs optiques organiques, les photorécepteurs organiques, les dispositifs à extinction de champ organiques (OFQD), les cellules électrochimiques à émission de lumière/électroluminescentes organiques (OLEC), les diodes laser organiques (O-laser) et les dispositifs électroluminescents organiques (OLED) qui comprennent au moins un composé selon une ou plusieurs des revendications 1 à 10.

14. Dispositif électronique selon la revendication 13, lequel est sélectionné parmi les dispositifs électroluminescents organiques, **caractérisé en ce que** le composé selon une ou plusieurs des revendications 1 à 10 est présent dans au moins une couche organique qui est sélectionnée parmi une couche de transport de trous, une couche d'injection de trous, une couche de blocage d'électrons et une couche d'émission.

15. Utilisation d'un composé selon une ou plusieurs des revendications 1 à 10 dans un dispositif électronique.
